(19) Europäisches Patentamt
European Patent Office
Office européen des brevets

(11) Veröffentlichungsnummer : **0 086 750**
**B1**

(12) **EUROPÄISCHE PATENTSCHRIFT**

(45) Veröffentlichungstag der Patentschrift : **08.07.87**

(21) Anmeldenummer : **83810059.2**

(22) Anmeldetag : **11.02.83**

(51) Int. Cl.[4] : **A 01 N 43/42**, A 01 N 43/54, C 07 D215/26, C 07 D215/28, C 07 D215/48, C 07 D407/12, C 07 D409/12, C 07 D301/12

(54) **Verwendung von Chinolinderivaten zum Schützen von Kulturpflanzen.**

(30) Priorität : **17.02.82 CH 980/82**

(43) Veröffentlichungstag der Anmeldung : **24.08.83 Patentblatt 83/34**

(45) Bekanntmachung des Hinweises auf die Patenterteilung : **08.07.87 Patentblatt 87/28**

(84) Benannte Vertragsstaaten : **AT BE CH DE FR IT LI NL SE**

(56) Entgegenhaltungen :
**EP-A- 0 023 306**
**EP-A- 0 023 307**
**EP-A- 0 031 938**
**EUROPEAN JOURNAL OF MEDICINAL CHEMISTRY - CHIMICA THERAPEUTICA, Nr. 5, September-Oktober 1975, Seiten 463-469, A. ARESCHKA u.a.: "Aryloxyalkylamidoximes à potentialités antiagressives"**

(73) Patentinhaber : **CIBA-GEIGY AG**
**Klybeckstrasse 141**
**CH-4002 Basel (CH)**

(72) Erfinder : **Hubele, Adolf, Dr.**
**Obere Egg 9**
**CH-4312 Magden (CH)**

EP 0 086 750 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von Chinolinderivaten zum Schützen von Kulturpflanzen gegen schädigende Wirkungen von aggressiven Agrarchemikalien, Mittel, welche diese Chinolinderivate enthalten, neue Chinolinderivate und ihre Herstellung.

Beim Einsatz aggressiver Agrarchemikalien, wie Pflanzenschutzmitteln, insbesondere Herbiziden, werden die Kulturpflanzen häufig nicht unerheblich geschädigt. Um diesem Problem zu begegnen, sind bereits Mittel vorgeschlagen worden, welche derartige negative Auswirkungen an den Kulturpflanzen abschwächen oder unterbinden sollen. So werden in der DE-OS 30 00 076 pflanzenschützende Mittel, welche Nitril- und Oximderivate von Aryloxyalkancarbonsäuren enthalten, beschrieben.

Es wurde nun gefunden, dass sich überraschenderweise eine Gruppe von Chinolinderivaten hervorragend dazu eignet, Kulturpflanzen gegen schädigende Wirkungen von aggressiven Agrarchemikalien, wie beispielsweise Pflanzenschutzmitteln, insbesondere Herbiziden, zu schützen. Diese Chinolinderivate werden daher im folgenden auch als « Gegenmittel » oder « Antidot » bezeichnet.

Chinolinderivate, welche zum Schützen von Kulturpflanzen gegen schädigende Wirkungen von aggressiven Agrarchemikalien geeignet sind, entsprechen der Formel I

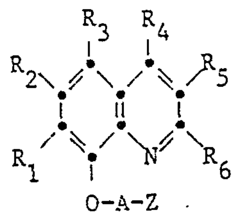

(I)

worin

$R_1$, $R_2$ und $R_3$ unabhängig voneinander Wasserstoff, Halogen, $C_1$-$C_3$-Alkyl, $C_1$-$C_3$-Alkoxy, Nitro oder Cyano,

$R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder Amidoxim, welches am Sauerstoffatom acyliert sein kann, bedeuten,

unter Einschluss ihrer Säureadditionssalze und Metallkomplexe.

Unter Amidoxim ist die Gruppe

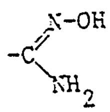

zu verstehen. Das Amidoxim kann am Sauerstoffatom acyliert sein. Als am Sauerstoffatom acylierte Amidoxime kommen solche der Formel

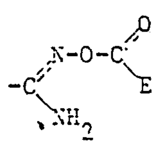

in Betracht, in denen E für $-R_7$, $-OR_8$, $-SR_9$ oder $-NR_{10}R_{11}$ steht, wobei $R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten.

Bei $R_7$ als Heterocyclus kann es sich um gesättigte, teilgesättigte oder ungesättigte Heterocyclen

handeln, wie beispielsweise Thiophen, Furan, Tetrahydrofuran und Pyrimidin.

Als Heterocyclen, welche von $R_{10}$ und $R_{11}$ gemeinsam mit dem Stickstoffatom, an das sie gebunden sind, gebildet werden, kommen gesättigte, teilgesättigte oder ungesättigte Heterocyclen in Betracht. Beispiele für solche Heterocyclen sind Pyrrolidin, Pyrrolin, Pyrrol, Imidazolidin, Imidazolin, Imidazol, Piperazin, Pyridin, Pyrimidin, Pyrazin, Thiazin, Oxazol, Thiazol und insbesondere Piperidin und Morpholin.

Als Salzbildner kommen organische und anorganische Säuren in Betracht. Beispiele organischer Säuren sind Essigsäure, Trichloressigsäure, Oxalsäure, Benzolsulfonsäure und Methansulfonsäure. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Als Metallkomplexbildner eignen sich beispielsweise Elemente der 3. und 4. Hauptgruppe, wie Aluminium, Zinn und Blei, sowie der 1. bis 8. Nebengruppe, wie beispielsweise Chrom, Mangan, Eisen, Kobalt, Nickel, Zirkon, Zink, Kupfer, Silber und Quecksilber. Bevorzugt sind die Nebengruppenelemente der 4. Periode.

Unter Halogen als Substituent oder Teil eines Substituenten sind Fluor, Chlor, Brom und Jod zu verstehen.

Unter Alkyl als Substituent oder Teil eines Substituenten kommen im Rahmen der jeweils angegebenen Anzahl von Kohlenstoffatomen alle geradkettigen und alle verzweigten Alkylgruppen in Betracht.

$C_3$-$C_6$-Cycloalkyl steht für Cyclopropyl, Cyclobutyl, Cyclopentyl und Cyclohexyl.

Von den $C_2$-$C_4$-Alkenyl- und $C_3$-$C_6$-Alkinylgruppen sind vor allem Vinyl, Allyl, 1-Propenyl, Isopropenyl und Propinyl zu erwähnen.

Besonders geeignet zur erfindungsgemässen Verwendung sind Verbindungen der Formel I, in denen $R_1$, $R_2$ und $R_3$ unabhängig voneinander Wasserstoff, Halogen, $C_1$-$C_3$-Alkyl, $C_1$-$C_3$-Alkoxy, Nitro oder Cyan, $R_4$, $R_5$ und $R_6$ unabhängig voneinander Wasserstoff, Halogen oder $C_1$-$C_3$-Alkyl, A eine der Gruppen $-CH_2-$, $-CH_2-CH_2-$ oder $-CH(CH_3)-$, Z Cyan oder eine der Gruppen

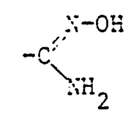 oder 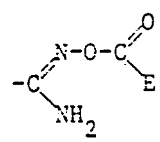

wobei E $-R_7$, $-OR_8$, $-SR_9$ oder $-NR_{10}R_{11}$, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe.

Von diesen Verbindungen sind diejenigen bevorzugt, in denen $R_1$ Wasserstoff, Chlor, Brom, Jod oder Nitro, $R_2$ Wasserstoff, $R_3$ Wasserstoff, Fluor, Chlor, Brom, Jod, $C_1$-$C_3$-Alkyl oder Nitro, $R_4$ Wasserstoff, Brom oder Methyl, $R_5$ Wasserstoff, $R_6$ Wasserstoff oder Methyl, A $-CH_2-$, $-CH_2-CH_2-$ oder $-CH(CH_3)-$, Z Cyan,

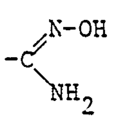 oder 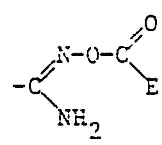

bedeuten, wobei

E für $-R_7$, $-OR_8$, $-SR_9$ oder $-NR_{10}R_{11}$ steht, worin $R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono- oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder durch Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy oder Trifluormethyl substituiert ist, und

$R_{11}$ Wasserstoff, Methyl oder Methoxy oder

$R_{10}$ und $R_{11}$ gemeinsam mit dem Stickstoffatom, an das sie gebunden sind, einen Piperidin- oder Morpholinring bedeuten.

Ganz besonders bevorzugt ist die Verwendung von Verbindungen der Formel I, in denen $R_1$ Wasserstoff, Chlor, Brom oder Jod, $R_2$ Wasserstoff, $R_3$ Wasserstoff, Chlor oder Nitro, $R_4$ und $R_5$ Wasserstoff, $R_6$ Wasserstoff oder Methyl, A $-CH_2-$, $-CH_2-CH_2-$ oder $-CH(CH_3)-$ und Z Cyan,

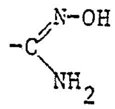 oder 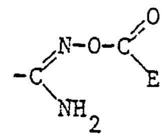

bedeuten, wobei

E für $-R_7$, $-OR_8$, $-SR_9$ oder $-NR_{10}R_{11}$ steht, worin

$R_7$ Methyl, Aethyl, n-Propyl, Isopropyl, n-Butyl, tert. Butyl, Isobutyl, Chlormethyl, Brommethyl, 2-Chloräthyl, 3-Chlor-n-propyl, 1,2-Dichloräthyl, Methoxymethyl, n-Propoxymethyl, sek. Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten.

Aus dieser Gruppe sind besonders diejenigen Verbindungen hervorzuheben, in denen $R_1$ Wasserstoff, Chlor, Brom oder Jod, $R_2$ Wasserstoff, $R_3$ Wasserstoff oder Chlor, $R_4$ und $R_5$ Wasserstoff, $R_6$ Wasserstoff oder Methyl, A $-CH_2-$ und Z Cyan,

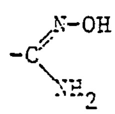 oder 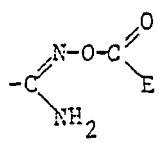

bedeuten, wobei

E für $-R_7$, $-OR_8$ oder $-NR_{10}R_{11}$ steht, worin

$R_7$ Chlormethyl, $R_8$ Methyl, $R_{10}$ Isopropyl und $R_{11}$ Wasserstoff bedeuten.

Bevorzugt zu verwendende Einzelverbindungen sind :

8-(Cyanomethoxy)-chinolin,
2-(8-Chinolinoxy)-acetamidoxim,
2-Methyl-8-(cyanomethoxy)-chinolin,
2-(2-Methyl-8-chinolinoxy)-acetamidoxim,
2-(5-Chlor-8-chinolinoxy)-acetamidoxim,
O-(Isopropylaminocarbonyl)-2-(8-chinolinoxy)-acetamidoxim,
5-Chlor-7-brom-8-(cyanomethoxy)-chinolin,
O-(Chlormethylcarbonyl)-2-(8-chinolinoxy)-acetamidoxim,
2-(5-Chlor-7-brom-8-chinolinoxy)-acetamidoxim,
2-(5-Chlor-7-jod-8-chinolinoxy)-acetamidoxim,
O-(Isopropylaminocarbonyl)-2-(5-chlor-7-brom-8-chinolinoxy)-acetamidoxim,
2-(2-Methyl-5,7-dichlor-8-chinolinoxy)-acetamidoxim),
5,7-Dichlor-8-(cyanomethoxy)-chinolin,
O-(Isopropylaminocarbonyl)-2-(5-chlor-7-jod-8-chinolinoxy)-acetamidoxim,
2-Methyl-5,7-dichlor-8-(cyanomethoxy)-chinolin,
O-(Isopropylaminocarbonyl)-2-(2-methyl-5,7-dichlor-8-chinolinoxy)-acetamidoxim, und insbesondere
5-Chlor-7-jod-8-(cyanomethoxy)-chinolin,
5-Chlor-8-(cyanomethoxy)-chinolin und
O-(Methoxycarbonyl)-2-(8-chinolinoxy)-acetamidoxim.

Als aggressive Agrarchemikalien kommen beispielsweise Defoliationsmittel, Desiccationsmittel, Mittel zum Schutz gegen Frostschäden und Pflanzenschutzmittel, wie beispielsweise Insektizide, Fungizide, Bakterizide, Nematozide und insbesondere Herbizide in Betracht. Die Agrarchemikalien können verschiedenen Stoffklassen angehören. Herbizide können beispielsweise zu einer der folgenden Stoffklassen gehören : Triazine und Triazinone ; Harnstoffe wie beispielsweise 1-(Benzthiazol-2-yl)-1,3-dimethylharnstoff (« Methabenzthiazuron ») oder insbesondere Phenylharnstoffe oder Sulfonylharnstoffe ; Carbamate und Thiocarbamate ; Halogenacetanilide, insbesondere Chloracetanilide ; Chloracetamide ; Halogenphenoxyessigsäureester ; Diphenyläther, wie beispielsweise substituierte Phenoxyphenoxyessigsäureester und -amide und substituierte Phenoxyphenoxypropionsäureester und -amide ; substituierte Pyridyloxyphenoxyessigsäureester und -amide und substituierte Pyridyloxyphenoxypropionsäureester und -amide, insbesondere 2-[4-(3,5-Dichlorpyridyl-2-oxy)-phenoxy]-propionsäure-2-propinylester und 2-[4-(5-Trifluormethylpyridyl-2-oxy)-phenoxy]-propionsäure-n-butylester ; Benzoesäurederivate ; Nitroaniline ; Oxadiazolone ; Phosphate ; und Pyrazole.

Im einzelnen kommen beispielsweise folgende Substanzen in Betracht :

Triazine und Triazinone : 2,4-Bis(isopropylamino)-6-methylthio-1,3,5-triazin (« Prometryn »), 2,4-bis(äthylamino)-6-methylthio-1,3,5-triazin (« Simetryn »), 2-(1',2'-Dimethylpropylamino)-4-äthylamino-6-methylthio-1,3,5-triazin (« Dimethametryn »), 4-Amino-6-tert. butyl-4,5-dihydro-3-methylthio-1,2,4-triazin-5-on (« Metribuzin »), 2-Chlor-4-äthylamino-6-isopropylamino-1,3,5-triazin (« Atrazin »), 2-Chlor-4,6-bis-(äthylamino)-1,3,5-triazin (« Simazin »), 2 tert. Butylamino-4-chlor-6-äthylamino-1,3,5-triazin (« Terbutylazin »), 2-tert. Butylamino-4-äthylamino-6-methoxy-1,3,5-triazin (« Terbumeton »), 2-tert.Butylamino-4-äthylamino-6-methylthio-1,3,5-triazin (« Terbutryn »), 2-Aethylamino-4-isopropylamino-6-methylthio-1,3,5-triazin (« Ametryn ») ;

Harnstoffe : 1-(Benzothiazol-2-yl)-1,3-dimethylharnstoff ; Phenylharnstoffe wie beispielsweise 3-(3-Chlor-p-tolyl)-1,1-dimethylharnstoff (« Clortoluron »), 1,1-Dimethyl-3-(ααα-trifluor-m-tolyl)-harnstoff (« Fluormeturon »), 3-(4-Brom-3-chlorphenyl)-1-methoxy-1-methylharnstoff (« Chlorbromuron »), 3-(4-Bromphenyl)-1-methoxy-1-methylharnstoff (« Metobromuron »), 3-(3,4-Dichlorphenyl)-1-methoxy-1-methylharnstoff (« Linuron »), 3-(4-Chlorphenyl)-1-methoxy-1-methylharnstoff (« Monolinuron »), 3-(3,4-Dichlorphenyl)-1,1-dimethylharnstoff (« Diuron »), 3-(4-Chlorphenyl)-1,1-dimethylharnstoff (« Monuron »), 3-(3-Chlor-4-methoxyphenyl)-1,1-dimethylharnstoff (« Metoxuron ») ; Sulfonylharnstoffe wie beispielsweise N-(2-Chlorphenylsulfonyl)-N'-(4-methoxy-6-methyl-1,3,5-triazin-2-yl)-harnstoff, N-(2-Methoxycarbonylphenylsulfonyl)-N'-(4,6-dimethylpyrimidin-2-yl)-harnstoff, N-(2,5-Dichlorphenylsulfonyl)-N'-(4,6-dimethoxypyrimidin-2-yl)-harnstoff, N-[2-(2-butenyloxy)-phenylsulfonyl]-N'-(4-methoxy-6-methyl-1,3,5-triazin-2-yl)-harnstoff sowie die in den europäischen Patentpublikationen 44808 und 44809 genannten Sulfonylharnstoffe ;

Carbamate und Thiocarbamate : N-(3',4'-Dichlorphenyl)-propionanilid (« propanil »), S-4-Chlorbenzyl-diäthyl-thiocarbamat (« Benthiocarb »), S-Aethyl-N,N-hexamethylen-thiocarbamat (« Molinate »), S-Aethyl-di-propyl-thiocarbamat (« EPTC »), N,N-di-sec.Butyl-S-benzyl-thiocarbamat, S-(2,3-Dichlorallyl)-di-isopropyl-thiocarbamat (« Di-allate »), 1-(Propylthiocarbonyl)-decahydro-chinaldin, S-Aethyl-diisobutylthiocarbamat (« Butylate ») ;

Chloracetanilide : 2-Chlor-2',6'-diäthyl-N-(2"-n-propoxyäthyl)-acetanilid (« Propalochlor »), 2-Chlor-6'-äthyl-N-(2"-methoxy-1"-methyläthyl)-acet-o-toluidid (« Metolachlor »), 2-Chlor-2',6'-diäthyl-N-(butoxymethyl)acetanilid (« Butachlor »), 2-Chlor-6'-äthyl-N-(äthoxymethyl)acet-o-toluidid (« Acetochlor »), 2-Chlor-6'-äthyl-N-(2"-propoxy-1"-methyläthyl)acet-o-toluidid, 2-Chlor-2',6'-dimethyl-N-(2"-methoxy-1"-methyläthyl)acetanilid, 2-Chlor-2',6'-dimethyl-N-(2"-methoxyäthyl)acetanilid (« Dimethachlor »), 2-Chlor-2',6'-diäthyl-N-(pyrazol-1-ylmethyl)acetanilid, 2-Chlor-6'-äthyl-N-(pyrazol-1-ylmethyl)acet-o-toluidid, 2-Chlor-6'-äthyl-N-(3,5-dimethyl-pyrazol-1-ylmethyl)acet-o-toluidid, 2-Chlor-6'-äthyl-N-(2"-butoxy-1"-methyläthyl)acet-o-toluidid (« Metazolachlor »), 2-Chlor-6'-äthyl-N-(2"-butoxyl-1"-(methyläthyl)acet-o-toluidid und 2-Chlor-2'-trimethylsilyl-N-(butoxymethyl)-acetanilid ;

Chloracetamide : N-[1-Isopropyl-2-methylpropen-1-yl-(1)]-N-(2'-methoxyäthyl)-chloracetamid.

Diphenyläther und Nitrodiphenyläther : 2,4-Dichlorphenyl-4'-nitro-phenyläther (« Nitrofen »), 2-Chlor-1-(3'-äthoxy-4'-nitrophenoxy)-4-trifluormethyl-benzol (« Oxyfluorfen »), 2',4'-Dichlorphenyl-3-methoxy-4-nitrophenyl-äther (« Chlormethoxynil »), 2-[4'-(2",4"-Dichlorphenoxy)-phenoxy]propionsäure-methylester, N-(2'-Phenoxyäthyl)-2-[5'(2"-chlor-4"-trifluormethylphenoxy)-phenoxy]-propionsäureamid, 2-[2-Nitro-5-(2-chlor-4-trifluormethylphenoxy)-phenoxy]-propionsäure-2-methoxyäthylester ; 2-Chlor-4-trifluormethylphenyl-3'-oxazolin-2'-yl-4'-nitrophenyläther ;

Benzoesäurederivate : Methyl-5-(2',4'-dichlorphenoxy)-2-nitrobenzoat (« Bifenox »), 5-(2'-Chlor-4'-trifluormethylphenoxy)-2-nitrobenzoesäure (« Acifluorfen »), 2,6-Dichlorbenzonitril (« Dichlobenil »).

Nitroaniline : 2,6-Dinitro-N,N-dipropyl-4-trifluormethylanilin (« Trifluralin »), N-(1'-Aethylpropyl)-2,6-dinitro-3,4-xylidin (« Pendimethalin »).

Oxadiazolone : 5-tert.-Butyl-3-(2',4'-dichlor-5'-isopropoxyphenyl)-1,3,4-oxadiazol-2-on (« Oxadiazon »).

Phosphate : S-2-Methylpiperidino-carbonylmethyl-O,O-dipropyl-phosphorodithioat (« Piperophos »).

Pyrazole : 1,3-Dimethyl-4-(2',4'-dichlorbenzoyl)-5-(4'-tolylsulfonyloxy)-pyrazol.

Besonders geeignet sind die Verbindungen der Formel I zum Schützen von Kulturpflanzen gegen schädigende Wirkungen von Herbiziden der Formel A

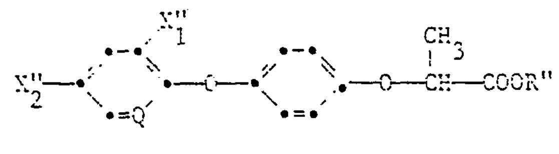

(A)

worin
$X_1''$ Wasserstoff oder Halogen,
$X_2''$ Wasserstoff, Halogen oder Trifluormethyl,
Q das Fragment =N— oder =CH—,
R'' $C_1$-$C_4$-Alkyl, welches unsubstituiert oder durch $C_1$-$C_4$-Alkoxy substituiert ist, $C_3$-$C_4$-Alkenyl, $C_3$-$C_4$-Alkinyl oder

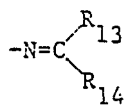

$R_{13}$ $C_1$-$C_4$-Alkyl, $R_{14}$ $C_1$-$C_4$-Alkyl oder $R_{13}$ und $R_{14}$ gemeinsam $C_1$-$C_5$-Alkylen bedeuten.

Als Kulturpflanzen, welche durch Chinolinderivate der Formel I gegen aggressive Agrarchemikalien geschützt werden können, kommen insbesondere diejenigen in Betracht, die auf dem Nahrungs- oder Textilsektor von Bedeutung sind, wie beispielsweise Kulturhirse, Reis, Mais, Getreidearten (Weizen, Roggen, Gerste, Hafer), Baumwolle, Zuckerrüben, Zuckerrohr und Soja.

Ein geeignetes Verfahren zum Schützen von Kulturpflanzen unter Verwendung von Verbindungen der Formel I besteht darin, dass man Kulturpflanzen, Teile dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz der Agrarchemikalie erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid und einer Verbindung der Formel I oder Ia oder einem Mittel, welches diese Kombination enthält, behandelt. Die die Herbizid/Antidot-Kombination enthaltenden Mittel bilden ebenfalls einen Bestandteil der vorliegenden Erfindung.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit Kulturpflanzen bewachsenen oder die ausgesäten Bodenareale, wie auch die zur Bebauung mit Kulturpflanzen bestimmten Böden.

Die zu applizierende Aufwandmenge Antidot im Verhältnis zur Agrarchemikalie richtet sich weitgehend nach der Anwendungsart. Bei einer Feldbehandlung, welche entweder unter Verwendung einer Tankmischung oder durch getrennte Applikation von Agrarchemikalie und Antidot durchgeführt wird, liegt in der Regel ein Verhältnis von Antidot zu Agrarchemikalie von 1 : 100 bis 10 : 1, bevorzugt 1 : 5 bis 8 : 1, und insbesondere 1 : 1, vor.

Dagegen werden bei der Samenbeizung und ähnlichen Einsatzmethoden weit geringere Mengen Antidot im Verhältnis zur Aufwandmenge an Agrarchemikalie/ha Anbaufläche benötigt. Bei der Samenbeizung werden in der Regel 0,1 bis 10 g Antidot/kg Samen, bevorzugt 1 bis 2 g, appliziert. Wird das Gegenmittel kurz vor der Aussaat unter Semenquellung appliziert, so werden zweckmässigerweise Antidot-Lösungen verwendet, welche den Wirkstoff in einer Konzentration von 1 bis 10 000 ppm,

bevorzugt 100 bis 1 000 ppm, enthalten.

Die Verbindungen der Formel I können für sich allein oder zusammen mit inerten Zusatzstoffen und/oder den zu antagonisierenden Agrarchemikalien zur Anwendung gelangen.

Die vorliegende Anmeldung betrifft daher auch Mittel, welche Verbindungen der Formel I und inerte Zusatzstoffe und/oder zu antagonisierende Agrarchemikalien, insbesondere Pflanzenschutzmittel und vor allem Herbizide, enthalten.

Zur Applikation werden die Verbindungen der Formel I oder Kombinationen von Verbindungen der Formel I mit zu antagonisierenden Agrarchemikalien zweckmässigerweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt.

Die Formulierungen, d. h., die den Wirkstoff der Formel I oder eine Kombination von Wirkstoff der Formel I mit zu antagonisierender Agrarchemikalie und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffe der Formel I und gegebenenfalls auch der zu antagonisierenden Agrarchemikalie nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können, genannt. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxyd-Adduktes oder Phospholipide in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood New Jersey, 1980 Sisely and Wood, « Encyclopedia of Surface Active Agents », Chemical Publishing Co., Inc. New York, 1980.

Die agrochemischen Zubereitungen enthalten in der Regel 0,1 bis 99 %, insbesondere 0,1 bis 95 %, Wirkstoff der Formel I, 99,9 bis 1 % insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Halfmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von aggressiven Agrarchemikalien kommen verschiedene Methoden und Techniken in Betracht, wie beispielsweise die folgenden :

i) Samenbeizung

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei etwa 10 bis 500 g Wirkstoff der Formel I (40 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50-3 200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 10 g bis 500 g, vorzugsweise 50 bis 250 g AS pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Gegenmittel und Herbizid (gegenseitiges Mengenverhältnis zwischen 10 : 1 und 1 : 10) wird verwendet, wobei die Aufwandmenge an Herbizid 0,1 bis 10 kg pro Hektar beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in die Saatfurche

Das Gegenmittel wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Verbindungen der Formel I, in denen gleichzeitig $R_1$, $R_2$, $R_4$, $R_5$ und $R_6$ Wasserstoff, $R_3$ Wasserstoff

oder Chlor, A eine der Gruppen $—CH_2—$ oder $—CH(CH_3)—$ und Z Cyan oder die Gruppe

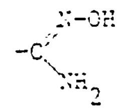

bedeuten, sind bekannt aus Areschka et al., Eur. J. Med. Chem. — Chimica Therapeutica, September-Oktober 1975, 10 (5), 463-469. Sie besitzen teilweise antiaggressive Eigenschaften.

Die übrigen Verbindungen der Formel I sind neu und stellen einen Gegenstand der vorliegenden Erfindung dar. Sie entsprechen der Formel Ia

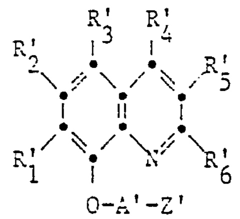

(Ia)

worin

$R_1'$, $R_2'$ und $R_3'$ unabhängig voneinander Wasserstoff, Halogen, $C_1$-$C_3$-Alkyl, $C_1$-$C_3$-Alkoxy, Nitro oder Cyano,

$R_4'$, $R_5'$ und $R_6'$ unabhängig voneinander Wasserstoff, Halogen oder $C_1$-$C_3$-Alkyl,

A' eine der Gruppen $—CH_2—$, $—CH_2—CH_2—$ oder $—CH(CH_3)—$ und

Z' Cyan oder Amidoxim, welches am Sauerstoffatom acyliert sein kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten.

Bevorzugt sind Verbindungen der Formel Ia, in denen $R_1'$, $R_2'$ und $R_3'$ unabhängig voneinander Wasserstoff, Halogen,

$C_1$-$C_3$-Alkyl, $C_1$-$C_3$-Alkoxy, Nitro oder Cyano,

$R_4'$, $R_5'$ und $R_6'$ unabhängig voneinander Wasserstoff, Halogen oder $C_1$-$C_3$-Alkyl,

A' eine der Gruppen $—CH_2—$, $—CH_2—CH_2—$ oder $—CH(CH_3)—$,

Z' Cyan oder eine der Gruppen

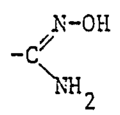 oder 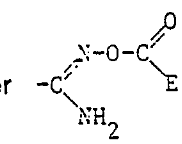

wobei

E für $—R_7$, $—OR_8$, $—SR_9$ oder $—NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl subsituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten.

Von diesen Verbindungen sind diejenigen bevorzugt, in denen $R_1'$ Wasserstoff, Chlor, Brom, Jod oder Nitro, $R_2'$ Wasserstoff, $R_3'$ Wasserstoff, Fluor, Chlor, Brom, Jod, $C_1$-$C_3$-Alkyl oder Nitro, $R_4'$ Wasserstoff, Brom oder Methyl, $R_5'$ Wasserstoff, $R_6'$ Wasserstoff oder Methyl, A' $—CH_2—$, $—CH_2—CH_2—$ oder

$-CH(CH_3)-$ und Z' Cyan, 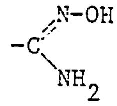 oder 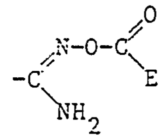

bedeuten, wobei

E für $-R_7$, $-OR_8$, $-SR_9$ oder $-NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono- oder disubstituierten Thiophen-, Furan, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder durch Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy,

oder $R_{10}$ und $R_{11}$ gemeinsam mit dem Stickstoffatom, an das sie gebunden sind, einen Piperidin- oder Morpholinring bedeuten, enthält, mit der Massgabe, dass Z' nicht für Cyan oder Amidoxin steht, wenn gleichzeitig $R_1'$, $R_2'$, $R_4'$, $R_5'$ und $R_6'$ Wasserstoff, $R_3'$ Wasserstoff oder Chlor und A' $-CH_2-$ oder $-CH(CH_3)-$ bedeuten.

Aus dieser Gruppe sind insbesondere diejenigen Verbindungen hervorzuheben, in denen $R_1'$ Wasserstoff, Chlor, Brom oder Jod, $R_2'$ Wasserstoff, $R_3'$ Wasserstoff, Chlor oder Nitro, $R_4'$ und $R_5'$ Wasserstoff, $R_6'$ Wasserstoff oder Methyl, A' $-CH_2-$, $-CH_2-CH_2-$ oder $-CH(CH_3)-$ und Z' Cyan,

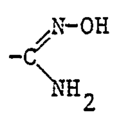 oder 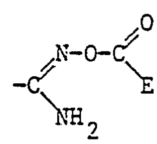

bedeuten, wobei

E für $-R_7$, $-OR_8$, $-SR_9$ oder $-NR_{10}R_{11}$ steht, worin

$R_7$ Methyl, Aethyl, n-Propyl, Isopropyl, n-Butyl, tert.Butyl, Isobutyl, Chlormethyl, Brommethyl, 2-Chloräthyl, 3-Chlor-n-propyl, 1,2-Dichloräthyl, Methoxymethyl, n-Propoxymethyl, sek.-Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten,

mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $-CH_2-$ oder $-CH(CH_3)-$ bedeuten.

Ganz besonders bevorzugt sind Verbindungen der Formel Ia, in denen $R_1'$ Wasserstoff, Chlor, Brom oder Jod, $R_2'$ Wasserstoff, $R_3'$ Wasserstoff oder Chlor, $R_4'$ und $R_5'$ Wasserstoff, $R_6'$ Wasserstoff oder Methyl, A' $-CH_2-$ und Z' Cyan,

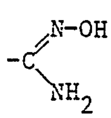 oder 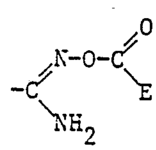

bedeuten, wobei

E für $-R_7$, $-OR_8$ oder $-NR_{10}R_{11}$ steht, worin $R_7$ Chlormethyl, $R_8$ Methyl, $R_{10}$ Isopropyl und $R_{11}$ Wasserstoff bedeuten, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $-CH_2-$ bedeuten.

Besonders hervorzuheben sind die folgenden Verbindungen :

2-Methyl-8-(cyanomethoxy)-chinolin,
2-(2-Methyl-8-chinolinoxy)-acetamidoxim,
O-(Isopropylaminocarbonyl)-2-(8-chinolinoxy)-acetamidoxim,
O-(Chlormethylcarbonyl)-2-(8-chinolinoxy)-acetamidoxim,
2-(5-Chlor-7-brom-8-chinolinoxy)-acetamidoxim,
5-Chlor-7-brom-8-(cyanomethoxy)-chinolin,
O-(Methoxycarbonyl)-2-(8-chinolinoxy)-acetamidoxim,
2-(5-Chlor-7-jod-8-chinolinoxy)-acetamidoxim,
O-(Isopropylaminocarbonyl)-2-(5-chlor-7-brom-8-chinolinoxy)-acetamidoxim,
2-(2-Methyl-5,7-dichlor-8-chinolinoxy)-acetamidoxim,
5,7-Dichlor-8-(cyanomethoxy)-chinolin,
O-(Isopropylaminocarbonyl)-2-(5-chlor-7-jod-8-chinolinoxy)-acetamidoxim,
2-Methyl-5,7-Dichlor-8-(cyanomethoxy)-chinolin,
O-(Isopropylaminocarbonyl)-2-(2-methyl-5,7-dichlor-8-chinolinoxy)-acetamidoxim,
und insbesondere
5-Chlor-7-jod-8-(cyanomethoxy)-chinolin.

Die Herstellung von Verbindungen der Formel Ia erfolgt, indem man

a) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für die Gruppe $-CH_2-CH_2-$ und Z' für Cyan steht, eine Verbindung der Formel II

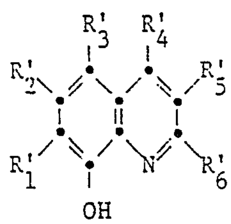

(II)

worin $R_1'$, $R_2'$, $R_3'$, $R_4'$, $R_5'$ und $R_6'$ die vorstehend angegebenen Bedeutungen haben, mit einer Verbindung der Formel III

$$CH_2{=}CH-CN \qquad (III)$$

umsetzt, oder

b) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für eine der Gruppen $-CH_2-$ oder $-CH(CH_3)-$ und Z' für Cyan steht, eine Verbindung der Formel II

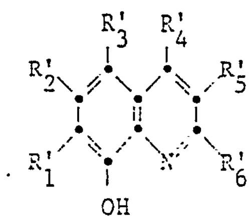

(II)

worin $R_1'$, $R_2'$, $R_3'$, $R_4'$, $R_5'$ und $R_6'$ die für Formel II angegebenen Bedeutungen haben, mit

i) einer Verbindung der Formel IV

$$Hal-A'-CH \qquad (IV)$$

worin Hal für ein Halogenatom steht und A' die vorstehend angegebene Bedeutung hat, umsetzt, oder

ii) einer Verbindung der Formel V

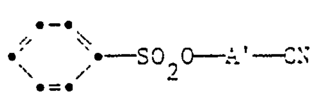

(V)

worin A' die vorstehend angegebene Bedeutung hat, umsetzt, oder

iii) einer Verbindung der Formel VI

$$Hal—A'—COOR_{12} \qquad (VI)$$

worin Hal für ein Halogenatom und $R_{12}$ für eine Alkylgruppe mit 1 bis 6 Kohlenstoffatomen stehen und A' die vorstehend angegebene Bedeutung hat, umsetzt und die erhaltenen Ester der Formel VII

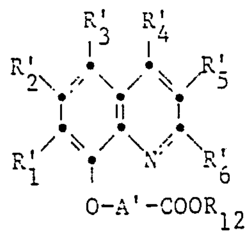

(VII)

worin $R_1'$, $R_2'$, $R_3'$, $R_4'$, $R_5'$, $R_6'$, A' und $R_{12}$ die vorstehend angegebenen Bedeutungen haben, mit Ammoniak in die entsprechenden Amide der Formel VIII

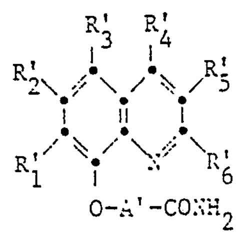

(VIII)

worin $R_1'$, $R_2'$, $R_3'$, $R_4'$, $R_5'$, $R_6'$ und A' die vorstehend angegebenen Bedeutungen haben, überführt und anschliessend dehydratisiert, und/oder

c) zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für Amidoxim, welches am Sauerstoffatom acyliert sein kann, steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Cyan steht, mit Hydroxylamin oder einem Säuresalz des Hydroxylamins umsetzt, und/oder

d) zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für acyliertes Amidoxim steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Amidoxim steht, acyliert.

So lassen sich beispielsweise Verbindungen der Formel Ia, in denen $R_1'$, $R_2'$, $R_3'$, $R_4'$, $R_5'$, $R_6'$ und A' die für Formel Ia angegebenen Bedeutungen haben und Z' für acyliertes Amidoxim der Formel

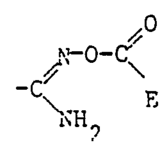

steht, wobei E $—R_7$, $—OR_8$, $—SR_9$ oder $—NR_{10}R_{11}$ ist, worin $R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist, $R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist, $R_{11}$ Wasserstoff, $C_1$-$C_8$-Alkyl oder $C_1$-$C_3$-Alkoxy oder $R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, in der Weise herstellen, dass man eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Amidoxim steht, mit einer Verbindung der Formel IX

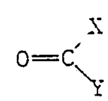

(IX)

worin X für ein Halogenatom und Y für $—R_7$, $—OR_8$, $—SR_9$ oder $—NR_{10}R_{11}$, wobei $R_7$, $R_8$, $R_9$, $R_{10}$ und $R_{11}$ die vorstehend angegebenen Bedeutungen haben, oder X und Y gemeinsam für die Iminogruppe $=N—R_{10}$ stehen, umsetzt.

Die Umsetzung (a) von Verbindungen der Formel II mit Verbindungen der Formel III kann bevorzugt in Gegenwart eines basischen Katalysators durchgeführt werden. Als Katalysatoren besonders geeignet sind Metallalkoholate, insbesondere Alkali- und Erdalkalimetallalkoholate, oder Hydroxyde, wie beispielsweise Natriumhydroxyd.

Die Umsetzung (b/i) von Verbindungen der Formel II mit Verbindungen der Formel IV wird bevorzugt in Methyläthylketon in Gegenwart von Kaliumcarbonat oder in Dimethylformamid in Gegenwart von Natriumhydrid vorgenommen, während die Umsetzung (b/ii) von Verbindungen der Formel II mit Verbindungen der Formel V am zweckmässigsten in einem Zweiphasensystem vorgenommen wird, wobei die eine Phase Wasser, die andere eine mit Wasser nicht mischbare Flüssigkeit, wie beispielsweise Toluol oder Methylenchlorid, darstellt. Als Katalysator dient bei diesen Umsetzungen ein Phasentransferkatalysator wie beispielsweise Benzyltriäthylammoniumchlorid.

In den Verbindungen der Formel IV steht Hal für Chlor, Brom, Fluor und Jod. Bevorzugt sind Chlor und Brom, wobei vorteilhafterweise Kaliumjodid als Katalysator eingesetzt wird.

In den Verbindungen der Formel VI steht Hal für Chlor, Brom, Jod und Fluor.

Die Dehydratisierung (b/iii) von Amiden der Formel VIII zu den entsprechenden Nitrilen lässt sich auf an sich bekannte Weise durchführen, beispielsweise mit Phosphorpentoxyd oder Phosphoroxychlorid.

Für die Umsetzung (c) von Nitrilen der Formel Ia mit Hydroxylamin oder Säuresalzen des Hydroxylamins kommen insbesondere Salze des Hydroxylamins mit anorganischen Säuren, vor allem Hydroxylaminhydrochlorid oder -sulfat, in Betracht, wobei die Umsetzung mit Säuresalzen zweckmässigerweise in Gegenwart einer Base durchgeführt wird, wie beispielsweise Alkali- oder Erdalkalimetallhydroxyden, beispielsweise Natriumhydroxyd, oder tertiären organischen Basen, beispielsweise tertiären Aminen wie Pyridin oder Trialkylamin.

In der Formel IX steht X für Chlor, Brom, Fluor oder Jod.

Die als Ausgangsprodukte zu verwendenden Chinoline und Chinaldine sind bekannt oder lassen sich analog bekannten Verfahren herstellen.

Die bekannten Verbindungen der Formel I, welche nicht durch die Formel Ia umfasst sind, lassen sich nach den für Verbindungen der Formel Ia beschriebenen Methoden herstellen.

Die nachfolgenden Beispiele dienen zur Illustration der Erfindung.

## Herstellungsbeispiele für Wirkstoffe

### Beispiel 1 : 2-Methyl-5,7-dichlor-8-(cyanomethoxy)-chinolin (Verbindung Nr. 18)

10,7 g 5,7 Dichlor-8-hydroxychinaldin werden in der Wärme in 150 ml 2-Butanon gelöst, portionenweise mit 10,4 g Kaliumcarbonat versetzt und eine Stunde unter Rückfluss erhitzt. Nach der Zugabe von 1 g Kaliumjodid werden unter Rühren und Kochen unter Rückfluss 7,1 g Chloracetonitril in 30 ml 2-Butanon zugetropft und anschliessend 3 Stunden bei einer Innentemperatur von 75 °C erwärmt. Das erhaltene Reaktionsgemisch wird nach Abkühlen auf Raumtemperatur mit 1 L Wasser versetzt, filtriert, der Rückstand mit Wasser nachgewaschen, getrocknet und aus Chloroform/Petroläther (40-60 °C) umkristallisiert. Man erhält 2-Methyl-5,7-dichlor-8-(cyanomethoxy)-chinolin. Smp. 157-158 °C.

### Beispiel 2 : 2-(8-Chinolinoxy)-acetamidoxim (Verbindung Nr. 2)

Zu 15,8 g 8-(Cyanomethoxy)-chinolin in 100 ml Aethanol wird eine Lösung von 6,4 g Hydroxylaminhydrochlorid in 10 ml Wasser und 6,4 g Kaliumcarbonat in 10 ml Wasser bei Raumtemperatur innerhalb von 15 Minuten zugetropft, wobei sich das Reaktionsgemisch auf 30 °C erwärmt. Nach dreistündigem Rühren bei Raumtemperatur wird das Reaktionsgemisch mit 250 ml Wasser verdünnt, filtriert, mit Wasser nachgewaschen und getrocknet. Man erhält 2-(8-Chinolinoxy)-acetamidoxim als hellbraunes Pulver. Smp. 201-204 °C (Zersetzung).

### Beispiel 3 : O-(Isopropylaminocarbonyl)-2-(5-chlor-7-brom-8-chinolinoxy)-acetamidoxim (Verbindung Nr. 14)

8,6 g 2-(5-Chlor-7-brom-8-chinolinoxy)-acetamidoxim in 100 ml Acetonitril werden unter Rühren bei 65 °C innerhalb 15 Minuten mit 3,3 g Isopropylisocyanat und 0,1 g 1,4-Diazabicyclo [2,2,2] octan versetzt und anschliessend zwei Stunden bei 60 °C erwärmt. Nach dem Abkühlen auf Raumtemperatur wird das Reaktionsgemisch filtriert, mit wenig Acetonitril nachgewaschen und getrocknet. Man erhält O-(Isopropylaminocarbonyl)-2-(5-chlor-7-brom-8-chinolinoxy)-acetamidoxim in Form weisser Kristalle. Smp. 162-165 °C.

Analog einer der vorstehend beschriebenen Methoden lassen sich auch die folgenden, in der Tabelle 1 zusammen mit den Verbindungen der vorstehenden Beispiele aufgeführten Verbindungen der Formeln I und Ia herstellen :

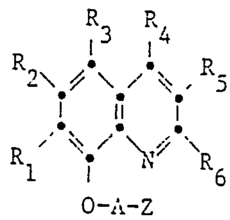

Tabelle 1

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 1 | H | H | H | H | H | H | $-CH_2-$ | $-CN$ | 118-119°C |
| 2 | H | H | H | H | H | H | $-CH_2-$ | 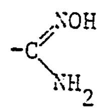 | 201-204°C (Z) |
| 3 | H | H | H | H | H | $CH_3$ | $-CH_2-$ | $-CN$ | 114-116°C |
| 4 | H | H | H | H | H | $CH_3$ | $-CH_2-$ | 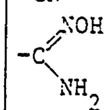 | 209-210°C (Z) |
| 5 | H | H | Cl | H | H | H | $-CH_2-$ | 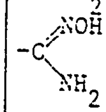 | 203-205°C (Z) |
| 6 | H | H | H | H | H | H | $-CH_2-$ | 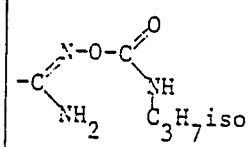 | 136-138°C |
| 7 | H | H | Cl | H | H | H | $-CH_2-$ | $-CN$ | 159-160°C |
| 8 | H | H | H | H | H | H | $-CH_2-$ | 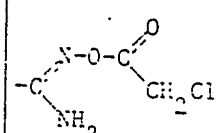 | 129-130°C |
| 9 | Br | H | Cl | H | H | H | $-CH_2-$ | 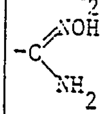 | 197-198°C (Z) |
| 10 | Br | H | Cl | H | H | H | $-CH_2-$ | $-CN$ | 150-151°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 11 | H | H | H | H | H | H | $-CH_2-$ | 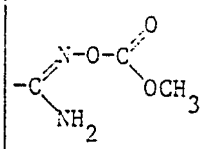 | 143-145°C |
| 12 | J | H | Cl | H | H | H | $-CH_2-$ | 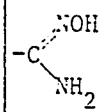 | 195-196°C (Z) |
| 13 | J | H | Cl | H | H | H | $-CH_2-$ | -CN | 141-143°C |
| 14 | Br | H | Cl | H | H | H | $-CH_2-$ | 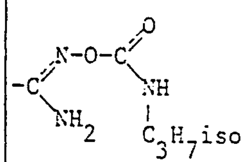 | 162-165°C |
| 15 | Cl | H | Cl | H | H | $CH_3$ | $-CH_2-$ | 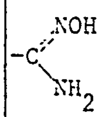 | 205-207°C (Z) |
| 16 | Cl | H | Cl | H | H | H | $-CH_2-$ | -CN | 150-152°C |
| 17 | J | H | Cl | H | H | H | $-CH_2-$ | 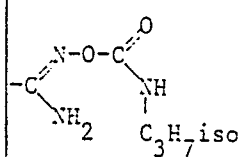 | 163-167°C |
| 18 | Cl | H | Cl | H | H | $CH_3$ | $-CH_2-$ | -CN | 157-158°C |
| 19 | Cl | H | Cl | H | H | $CH_3$ | $-CH_2-$ | 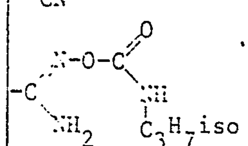 | 149-152°C |
| 20 | H | H | H | H | H | H | $-CH_2-CH_2-$ | -CN | 108-112°C |

Tabelle 1 (Fortsetzung)

TABLE

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 21 | H | H | H | $CH_3$ | H | H | $-CH_2-$ | $-CN$ | |
| 22 | H | H | H | H | H | H | $CH_3$<br>$-CH-$ | $-CN$ | 121-124°C |
| 23 | H | H | $CH_3$ | H | H | H | $-CH_2-$ | $-CN$ | |
| 24 | H | H | H | H | H | H | $-CH_2$<br>$CH_2-$ | NOH<br>$-C$<br>$NH_2$ | 186-189°C |
| 25 | H | H | H | H | H | $CH_3$ | $CH_3$<br>$-CH-$ | $-CN$ | |
| 26 | H | H | $C_2H_5$ | H | H | H | $-CH_2-$ | $-CN$ | |
| 27 | H | H | Br | H | H | H | $-CH_2-$ | NOH<br>$-C$<br>$NH_2$ | |
| 28 | H | H | H | H | H | $CH_3$ | $-CH_2$<br>$CH_2-$ | $-CN$ | |
| 29 | H | H | H | Br | H | H | $-CH_2-$ | $-CN$ | |
| 30 | H | H | H | H | H | $CH_3$ | $-CH_2$<br>$CH_2-$ | NOH<br>$-C$<br>$NH_2$ | |
| 31 | H | H | Cl | H | H | H | $CH_3$<br>$-CH-$ | $-CN$ | 143-145°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 32 | H | H | J | H | H | H | $-CH_2-$ | 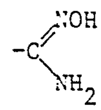 | |
| 33 | H | H | Br | H | H | H | 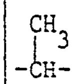 | -CN | |
| 34 | H | H | Br | H | H | $CH_3$ | $-CH_2-$ | 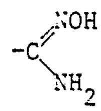 | |
| 35 | H | H | H | H | H | H | 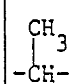 | 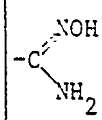 | 191-194°C (Z) |
| 36 | H | H | F | H | H | H | $-CH_2-$ | -CN | |
| 37 | H | H | Cl | H | H | $CH_3$ | $-CH_2-$ | 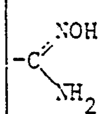 | |
| 38 | H | H | Br | H | H | $CH_3$ | 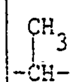 | -CN | |
| 39 | H | H | H | H | H | $CH_3$ | 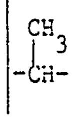 | 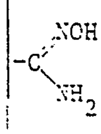 | |
| 40 | H | H | Br | H | H | H | $-CH_2-$ | -CN | |
| 41 | Cl | H | Br | H | H | H | $-CH_2-$ | 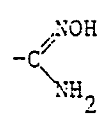 | |
| 42 | H | H | J | H | H | H | $-CH_2-$ | -CN | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 43 | H | H | Cl | H | H | $CH_3$ | 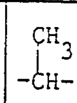 | -CN | |
| 44 | H | H | Br | H | H | $CH_3$ | $-CH_2-$ | -CN | |
| 45 | Cl | H | Br | H | H | H | 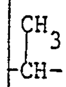 | -CN | |
| 46 | H | H | Cl | H | H | $CH_3$ | $-CH_2-$ | -CN | |
| 47 | H | H | Cl | H | H | H | 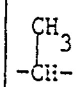 | 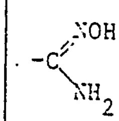 | 186-189°C (Z) |
| 48 | Cl | H | Br | H | H | $CH_3$ | $-CH_2-$ | 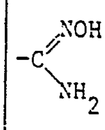 | |
| 49 | H | H | J | H | H | $CH_3$ | $-CH_2-$ | -CN | |
| 50 | H | H | Br | H | H | H | 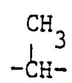 | 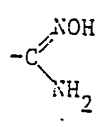 | |
| 51 | Cl | H | Br | H | H | H | $-CH_2-$ | -CN | |
| 52 | Br | H | Cl | H | H | H | 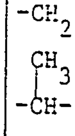 | -CN | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 53 | Br | H | Cl | H | H | $CH_3$ | $-CH_2-$ | 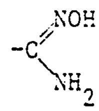 | |
| 54 | Cl | H | Br | H | H | $CH_3$ | $-CH_2-$ | -CN | |
| 55 | H | H | Br | H | H | $CH_3$ | 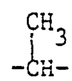 | 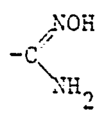 | |
| 56 | Br | H | Cl | H | H | $CH_3$ | $-CH_2-$ | -CN | |
| 57 | J | H | Cl | H | H | H | 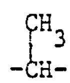 | -CN | |
| 58 | J | H | Br | H | H | H | $-CH_2-$ | -CN | |
| 59 | H | H | Cl | H | H | $CH_3$ | 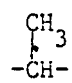 | 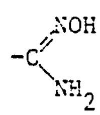 | |
| 60 | Br | H | J | H | H | H | $-CH_2-$ | -CN | |
| 61 | H | H | $NO_2$ | H | H | H | 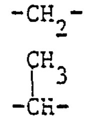 | -CN | 154-156°C |
| 62 | Br | H | $NO_2$ | H | H | H | $-CH_2-$ | -CN | |
| 63 | J | H | Cl | H | H | $CH_3$ | $-CH_2-$ | 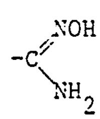 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 64 | Cl | H | J | H | H | H | $-CH_2-$ | -CN | |
| 65 | Cl | H | $NO_2$ | H | H | H | $-CH_2-$ | 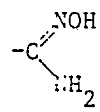 | 214-216°C (Z) |
| 66 | J | H | Cl | H | H | $CH_3$ | $-CH_2-$ | -CN | |
| 67 | Br | H | Br | H | H | H | $-CH_2-$ | 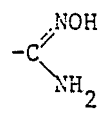 | |
| 68 | Cl | H | H | H | H | $CH_3$ | $-CH_2-$ | -CN | |
| 69 | Cl | H | Br | H | H | H | 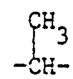 | 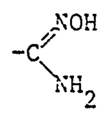 | |
| 70 | Cl | H | $NO_2$ | H | H | H | $-CH_2-$ | -CN | 166-169°C |
| 71 | Cl | H | Cl | H | H | H | $-CH_2-$ | 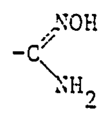 | |
| 72 | Cl | H | $C_3H_7$-n | H | H | H | $-CH_2-$ | -CN | |
| 73 | Br | H | Br | H | H | H | $-CH_2-$ | -CN | |
| 74 | Br | H | Br | H | H | $CH_3$ | $-CH_2-$ | -CN | |
| 75 | Br | H | Cl | H | H | H | 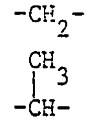 | 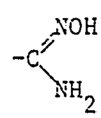 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 76 | J | H | J | H | H | H | $-CH_2-$ | 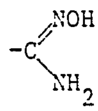 | |
| 77 | H | H | H | H | H | H | $-CH_2-$ | 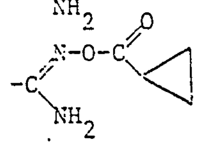 | 165-166°C |
| 78 | J | H | J | H | H | H | $-CH_2-$ | -CN | |
| 79 | H | H | H | H | H | H | $-CH_2-$ | 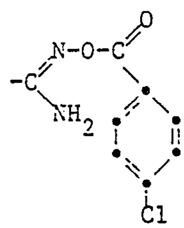 | 139-141°C |
| 80 | J | H | J | H | H | $CH_3$ | $-CH_2-$ | -CN | |
| 81 | H | H | H | H | H | H | $-CH_2-$ | 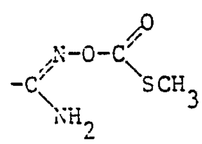 | |
| 82 | $NO_2$ | H | $NO_2$ | H | H | H | $-CH_2-$ | -CN | |
| 83 | $NO_2$ | H | $NO_2$ | H | H | $CH_3$ | $-CH_2-$ | 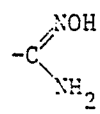 | |
| 84 | J | H | F | H | H | H | $-CH_2-$ | -CN | |
| 85 | H | H | Cl | H | H | H | $-CH_2-$ | 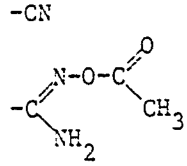 | 141-143°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 86 | J | H | $NO_2$ | H | H | $CH_3$ | $-CH_2-$ | $-CN$ | |
| 87 | H | H | Cl | H | H | H | $-CH_2-$ | 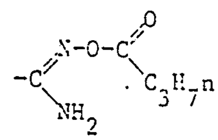 | |
| 88 | $NO_2$ | H | $NO_2$ | H | H | $CH_3$ | $-CH_2-$ | $-CN$ | |
| 89 | H | H | Cl | H | H | H | $-CH_2-$ | 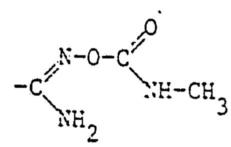 | |
| 90 | H | H | $NO_2$ | H | H | H | $-CH_2-$ | $-CN$ | 162-164°C |
| 91 | J | H | Cl | H | H | H | 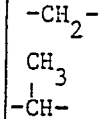 | 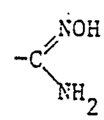 | |
| 92 | H | H | $NO_2$ | H | H | H | $-CH_2-$ | 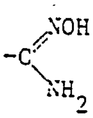 | 212-215°C (Z) |
| 93 | H | H | $NO_2$ | H | H | $CH_3$ | $-CH_2-$ | $-CN$ | |
| 94 | H | H | Cl | H | H | H | $-CH_2-$ | 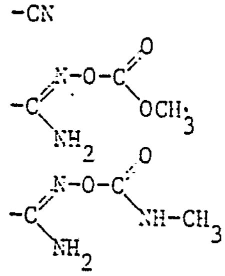 | 148-149°C |
| 95 | H | H | H | H | H | $CH_3$ | $-CH_2-$ | | |
| 96 | H | H | $NO_2$ | H | H | H | 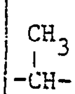 | 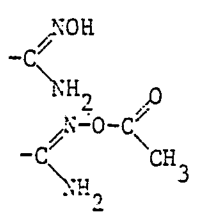 | |
| 97 | H | H | H | H | H | $CH_3$ | $-CH_2-$ | | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 98 | H | H | Cl | H | H | H | $-CH_2-$ | 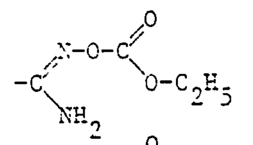 | 139-140°C |
| 99 | H | H | H | H | H | H | $-CH_2-$ | 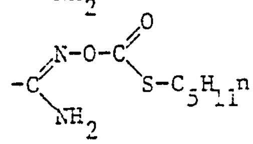 | 111-114°C |
| 100 | H | H | H | H | H | H | $-CH_2-$ | 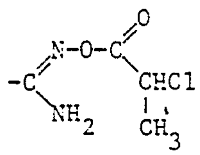 | |
| 101 | H | H | H | H | H | H | $-CH_2-$ | 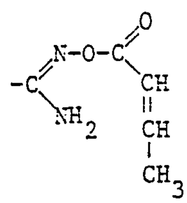 | 158-162°C |
| 102 | H | H | H | H | H | H | $-CH_2-$ | 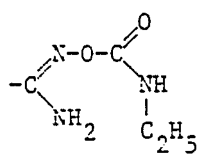 | 123-125°C |
| 103 | H | H | H | H | H | H | $-CH_2-$ | 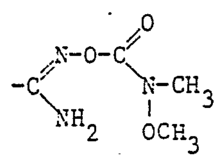 | 138-139°C |
| 104 | H | H | H | H | H | H | $-CH_2-$ | 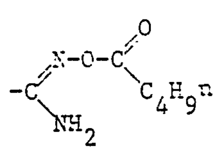 | 120-122°C |
| 105 | H | H | Cl | H | H | H | $-CH_2-$ | 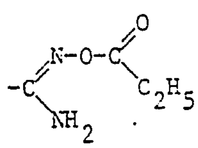 | 157-158°C (Z) |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 106 | H | H | H | H | H | H | $-CH_2-$ | 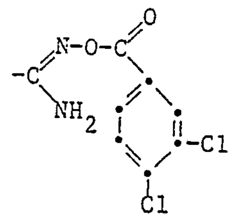 | |
| 107 | H | H | Cl | H | H | H | $-CH_2-$ | 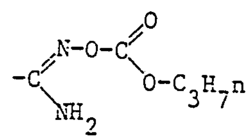 | |
| 108 | H | H | H | H | H | H | $-CH_2-$ | 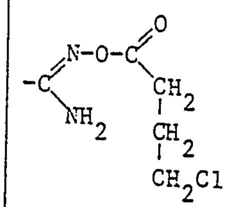 | 144-146°C |
| 109 | H | H | Cl | H | H | H | $-CH_2-$ | 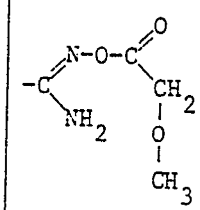 | |
| 110 | H | H | H | H | H | H | $-CH_2-$ | 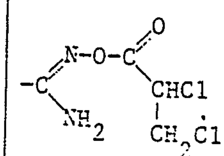 | 112-114°C |
| 111 | H | H | Cl | H | H | H | $-CH_2-$ | 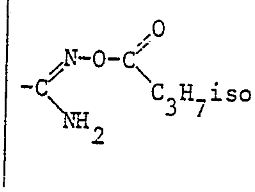 | 173-174°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 112 | H | H | H | H | H | H | $-CH_2-$ | 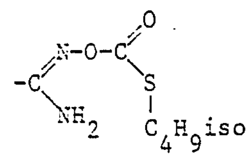 | |
| 113 | H | H | Cl | H | H | H | $-CH_2-$ | 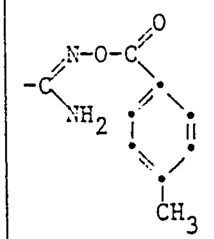 | |
| 114 | H | H | H | H | H | H | $-CH_2-$ | 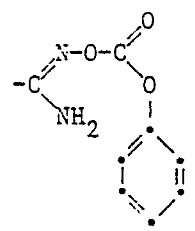 | 155-156°C |
| 115 | H | H | H | H | H | H | $-CH_2-$ | 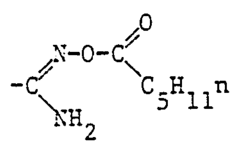 | |
| 116 | H | H | H | H | H | H | $-CH_2-$ | 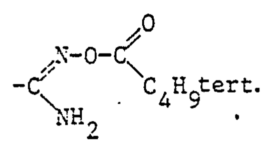 | 107-110,5°C |
| 117 | H | H | H | H | H | H | $-CH_2-$ | 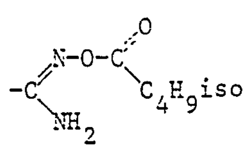 | 124-126°C |
| 118 | H | H | H | H | H | H | $-CH_2-$ | 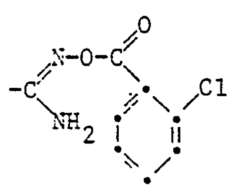 | 131-132°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 119 | H | H | Cl | H | H | H | $-CH_2-$ | 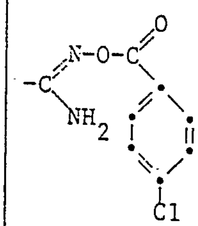 | |
| 120 | H | H | H | H | H | H | $-CH_2-$ | 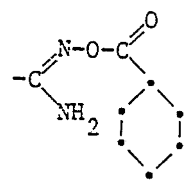 | |
| 121 | H | H | H | H | H | H | $-CH_2-$ | 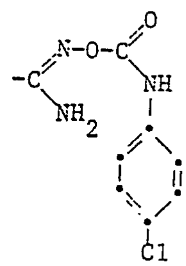 | |
| 122 | H | H | H | H | H | H | $-CH_2-$ | 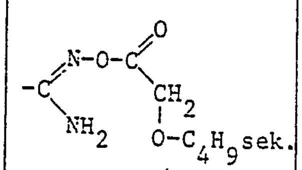 | 84-86°C |
| 123 | H | H | H | H | H | H | $-CH_2-$ | 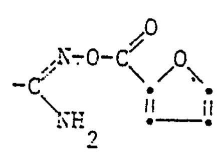 | 168-169°C |
| 124 | H | H | H | H | H | H | $-CH_2-$ | 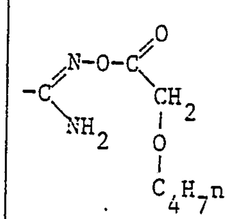 | 100-103°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 125 | H | H | H | H | H | H | $-CH_2-$ | 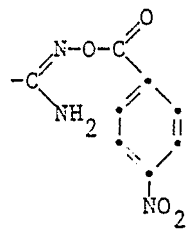 | |
| 126 | H | H | Cl | H | H | H | $-CH_2-$ | 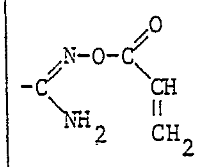 | |
| 127 | H | H | Cl | H | H | H | $-CH_2-$ | 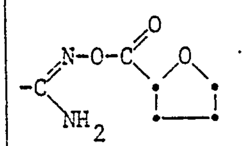 | 156-157°C (Z) |
| 128 | H | H | H | H | H | H | $-CH_2-$ | 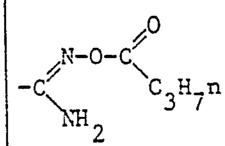 | 82-85°C |
| 129 | H | H | H | H | H | H | $-CH_2-$ | 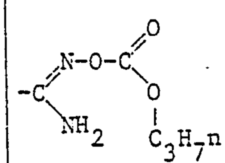 | 144-147°C |
| 130 | H | H | H | H | H | H | $-CH_2-$ | 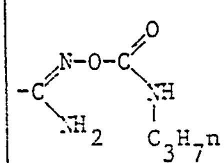 | |
| 131 | H | H | H | H | H | H | $-CH_2-$ | 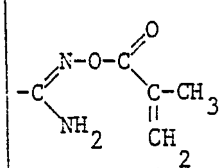 | 128-130°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 132 | H | H | H | H | H | H | $-CH_2-$ | 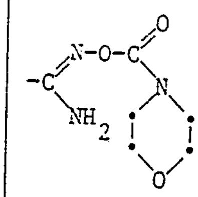 | |
| 133 | H | H | H | H | H | H | $-CH_2-$ | 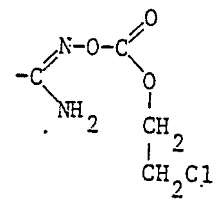 | |
| 134 | H | H | H | H | H | H | $-CH_2-$ | 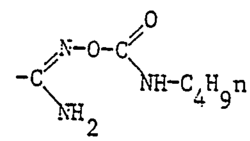 | 90-92°C |
| 135 | H | H | Cl | H | H | H | $-CH_2-$ | 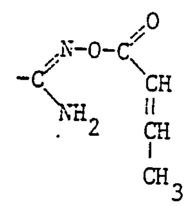 | |
| 136 | H | H | Cl | H | H | H | $-CH_2-$ | 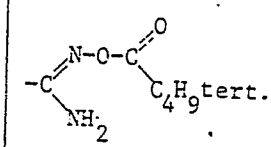 | |
| 137 | H | H | H | H | H | H | $-CH_2-$ | 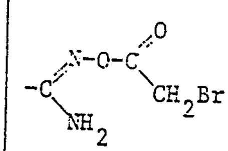 | 132-134°C |
| 138 | H | H | H | H | H | H | $-CH_2-$ | 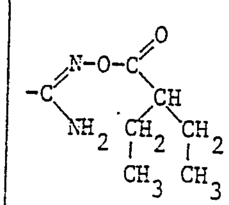 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 139 | H | H | H | H | H | H | $-CH_2-$ | 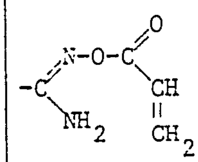 | 138-140°C |
| 140 | H | H | H | H | H | H | $-CH_2-$ | 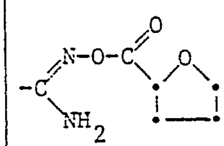 | 129-131°C |
| 141 | H | H | H | H | H | H | $-CH_2-$ | 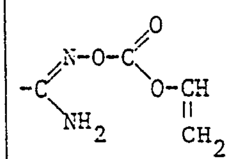 | |
| 142 | H | H | H | H | H | H | $-CH_2-$ | 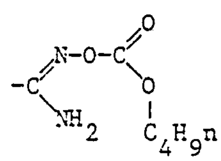 | 121-123°C |
| 143 | H | H | H | H | H | H | $-CH_2-$ | 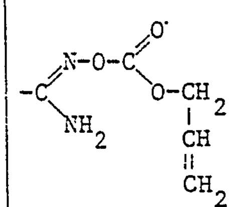 | 123-125°C |
| 144 | H | H | Cl | H | H | H | $-CH_2-$ | 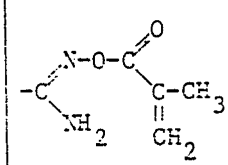 | |
| 145 | H | H | H | H | H | H | $-CH_2-$ | 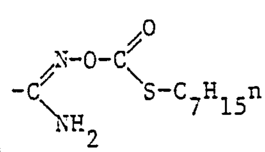 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 146 | H | H | H | H | H | H | $-CH_2-$ | 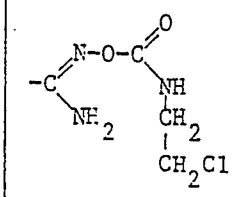 | |
| 147 | H | H | H | H | H | H | $-CH_2-$ | 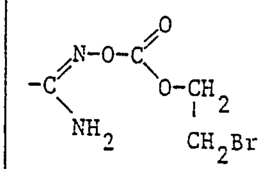 | 127-128°C (Z) |
| 148 | H | H | H | H | H | H | $-CH_2-$ | 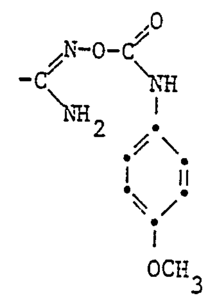 | |
| 149 | H | H | Cl | H | H | H | $-CH_2-$ | 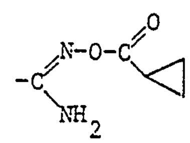 | 173-175°C |
| 150 | H | H | H | H | H | H | $-CH_2-$ | 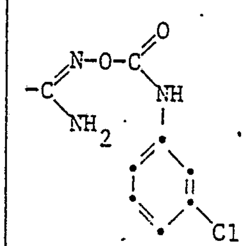 | |
| 151 | H | H | H | H | H | H | $-CH_2-$ | 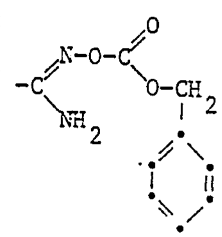 | 135-137°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 152 | H | H | Cl | H | H | H | $-CH_2-$ | 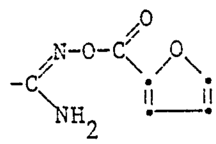 | 191-192°C (Z) |
| 153 | H | H | H | H | H | H | $-CH_2-$ | 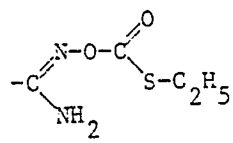 | 120-121°C |
| 154 | H | H | H | H | H | H | $-CH_2-$ | 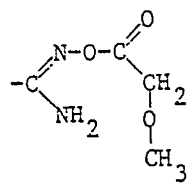 | 118-120°C |
| 155 | H | H | Cl | H | H | H | $-CH_2-$ | 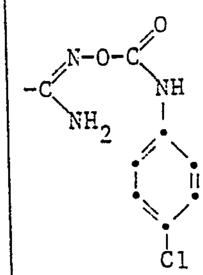 | 191-192°C (Z) |
| 156 | H | H | H | H | H | H | $-CH_2-$ | 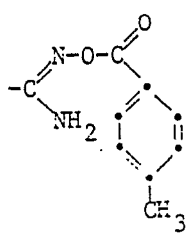 | |
| 157 | H | H | Cl | H | H | H | $-CH_2-$ | 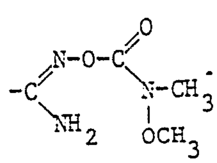 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 158 | H | H | H | H | H | H | $-CH_2-$ | 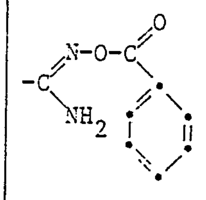 | 158-159°C |
| 159 | H | H | Cl | H | H | H | $-CH_2-$ | 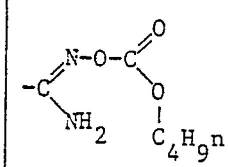 | |
| 160 | H | H | H | H | H | H | $-CH_2-$ | 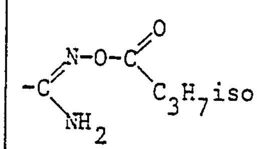 | 115-117,5°C |
| 161 | H | H | H | H | H | H | $-CH_2-$ | 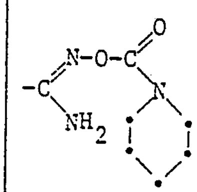 | |
| 162 | H | H | H | H | H | H | $-CH_2-$ | 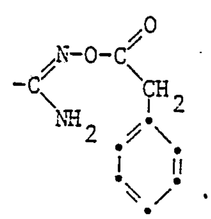 | 140-142°C |
| 163 | H | H | Cl | H | H | H | $-CH_2-$ | 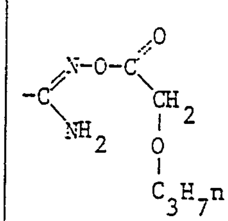 | |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 164 | H | H | H | H | H | H | $-CH_2-$ | 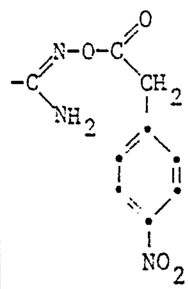 | |
| 165 | H | H | H | H | H | H | $-CH_2-$ | 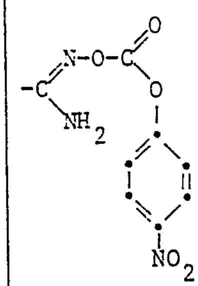 | |
| 166 | H | H | H | H | H | H | $-CH_2-$ | 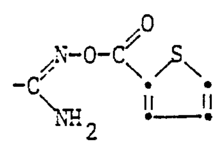 | 164-165°C |
| 167 | H | H | H | H | H | H | $-CH_2-$ | 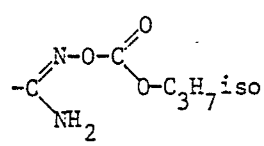 | |
| 168 | H | H | Cl | H | H | H | $-CH_2-$ | 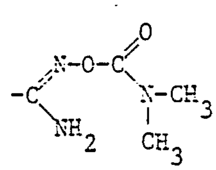 | |
| 169 | H | H | H | H | H | H | $-CH_2-$ | 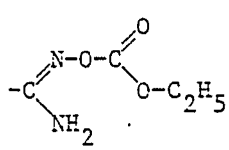 | 129-132°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 170 | H | H | H | H | H | H | $-CH_2-$ | 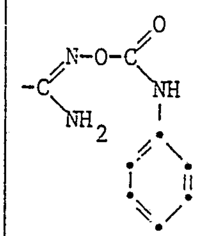 | 155-157,5°C |
| 171 | H | H | H | H | H | H | $-CH_2-$ | 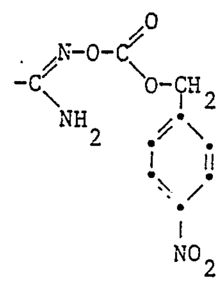 | |
| 172 | H | H | H | H | H | H | $-CH_2-$ | 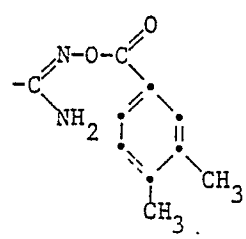 | |
| 173 | H | H | Cl | H | H | H | $-CH_2-$ | 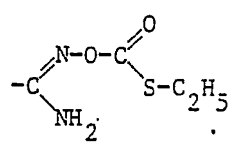 | |
| 174 | H | H | H | H | H | H | $-CH_2-$ | 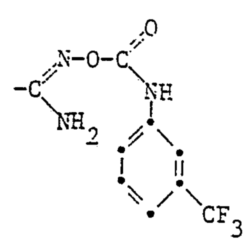 | 158-160°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 175 | H | H | H | H | H | H | $-CH_2-$ | 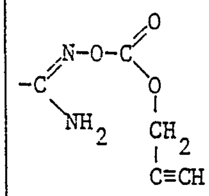 | |
| 176 | H | H | Cl | H | H | H | $-CH_2-$ | 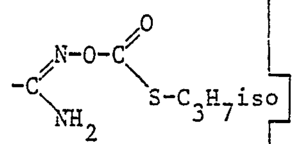 | |
| 177 | H | H | Cl | H | H | H | $-CH_2-$ | 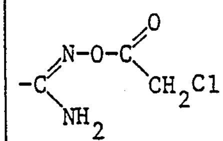 | 155-158°C (Z) |
| 178 | H | H | H | H | H | H | $-CH_2-$ | 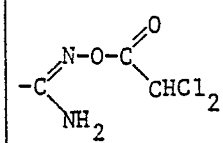 | |
| 179 | H | H | H | H | H | H | $-CH_2-$ | 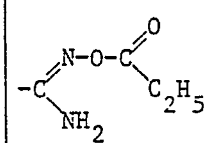 | 144-146°C |
| 180 | H | H | H | H | H | H | $-CH_2-$ | 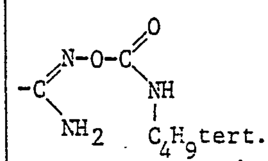 | |
| 181 | H | H | H | H | H | H | $-CH_2-$ | 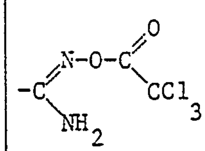 | |
| 182 | H | H | H | H | H | H | $-CH_2-$ | 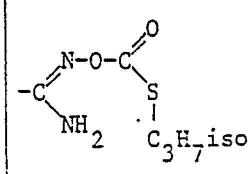 | 123-124°C |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 183 | H | H | H | H | H | H | $-CH_2-$ | 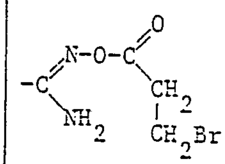 | |
| 184 | H | H | H | H | H | H | $-CH_2-$ | 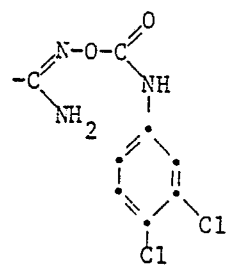 | |
| 185 | H | H | H | H | H | H | $-CH_2-$ | 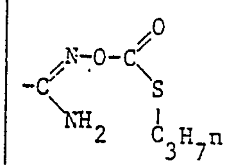 | |
| 186 | H | H | H | H | H | H | $-CH_2-$ | 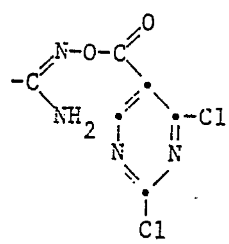 | 173-176°C (Z) |
| 187 | H | H | H | H | H | H | $-CH_2-$ | 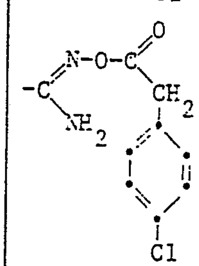 | |
| 188 | H | H | H | H | H | H | $-CH_2-$ | 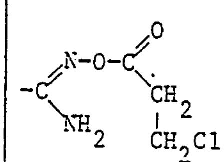 | 134-136°C (Z) |

Tabelle 1 (Fortsetzung)

| Nr. | $R_1$ | $R_2$ | $R_3$ | $R_4$ | $R_5$ | $R_6$ | A | Z | physikalische Daten; Smp. |
|---|---|---|---|---|---|---|---|---|---|
| 189 | H | H | H | H | H | H | $-CH_2-$ | 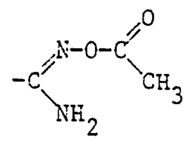 | 100-102°C |
| 190 | H | H | H | H | H | H | $-CH_2-$ | 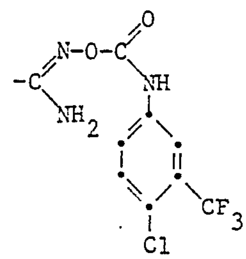 | |
| 191 | H | H | H | H | H | H | $-CH_2-$ | 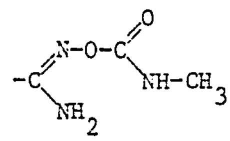 | |
| 192 | H | H | H | H | H | H | $-CH_2-$ | 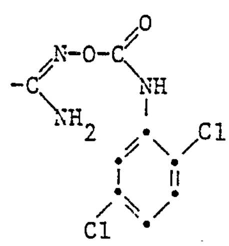 | 197-199°C |
| 193 | H | H | Cl | H | H | H | $-CH_2-$ | 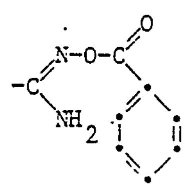 | |
| 194 | H | H | Cl | H | H | H | $-CH_2-$ | 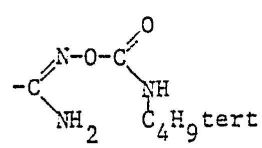 | |
| 195 | H | H | H | H | H | H | $-CH_2-$ | 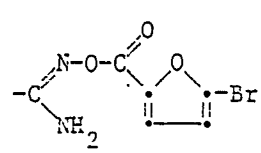 | 170-171°C |

Formulierungsbeispiele für flüssige Wirkstoffe der Formel I

(% = Gewichtsprozent)

4. Emulsions-Konzentrate

| | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyäthylenglykoläther (36 Mol AeO) | 5 % | - | - |
| Tributylphenol-polyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

5. Lösungen

| | a) | b) | c) | d) |
|---|---|---|---|---|
| Wirkstoff aus Tabelle 1 | 80 % | 10 % | 5 % | 95 % |
| Aethylenglykol-monomethyl-äther | 20 % | - | - | - |
| Polyäthylenglykol M G 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

6. Granulate

| | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

7. Stäubemittel

| | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

8. Spritzpulver

| | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

9. Emulsions-Konzentrat

| | |
|---|---|
| Wirkstoff aus Tabelle 1 | 10 % |
| Octylphenolpolyäthylenglykoläther (4-5 Mol AeO) | 3 % |
| Ca-Dodecylbenzolsulfonat | 3 % |
| Ricinusölpolyglykoläther (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

10. Stäubemittel

| | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

11. Extruder Granulat

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

12. Umhüllungs-Granulat

| | |
|---|---|
| Wirkstoff aus Tabelle 1 | 3 % |
| Polyäthylenglykol (M G 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

13. Suspensions-Konzentrat

| | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel 14 : Tankmischung im Nachauflaufverfahren in Gerste und Weizen

Gersten- oder Weizensamen werden in Plastiktöpfe, die 0,5 l Erde enthalten, im Gewächshaus ausgesät. Nach dem Auflaufen der Pflanzen bis zum 2- bis 3-Blattstadium wird die als Antidot zu prüfende Substanz zusammen mit dem Herbizid 2-[4-(3,5-Dichlorpyridyl-2-oxy)-phenoxy]-propionsäure-2-propinylester als Tankmischung appliziert. 20 Tage nach der Applikation wird die Schutzwirkung des Antidots in Prozent bonitiert. Als Referenzen dienen dabei die mit dem Herbizid allein behandelten Pflanzen sowie die vollständig unbehandelte Kontrolle. Die Ergebnisse zeigen die folgenden Tabellen :

Tabelle 2 : Versuchsergebnis in Gerste

| Antidot Verbindung Nr. | Antidot kg AS/ha | Herbizid kg AS/ha | Relative Schutzwirkung in % |
|---|---|---|---|
| 7 | 0,5 | 0,5 | 38 |
| 13 | 0,5 | 0,5 | 25 |

Tabelle 3 : Versuchsergebnis in Weizen

| Antidot Verbindung Nr. | Antidot kg AS/ha | Herbizid kg AS/ha | Relative Schutzwirkung in % |
|---|---|---|---|
| 1 | 1,5 | 0,75 | 38 |
| 2 | 1,5 | 0,75 | 50 |
| 3 | 1,5 | 0,75 | 50 |
| 4 | 1,5 | 0,75 | 38 |
| 5 | 1,5 | 0,75 | 50 |
| 6 | 1,5 | 0,75 | 50 |
| 7 | 1,5 | 0,75 | 38 |
| 8 | 1,5 | 0,75 | 50 |
| 9 | 1,5 | 0,75 | 25 |
| 10 | 1,5 | 0.75 | 50 |
| 11 | 1,5 | 0,75 | 50 |
| 12 | 1,5 | 0,75 | 50 |
| 13 | 1,5 | 0,75 | 50 |
| 15 | 1,5 | 0,75 | 50 |
| 19 | 1,5 | 0,75 | 38 |

Beispiel 15 : Samenquellung Reis, Herbizid im Vorauflaufverfahren

Reissamen werden 48 Stunden mit Lösungen der als Antidot zu prüfenden Substanz in einer Konzentration von 100 ppm getränkt. Man lässt die Samen dann etwa zwei Stunden trocknen, bis sie nicht mehr kleben. Plastikbehälter (Länge × Breite × Höhe = 25 × 17 × 12 cm) werden bis 2 cm unter dem Rand mit sandigem Lehm gefüllt. Die vorgequollenen Samen werden auf die Bodenoberfläche des Behälters gesät und nur ganz schwach mit Erde bedeckt. Die Erde wird in einem feuchten (nicht sumpfigen) Zustand gehalten. Nun wird das Herbizid 2-Chlor-2',6'-diäthyl-N-[2"-(n-propoxy)-äthyl]-acetanilid in verdünnter Lösung auf die Bodenoberfläche versprüht. Der Wasserstand wird entsprechend dem Wachstum der Pflanen sukzessive erhöht. 18 Tage nach der Herbizidapplikation wird die Schutzwirkung des Antidots in Prozent bonitiert. Als Referenz dienen dabei die mit dem Herbizid allein behandelten Pflanzen sowie die vollständig unbehandelte Kontrolle. Die Ergebnisse zeigt die folgende Tabelle :

Tabelle 4

| Antidot Verbindung Nr. | Antidot ppm | Herbizid kg AS/ha | Relative Schutzwirkung in % |
|---|---|---|---|
| 1 | 100 | 0,25 | 50 |
| 8 | 100 | 0,25 | 38 |

Beispiel 16 : Tankmischung im Vorauflaufverfahren in Soja

Töpfe (oberer Durchmesser 6 cm) werden mit sandiger Lehmerde gefüllt und Sojasamen der Sorte « Hark » eingesät. Nach dem Bedecken der Samen mit Erde wird die als Antidot zu prüfende Substanz zusammen mit dem Herbizid 4-Amino-6-tert.butyl-4,5-dihydro-3-methylthio-1,2,4-triazin-5-on in verdünnter Lösung als Tankmischung auf die Bodenoberfläche versprüht. 21 Tage nach der Herbizidapplikation wird die Schutzwirkung des Antidots in Prozent bonitiert. Als Referenzen dienen dabei die mit Herbizid allein behandelten Pflanzen sowie die vollständig unbehandelte Kontrolle. Ein Ergebnis zeigt die folgende Tabelle :

Tabelle 5

| Antidot Verbindung Nr. | Antidot kg AS/ha | Herbizid kg AS/ha | Relative Schutzwirkung in % |
|---|---|---|---|
| 17 | 1,5 | 0,75 | 38 |

Beispiel 17 : Saatbeizung Mais, Herbizid im Nachauflaufverfahren

Maissamen der Sorte « LG 5 » werden mit der als Antidot zu prüfenden Substanz in einen Glasbehälter gegeben und durch Schütteln und Rotation gut durchgemischt. Plastiktöpfe (oberer Durchmesser 11 cm) werden mit Erde gefüllt und die gebeizten Samen eingesät. Nach dem Bedecken der Samen mit Erde wird das Herbizid N-[2-(2-butenyloxy)-phenyl-sulfonyl]-N'-(4-methoxy-6-methyl-1,3,5-triazin-2-yl)-harnstoff im Nachauflaufverfahren appliziert. 18 Tage nach der Herbizidapplikation wird die Schutzwirkung des Antidots in Prozent bonitiert. Als Referenz dienen dabei die mit dem Herbizid allein behandelten Pflanzen sowie die vollständig unbehandelte Kontrolle. Die Ergebnisse zeigt die folgende Tabelle :

Tabelle 6

| Herbizid kg AS/ha | 1,5 | | | 1,0 | | | 0,5 | | |
|---|---|---|---|---|---|---|---|---|---|
| Antidot Verbindung Nr. 7 g AS/kg Samen | 4 | 2 | 1 | 4 | 2 | 1 | 4 | 2 | 1 |
| Relative Schutzwirkung in % | 25 | 38 | 38 | 50 | 63 | 50 | 25 | 25 | 25 |

Beispiel 18 : Saatbeizung Mais, Herbizid im Vorauflaufverfahren

Maissamen der Sorte « LG 5 » werden mit der als Antidot zu prüfenden Substanz in einen Glasbehälter gegeben und durch Schütteln und Rotation gut durchgemischt. Plastiktöpfe (oberer Durchmesser 11 cm) werden mit Erde gefüllt und die gebeizten Samen eingesät. Nach dem Bedecken der Samen mit Erde wird das Herbizid N-[2-(2-butenyloxy)-phenyl-sulfonyl]-N'-(4-methoxy-6-methyl-1,3,5-triazin-2-yl)-harnstoff im Vorauflaufverfahren appliziert. 18 Tage nach der Herbizidapplikation wird die Schutzwirkung des Antidots in Prozent bonitiert. Als Referenz dienen dabei die mit dem Herbizid allein behandelten Pflanzen sowie die vollständig unbehandelte Kontrolle. Ein Ergebnis zeigt die folgende Tabelle :

Tabelle 7

| Antidot Verbindung Nr. | Antidot g AS/kg Samen | Herbizid kg AS/ha | Relative Schutzwirkung in % |
|---|---|---|---|
| 7 | 1 | 1,0 | 25 |

Beispiel 19 : Saatbeizung in Gerste, Herbizid im Vorauflaufverfahren

Gerstensamen werden mit der als Antidot zu prüfenden Substanz in einen Glasbehälter gegeben und durch Schütteln und Rotation gut durchgemischt. Plastikbehälter (Länge × Breite × Höhe = 25 × 17 × 12 cm) werden mit sandiger Lehmerde gefüllt und die gebeizten Samen eingesät. Nach dem Bedecken des Samens mit Erde wird das Herbizid N-(2-chlorphenylsulfonyl)-N'-(4-methoxy-6-methyl-1,3,5-triazin-2-yl)-harnstoff auf die Bodenoberfläche gesprüht. 21 Tage nach der Herbizidapplikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die nachfolgende Tabelle :

Tabelle 8

| Antidot Verbindung Nr. | Antidot g AS/kg Samen | Herbizid kg AS/ha | Relative Schutzwirkung in % |
|---|---|---|---|
| 7 | 0,5<br>0,25<br>0,125 | 1,0<br>1,0<br>1,0 | 63<br>63<br>63 |
| 7 | 0,5<br>0,25<br>0,125 | 0,5<br>0,5<br>0,5 | 75<br>75<br>75 |
| 7 | 0,5<br>0,25<br>0,125 | 0,25<br>0,25<br>0,25 | 63<br>75<br>63 |
| 7 | 0,5<br>0,25<br>0,125 | 0,125<br>0,125<br>0,125 | 63<br>63<br>50 |

Beispiel 20 : Saatbeizung Weizen, Herbizid im Vorauflaufverfahren

Weizensamen werden mit der als Antidot zu prüfenden Substanz in einen Glasbehälter gegeben und durch Schütteln und Rotation gut durchgemischt. Plastikbehälter (Länge × Breite × Höhe = 25 × 17 × 12 cm) werden mit sandiger Lehmerde gefüllt und die gebeizten Samen eingesät. Nach dem Bedecken des Samens mit Erde wird das Herbizid N-(2-chlorphenylsulfonyl)-N'-(4-methoxy-6-methyl-1,3,5-triazin-2-yl)-harnstoff auf die Bodenoberfläche gesprüht. 21 Tage nach der Herbizidapplikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die nachfolgende Tabelle :

Tabelle 9

| Antidot Verbindung Nr. | Antidot g AS/kg Samen | Herbizid kg AS/ha | Relative Schutzwirkung in % |
|---|---|---|---|
| 7 | 1 | 1,5 | 38 |
| | 0,5 | 1,5 | 38 |
| | 1 | 1,0 | 25 |
| | 0,5 | 1,0 | 25 |

Beispiel 21 : Saatbeizung Gerste, Herbizid im Nachauflaufverfahren

Gerstensamen werden mit der als Antidot zu prüfenden Substanz in einen Glasbehälter gegeben und durch Schütteln und Rotation gut durchgemischt. Plastikbehälter (Länge × Breite × Höhe = 25 × 17 × 12 cm) werden mit sandiger Lehmerde gefüllt und die gebeizten Samen eingesät. Nach dem Bedecken des Samens mit Erde wird das Herbizid N-(2-chlorphenylsulfonyl)-N'-(4-methoxy-6-methyl-1,3,5-triazin-2-yl)-harnstoff im Nachauflaufverfahren appliziert. 21 Tage nach der Applikation wird die Schutzwirkung des Antidots in Prozent bonitiert. Als Referenz dienen dabei die mit Herbizid allein behandelten Pflanzen sowie die vollständig unbehandelte Kontrolle. Die Ergebnisse zeigt die folgende Tabelle :

Tabelle 10

| Antidot Verbindung Nr. | Antidot g AS/kg Samen | Herbizid kg AS/ha | Relative Schutzwirkung in % |
|---|---|---|---|
| 7 | 2 | 1,5 | 50 |
| | 1 | 1,5 | 50 |
| | 0,5 | 1,5 | 63 |
| 7 | 2 | 1,0 | 63 |
| | 1 | 1,0 | 63 |
| | 0,5 | 1,0 | 63 |
| 7 | 2 | 0,5 | 38 |
| | 1 | 0,5 | 38 |
| | 0,5 | 0,5 | 38 |

Beispiel 22 : Saatbeizung Weizen, Herbizid im Nachauflaufverfahren

Weizensamen werden mit der als Antidot zu prüfenden Substanz in einen Glasbehälter gegeben und durch Schütteln und Rotation gut durchgemischt. Plastikbehälter (Länge × Breite × Höhe = 25 × 17 × 12 cm) werden mit sandiger Lehmerde gefüllt und die gebeizten Samen eingesät. Nach dem Bedecken des Samens mit Erde wird das Herbizid N-(2-chlorphenylsulfonyl)-N'-(4-methoxy-6-methyl-1,3,5-triazin-2-yl)-harnstoff im Nachauflaufverfahren appliziert. 21 Tage nach der Applikation wird die Schutzwirkung

des Antidots in Prozent bonitiert. Als Referenz dienen dabei die mit Herbizid allein behandelten Pflanzen sowie die vollständig unbehandelte Kontrolle. Die Ergebnisse zeigt die folgende Tabelle :

Tabelle 11

| Antidot Verbindung Nr. | Antidot g AS/kg Samen | Herbizid kg AS/ha | Relative Schutzwirkung in % |
|---|---|---|---|
| 7 | 1 | 1,0 | 25 |
| | 0,5 | 1,0 | 25 |

Beispiel 23 : Tankmischung im Nachauflaufverfahren in Mais

Maissamen der Sorte « LG 5 » werden in Plastiktöpfe (oberer Durchmesser 11 cm), die 0,5 l Erde enthalten, im Gewächshaus ausgesät. Nach dem Bedecken der Samen mit Erde wird die als Antidot zu prüfende Substanz zusammen mit dem Herbizid N-[2-(2-butenyloxy)-phenyl-sulfonyl]-N'-(4-methoxy-6-methyl-1,3,5-triazin-2-yl)-harnstoff als Tankmischung im Nachauflaufverfahren appliziert. 18 Tage nach der Applikation wird die Schutzwirkung des Antidots in Prozent bonitiert. Als Referenz dienen dabei die mit Herbizid allein behandelten Pflanzen sowie die vollständig unbehandelte Kontrolle. Ein Ergebnis zeigt die folgende Tabelle :

Tabelle 12

| Antidot Verbindung Nr. | Antidot kg AS/ha | Herbizid kg AS/ha | Relative Schutzwirkung in % |
|---|---|---|---|
| 7 | 1,0 | 1,0 | 38 |

Beispiel 24 : Saatbeizung Reis, Herbizid im Vorauflaufverfahren

Reissamen werden mit der als Antidot zu prüfenden Substanz in einen Glasbehälter gegeben und durch Schütteln und Rotation gut durchgemischt. Behälter (Länge × Breite × Höhe = 47 × 29 × 24 cm) werden mit sandiger Lehmerde gefüllt und die gebeizten Samen eingesät. Nach dem Bedecken des Samens mit Erde wird das Herbizid 2-[4-(3,5-Dichlorpyridyl-2-oxy)-phenoxy]-propionsäure-2-propinylester in einer verdünnten Lösung auf die Bodenoberfläche versprüht. 20 Tage nach der Aussaat, wenn die Pflanzen das 3-Blattstadium erreicht haben, wird die Bodenoberfläche mit Wasser 4 cm hoch überschichtet. 30 Tage nach der Herbizidapplikation wird die Schutzwirkung des Antidots in Prozent bonitiert. Als Referenz dinen dabei die mit Herbizid allein behandelten Pflanzen sowie die vollständig unbehandelte Kontrolle. Die Ergebnisse zeigt die folgende Tabelle :

Tabelle 13

| Antidot Verbindung Nr. | Antidot g AS/kg Samen | Herbizid kg AS/ha | Relative Schutzwirkung in % |
|---|---|---|---|
| 7 | 0,6 | 0,25 | 50 |
| | 0,3 | 0,25 | 50 |
| | 0,2 | 0,25 | 38 |

Beispiel 25 : Saatbeizung Reis, Herbizid im Vorauflaufverfahren

Reissamen der Sorte IR-36 werden mit der als Antidot zu prüfenden Substanz in einen Glasbehälter gegegen und durch Schütteln und Rotation gut durchgemischt. Plastikbehälter (Länge × Breite × Hö-

he = 47 × 29 × 24 cm) werden mit sandiger Lehmerde gefüllt und die gebeizten Samen eingesät. Nach dem Bedecken des Samens mit Erde wird das Herbizid 2-[4-(3,5-Dichlorpyridyl-2-oxy)-phenoxy]-propionsäure-2-propinylester auf die Bodenoberfläche versprüht. 18 Tage nach der Aussaat wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle :

Tabelle 14

| Antidot Verbindung Nr. | Antidot g AS/kg Samen | Herbizid kg AS/ha | Relative Schutzwirkung in % |
|---|---|---|---|
| | 0,6 | 0,25 | 50 |
| 7 | 0,3 | 0,25 | 50 |
| | 0,2 | 0,25 | 38 |

Beispiel 26 : Tankmischung im Nachauflaufverfahren in Weizen

Weizensamen der Sorte « Farnese » werden in Plastiktöpfe (oberer Durchmesser 11 cm) die 0,5 l Erde enthalten, im Gewächshaus ausgesät. Nach dem Bedecken der Samen mit Erde wird die als Antidot zu prüfende Substanz zusammen mit dem Herbizid 2-Chlor-4-trifluormethylphenyl-3'-oxazolin-2'-yl-4'-nitrophenyläther als Tankmischung im Nachauflaufverfahren appliziert. 20 Tage nach der Applikation wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle :

Tabelle 15

| Antidot Verbindung Nr. | Antidot kg AS/ha | Herbizid kg AS/ha | Relative Schutzwirkung in % |
|---|---|---|---|
| | 0,25 | 0,25 | 25 |
| 13 | 0,125 | 0,25 | 25 |
| | 0,25 | 0,125 | 25 |
| 13 | 0,125 | 0,125 | 25 |
| | 0,062 | 0,125 | 25 |

Beispiel 27 : Tankmischung im Nachauflaufverfahren in Weizen

Weizensamen der Sorte « Farnese » werden in Plastiktöpfe (oberer Durchmesser 11 cm), die 0,5 l Erde enthalten, im Gewächshaus ausgesät. Nach dem Bedecken der Samen mit Erde wird die als Antidot zu prüfende Substanz zusammen mit dem Herbizid 2-[4-(5-Trifluormethylpyridyl-2-oxy)-phenoxy]-propionsäure-n-butylester als Tankmischung im Nachauflaufverfahren appliziert. 20 Tage nach der Applikation wird die Schutzwirkung des Antidots in Prozent bonitiert. Als Referenzen dienen dabei die mit Herbizid allein behandelten Pflanzen sowie die vollständig unbehandelte Kontrolle. Ein Ergebnis zeigt die folgende Tabelle :

Tabelle 16

| Antidot Verbindung Nr. | Antidot kg AS/ha | Herbizid kg AS/ha | Relative Schutzwirkung in % |
|---|---|---|---|
| 13 | 0,125 | 0,060 | 25 |

Beispiel 28 : Saatbeizung Sorghum, Herbizid im Vorauflaufverfahren

Sorghumsamen der Sorte Funk G 623 werden mit der als Antidot zu prüfenden Substanz in einen Glasbehälter gegeben und durch Schütteln und Rotation gut durchgemischt. Plastiktöpfe (oberer Durchmesser 11 cm) werden mit Erde gefüllt und die gebeizten Samen eingesät. Nach dem Bedecken der Samen mit Erde wird als Herbizid entweder N-(2-chlorphenylsulfonyl)-N'-(4-methoxy-6-methyl-1,3,5-triazin-2-yl)-harnstoff (A) oder N-(2-Methoxycarbonylphenylsulfonyl)-N'-4,6-dimethylpyrimidin-2-yl)-harnstoff (B) im Vorauflaufverfahren appliziert. 18 Tage nach der Herbizidapplikation wird die Schutzwirkung des Antidots in Prozent bonitiert. Als Referenzen dienen dabei die mit Herbizid allein behandelten Pflanzen sowie die vollständig unbehandelte Kontrolle. Die Ergebnisse zeigt die folgende Tabelle :

Tabelle 17

| Herbizid | | Antidot | | Relative Schutzwirkung in % |
|---|---|---|---|---|
| Verbindung | kg AS/ha | Verbindung Nr. | g AS/kg Samen | |
| A | 0,062 | 7 | 2 | 12,5 |
| | | | 1 | 25 |
| | | | 0,5 | 25 |
| A | 0,031 | 7 | 2 | 25 |
| | | | 1 | 38 |
| | | | 0,5 | 50 |
| A | 0,015 | 7 | 2 | 50 |
| | | | 1 | 63 |
| | | | 0,5 | 63 |
| B | 0,062 | 7 | 2 | 38 |
| | | | 1 | 38 |
| | | | 0,5 | 25 |
| B | 0,031 | 7 | 2 | 50 |
| | | | 1 | 38 |
| | | | 0,5 | 25 |
| B | 0,015 | 7 | 2 | 50 |
| | | | 1 | 50 |
| | | | 0,5 | 50 |

**Patentansprüche** (für die Vertragsstaaten : BE, CH, DE, FR, IT, LI, NL, SE)

1. Verfahren zum Schützen von Kulturpflanzen gegen schädigende Wirkungen von agressiven Agrarchemikalien, dadurch gekennzeichnet, dass man die Kulturpflanzen, Teile dieser Pflanzen oder für den Anbau der Kulturpflanzen bestimmte Böden mit einer Verbindung der Formel I

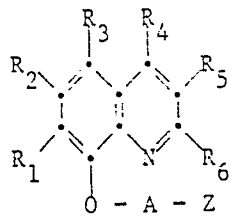

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyano,

$R_4$, $R_5$ und $R_6$ unabhängig voneinander Wasserstoff, Halogen oder $C_1$-$C_3$-Alkyl,

A eine der Gruppen $—CH_2—$, $—CH_2—CH_2—$ oder $—CH(CH_3)—$ und

Z Cyan oder Amidoxim, welches am Sauerstoffatom acyliert sein kann, bedeuten, unter Einschluss ihrer Säureadditionssalze und Metallkomplexe, oder einem Mittel, welches eine dieser Verbindungen enthält, behandelt.

2. Verfahren nach Anspruch 1, dadurch gekennzeichnet, dass man eine Verbindung der Formel I, in welcher $R_1$, $R_2$ und $R_3$ unabhängig voneinander Wasserstoff, Halogen, $C_1$-$C_2$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $—CH_2—$, $—CH_2—CH_2—$ oder $—CH(CH_3)—$ und

Z Cyan oder eine der Gruppen

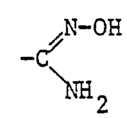 oder 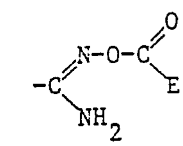

bedeuten, wobei

E für $—R_7$, $—OR_8$, $—SR_9$ oder $—NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, oder ein Mittel, welches eine dieser Verbindungen enthält, verwendet.

3. Verfahren nach Anspruch 2, dadurch gekennzeichnet, dass man eine Verbindung der Formel I, in welcher

$R_1$ Wasserstoff, Chlor, Brom, Jod oder Nitro, $R_2$ Wasserstoff,

$R_3$ Wasserstoff, Fluor, Chlor, Brom, Jod, $C_1$-$C_3$-Alkyl oder Nitro,

$R_4$ Wasserstoff, Brom oder Methyl, $R_5$ Wasserstoff, $R_6$ Wasserstoff oder Methyl, A $—CH_2—$, $—CH_2—CH_2—$ oder $—CH(CH_3)—$ und Z Cyan,

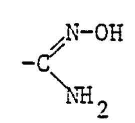 oder 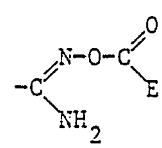

bedeuten, wobei

E für $—R_7$, $—OR_8$, $—SR_9$ oder $—NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono-

oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder durch Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy oder

$R_{10}$ und $R_{11}$ gemeinsam mit dem Stickstoffatom, an das sie gebunden sind, einen Piperindin- oder Morpholinring bedeuten, oder ein Mittel, welches eine dieser Verbindungen enthält, verwendet.

4. Verfahren nach Anspruch 3, dadurch gekennzeichnet, dass man eine Verbindung der Formel I, in welcher

$R_1$ Wasserstoff, Chlor, Brom oder Jod, $R_2$ Wasserstoff, $R_3$ Wasserstoff, Chlor oder Nitro, $R_4$ und $R_5$ Wasserstoff, $R_6$ Wasserstoff oder Methyl, A $—CH_2—$, $—CH_2—CH_2—$ oder $—CH(CH_3)—$ und Z Cyan,

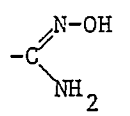 oder 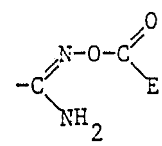

bedeuten, wobei

E für $—R_7$, $—OR_8$, $—SR_9$ oder $—NR_{10}R_{11}$ steht, worin

$R_7$ Methyl, Aethyl, n-Propyl, Isopropyl, n-Butyl, tert. Butyl, Isobutyl, Chlormethyl, Brommethyl, 2-Chloräthyl, 3-Chlor-n-propyl, 1,2-Dichloräthyl, Methoxymethyl, n-Propoxymethyl, sek.Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten, oder ein Mittel, welches eine dieser Verbindungen enthält, verwendet.

5. Verfahren nach Anspruch 4, dadurch gekennzeichnet, dass man eine Verbindung der Formel I, in welcher $R_1$ Wasserstoff, Chlor, Brom oder Jod, $R_2$ Wasserstoff, $R_3$ Wasserstoff oder Chlor, $R_4$ und $R_5$ Wasserstoff,

$R_6$ Wasserstoff oder Methyl, A $—CH_2—$ und Z Cyan,

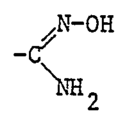 oder 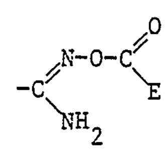

bedeuten, wobei E für $—R_7$, $—OR_8$ oder $—NR_{10}R_{11}$ steht, worin

$R_7$ Chlormethyl, $R_8$ Methyl, $R_{10}$ Isopropyl und $R_{11}$ Wasserstoff bedeuten, oder ein Mittel, welches eine dieser Verbindungen enthält, verwendet.

6. Verfahren nach Anspruch 5, dadurch gekennzeichnet, dass man 5-Chlor-8-(cyanomethoxy)-chinolin oder ein Mittel, welches diese Verbindung enthält, verwendet.

7. Verfahren nach Anspruch 5, dadurch gekennzeichnet, dass man 5-Chlor-7-jod-8-(cyanomethoxy)-chinolin oder ein Mittel, welches diese Verbindung enthält, verwendet.

8. Verfahren nach Anspruch 5, dadurch gekennzeichnet, dass man O-(Methoxycarbonyl)-2-(8-chinolinoxy)-acetamidoxim oder ein Mittel, welches diese Verbindung enthält, verwendet.

9. Verfahren nach Anspruch 1 zum Schützen von Kulturpflanzen gegen schädigende Wirkungen von Pflanzenschutzmitteln.

10. Verfahren nach Anspruch 9 zum Schützen von Kulturpflanzen gegen schädigende Wirkungen von Herbiziden.

11. Verfahren nach Anspruch 10 zum Schützen von Kulturpflanzen gegen schädigende Wirkungen von substituierten Pyridyloxyphenoxypropionsäureestern.

12. Verfahren nach Anspruch 11 zum Schützen von Kulturpflanzen gegen schädigende Wirkungen von 2-[4-(3,5-Dichlorpyridyl-2-oxy)-phenoxy]-propionsäure-2-propinylester.

13. Verfahren nach Anspruch 10 zum Schützen von Kulturpflanzen gegen schädigende Wirkungen von Phenylharnstoffen.

14. Verfahren nach Anspruch 10 zum Schützen von Kulturpflanzen gegen schädigende Wirkungen von Sulfonylharnstoffen.

15. Verfahren nach Anspruch 1 zum Schützen von Reis, Mais, Weizen, Roggen, Gerste, Hafer, Baumwolle, Zuckerrüben, Zuckerrohr und Soja.

16. Mittel zum Schützen von Kulturpflanzen gegen schädigende Wirkungen von aggressiven Agrarchemikalien, dadurch gekennzeichnet, dass es eine Verbindung der Formel I

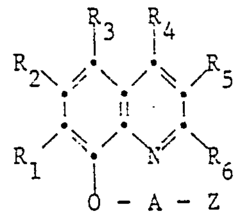

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyano,

$R_4$, $R_5$ und $R_6$ unabhängig voneinander Wasserstoff, Halogen oder $C_1$-$C_3$-Alkyl, A eine der Gruppen $-CH_2-$, $-CH_2-CH_2-$ oder $-CH(CH_3)-$ und

Z Cyan oder Amidoxim, welches am Sauerstoffatom acyliert sein kann, bedeuten, unter Einschluss ihrer Säureadditionssalze und Metallkomplexe, enthält.

17. Mittel nach Anspruch 16, dadurch gekennzeichnet, dass es eine Verbindung der Formel I, in welcher $R_1$, $R_2$ und $R_3$ unabhängig voneinander Wasserstoff, Halogen, $C_1$-$C_3$-Alkyl, $C_1$-$C_3$-Alkoxy, Nitro oder Cyan, $R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder eine der Gruppen

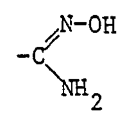

oder

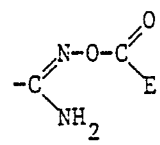

bedeuten, wobei

E für $-R_7$, $-OR_8$, $-SR_9$ oder $-NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, enthält.

18. Mittel nach Anspruch 17, dadurch gekennzeichnet, dass es eine Verbindungen der Formel I, in welcher

$R_1$ Wasserstoff, Chlor, Brom, Jod oder Nitro, $R_2$ Wasserstoff,

$R_3$ Wasserstoff, Fluor, Chlor, Brom, Jod, $C_1$-$C_3$-Alkyl oder Nitro,

$R_4$ Wasserstoff, Brom oder Methyl, $R_5$ Wasserstoff, $R_6$ Wasserstoff oder Methyl, A $-CH_2-$, $-CH_2-CH_2-$ oder $-CH(CH_3)-$ und Z Cyan,

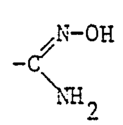

oder

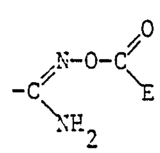

bedeuten, wobei

E für $-R_7$, $-OR_8$, $-SR_9$ oder $-NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder

durch Chlor oder Nitro monosubstituiert ist, einen unsubstituierten oder durch Chlor oder Brom mono- oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy oder $R_{10}$ und $R_{11}$ gemeinsam mit dem Stickstoffatom, an das sie gebunden sind, einen Piperidin- oder Morpholinring bedeuten, enthält.

19. Mittel nach Anspruch 18, dadurch gekennzeichnet, dass es eine Verbindung der Formel I, in welcher

$R_1$ Wasserstoff, Chlor, Brom oder Jod, $R_2$ Wasserstoff, $R_3$ Wasserstoff, Chlor oder Nitro, $R_4$ und $R_5$ Wasserstoff, $R_6$ Wasserstoff oder Methyl,

A $—CH_2—$, $—CH_2—CH_2—$ oder $—CH(CH_3)—$ und Z Cyan,

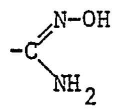 oder 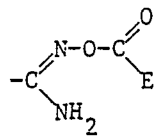

bedeuten, wobei

E für $—R_7$, $—OR_8$, $—SR_9$ oder $—NR_{10}R_{11}$

steht, worin

$R_7$ Methyl, Aethyl, n-Propyl, Isopropyl, n-Butyl, tert.Butyl, Isobutyl, Chlormethyl, Brommethyl, 2-Chloräthyl, 3-Chlor-n-propyl, 1,2-Dichloräthyl, Methoxymethyl, n-Propoxymethyl, sek.-Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten, enthält.

20. Mittel nach Anspruch 19, dadurch gekennzeichnet, dass es eine Verbindung der Formel I, in welcher $R_1$ Wasserstoff, Chlor, Brom oder Jod, $R_2$ Wasserstoff, $R_3$ Wasserstoff oder Chlor, $R_4$ und $R_5$ Wasserstoff, $R_6$ Wasserstoff oder Methyl, A $—CH_2—$ und Z Cyan,

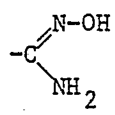 oder 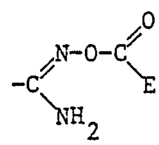

bedeuten, wobei E für $—R_7$, $—OR_8$ oder $NR_{10}R_{11}$ steht, worin $R_7$ Chlormethyl, $R_8$ Methyl, $R_{10}$ Isopropyl und $R_{11}$ Wasserstoff bedeuten, enthält.

21. Mittel nach Anspruch 20, dadurch gekennzeichnet, dass es 5-Chlor-8-(cyanomethoxy)-chinolin enthält.

22. Mittel nach Anspruch 20, dadurch gekennzeichnet, dass es 5-Chlor-7-jod-8-(cyanomethoxy)-chinolin enthält.

23. Mittel nach Anspruch 20, dadurch gekennzeichnet, dass es O-(Methoxycarbonyl)-2-(8-chinolinoxy)-acetamidoxim enthält.

24. Mittel nach Anspruch 16, dadurch gekennzeichnet, dass es eine Verbindung der Formel I und ein Herbizid enthält.

25. Mittel nach Anspruch 24, dadurch gekennzeichnet, dass es als Herbizid eine Verbindung der Formel (A)

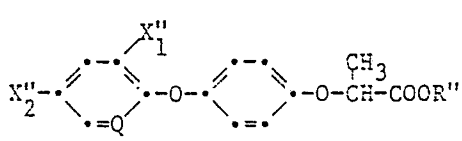 (A)

worin

$X_1''$ Wasserstoff oder Halogen,

$X_2''$ Wasserstoff, Halogen oder Trifluormethyl,

Q das Fragment =N— oder =CH—,

R″ $C_1$-$C_4$-Alkyl, welches unsubstituiert oder durch $C_1$-$C_4$-Alkoxy substituiert ist, $C_3$-$C_4$-Alkenyl, $C_3$-$C_4$-Alkinyl oder

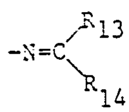

$R_{13}$ $C_1$-$C_4$-Alkyl, $R_{14}$ $C_1$-$C_4$-Alkyl oder $R_{13}$ und $R_{14}$ gemeinsam $C_4$-$C_5$-Alkylen bedeuten, enthält.

26. Mittel nach Anspruch 24, dadurch gekennzeichnet, dass es als Herbizid einen substituierten Pyridyloxyphenoxypropionsäureester enthält.

27. Mittel nach Anspruch 24, dadurch gekennzeichnet, dass es als Herbizid 2-[4-(3,5-Dichlorpyridyl-2-oxy)-phenoxy]-propionsäure-2-propinylester enthält.

28. Mittel nach Anspruch 24, dadurch gekennzeichnet, dass es als Herbizid einen Phenylharnstoff enthält.

29. Mittel nach Anspruch 24, dadurch gekennzeichnet, dass es als Herbizid einen Sulfonylharnstoff enthält.

30. Mittel nach Anspruch 24, dadurch gekennzeichnet, dass es als Verbindung der Formel I 5-Chlor-8-(cyanomethoxy)-chinolin und als Herbizid 2-[4-(3,5-Dichlorpyridyl-2-oxy)-phenoxy]-propionsäure-2-propinylester enthält.

31. Mittel nach Anspruch 16, dadurch gekennzeichnet, dass es eine Verbindung der Formel Ia

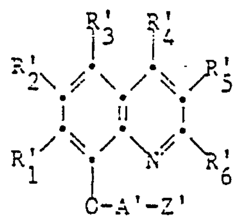

(Ia)

worin $R_1'$, $R_2'$ und $R_3'$ unabhängig voneinander Wasserstoff, Halogen, $C_1$-$C_3$-Alkyl, $C_1$-$C_3$-Alkoxy, Nitro oder Cyano,

$R_4'$, $R_5'$ und $R_6'$ unabhängig voneinander Wasserstoff, Halogen oder $C_1$-$C_3$-Alkyl,

A′ eine der Gruppen $—CH_2—$, $—CH_2—CH_2—$ oder $—CH(CH_3)—$ und

Z′ Cyan oder eine der Gruppen

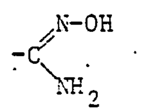 oder 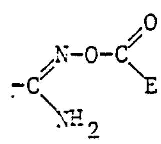

bedeuten, wobei

E für $—R_7$, $—OR_8$, $—SR_9$ oder $—NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, mit der Massgabe, dass Z′ nicht für Cyan oder die Gruppe

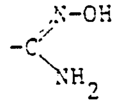

steht, wenn gleichzeitig $R_1'$, $R_2'$, $R_4'$, $R_5'$ und $R_6'$ Wasserstoff, $R_3'$ Wasserstoff oder Chlor und A' $-CH_2-$ oder $-CH(CH_3)-$ bedeuten, enthält.

32. Verbindungen der Formel Ia

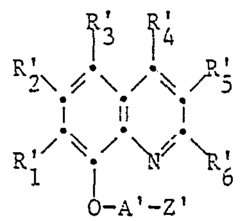

(Ia)

worin $R_1'$, $R_2'$ und $R_3'$ unabhängig voneinander Wasserstoff, Halogen, $C_1$-$C_3$-Alkyl, $C_1$-$C_3$-Alkoxy, Nitro oder Cyan,

$R_4'$, $R_5'$ und $R_6'$ unabhängig voneinander Wasserstoff, Halogen oder $C_1$-$C_3$-Alkyl,

A' eine der Gruppen $-CH_2-$, $-CH_2-CH_2-$ oder $-CH(CH_3)-$ und

Z' Cyan oder Amidoxim, welches am Sauerstoffatom acyliert sein kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $-CH_2-$ oder $-CH(CH_3)-$ bedeuten.

33. Verbindungen nach Anspruch 32, dadurch gekennzeichnet, dass

$R_1'$, $R_2'$ und $R_3'$ unabhängig voneinander Wasserstoff, Halogen, $C_1$-$C_3$-Alkyl, $C_1$-$C_3$-Alkoxy, Nitro oder Cyano,

$R_4'$, $R_5'$ und $R_6'$ unabhängig voneinander Wasserstoff, Halogen oder $C_1$-$C_3$-Alkyl,

A' eine der Gruppen $-CH_2-$, $-CH_2-CH_2-$ oder $-CH(CH_3)-$ und

Z' Cyan oder ein der Gruppen

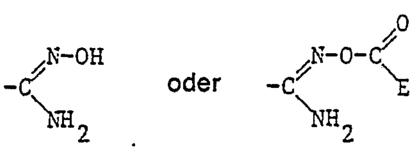

bedeuten, wobei

E für $-R_7$, $-OR_8$, $-SR_9$ oder $-NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $-CH_2-$ oder $-CH(CH_3)-$ bedeuten.

34. Verbindungen nach Anspruch 33, dadurch gekennzeichnet, dass $R_1'$ Wasserstoff, Chlor, Brom, Jod oder Nitro, $R_2'$ Wasserstoff, $R_3'$ Wasserstoff, Fluor, Chlor, Brom, Jod, $C_1$-$C_3$-Alkyl oder Nitro, $R_4'$ Wasserstoff, Brom oder Methyl, $R_5'$ Wasserstoff, $R_6'$ Wasserstoff oder Methyl, A' $-CH_2-$, $-CH_2-CH_2-$ oder $-CH(CH_3)-$ und Z' Cyan,

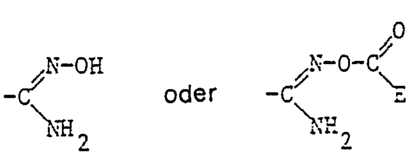

bedeuten, wobei

E für $R_7$, $—OR_8$, $—SR_9$ oder $—NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono- oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder durch Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy

oder $R_{10}$ und $R_{11}$ gemeinsam mit dem Stickstoffatom, an das sie gebunden sind, einen Piperidin- oder Morpholinring bedeuten, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten.

35. Verbindungen nach Anspruch 34, dadurch gekennzeichnet, dass $R_1'$ Wasserstoff, Chlor, Brom oder Jod, $R_2'$ Wasserstoff, $R_3'$ Wasserstoff, Chlor oder Nitro, $R_4'$ und $R_5'$ Wasserstoff, $R_6'$ Wasserstoff oder Methyl, A' $—CH_2—$, $—CH_2—CH_2—$ oder $—CH(CH_3)—$ und Z' Cyan,

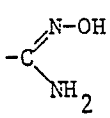 oder 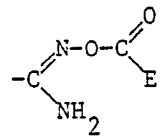

bedeuten, wobei

E für $—R_7$, $—OR_8$, $—SR_9$ oder $—NR_{10}R_{11}$ steht, worin

$R_7$ Methyl, Aethyl, n-Propyl, Isopropyl, n-Butyl, tert.Butyl, Isobutyl, Chlormethyl, Brommethyl, 2-Chloräthyl, 3-Chlor-n-propyl, 1,2-Dichloräthyl, Methoxymethyl, n-Propoxymethyl, sek.-Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten,

mit der Massgabe, dass Z' nicht für Cyan oder Amidoxim steht, wenn

$R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten.

36. Verbindungen nach Anspruch 35, dadurch gekennzeichnet, dass $R_1'$ Wasserstoff, Chlor, Brom oder Jod, $R_2'$ Wasserstoff, $R_3'$ Wasserstoff oder Chlor, $R_4'$ und $R_5'$ Wasserstoff, $R_6'$ Wasserstoff oder Methyl, A' $—CH_2—$ und Z' Cyan,

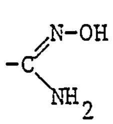 oder 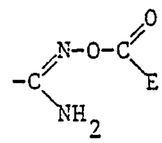

bedeuten, wobei E für $—R_7$, $—OR_8$ oder $NR_{10}R_{11}$ steht, worin $R_7$ Chlormethyl, $R_8$ Methyl, $R_{10}$ Isopropyl und $R_{11}$ Wasserstoff bedeuten, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten.

37. 2-Methyl-8-(cyanomethoxy)-chinolin.

38. 2-(2-Methyl-8-chinolinoxy)-acetamidoxim.

39. O-(Isopropylaminocarbonyl)-2-(8-chinolinoxy)-acetamidoxim.

40. O-(Chlormethylcarbonyl)-2-(8-chinolinoxy)-acetamidoxim.

41. 2-(5-Chlor-7-brom-8-chinolinoxy)-acetamidoxim.

42. 5-Chlor-7-brom-8-(cyanomethoxy)-chinolin.

43. O-(Methoxycarbonyl)-2-(8-chinolinoxy)-acetamidoxim.

44. 2-(5-Chlor-7-jod-8-chinolinoxy)-acetamidoxim.

45. O-(Isopropylaminocarbonyl)-2-(5-chlor-7-brom-8-chinolinoxy)-acetamidoxim.

46. 2-(2-Methyl-5,7-dichlor-8-chinolinoxy)-acetamidoxim.

47. 5,7-Dichlor-8-(cyanomethoxy)-chinolin.
48. O-(Isopropylaminocarbonyl)-2-(5-chlor-7-jod-8-chinolinoxy)-acetamidoxim.
49. 2-Methyl-5,7-Dichlor-8-(cyanomethoxy)-chinolin.
50. O-(Isopropylaminocarbonyl)-2-(2-methyl-5,7-dichlor-8-chinolinoxy)-acetamidoxim.
51. 5-Chlor-7-jod-8-(cyanomethoxy)-chinolin.
52. Verfahren zur Herstellung von Verbindungen der Formel Ia

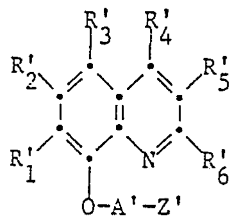

(Ia)

worin $R_1'$, $R_2'$ und $R_3'$ unabhängig voneinander Wasserstoff, Halogen, $C_1$-$C_3$-Alkyl, $C_1$-$C_3$-Alkoxy, Nitro oder Cyan,

$R_4'$, $R_5'$ und $R_6'$ unabhängig voneinander Wasserstoff, Halogen oder $C_1$-$C_3$-Alkyl,

A' eine der Gruppen $—CH_2—$, $—CH_2—CH_2—$ oder $—CH(CH_3)—$ und

Z' Cyan oder Amidoxim, welches am Sauerstoffatom acyliert sein kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff, $R_3'$ Wasserstoff oder Chlor und A' $—CH_2—$ oder $—CH(CH_3)—$ bedeuten, dadurch gekennzeichnet, dass man

a) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für die Gruppe $—CH_2—CH_2—$ und Z' für Cyan steht, eine Verbindung der Formel II

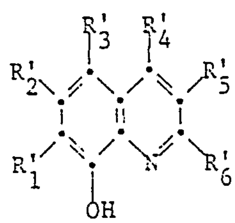

(II)

worin $R_1'$, $R_2'$, $R_3'$, $R_4'$, $R_5'$ und $R_6'$ die vorstehend angegebenen Bedeutungen haben, mit einer Verbindung der Formel III

$$CH_2{=}CH—CN \qquad (III)$$

umsetzt, oder

b) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für eine der Gruppen $—CH_2—$ oder $—CH(CH_3)—$ und Z' für Cyan steht, eine Verbindung der Formel II

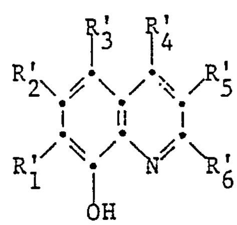

(II)

worin $R_1'$, $R_2'$, $R_3'$, $R_4'$, $R_5'$ und $R_6'$ die für Formel II angegebenen Bedeutungen haben, mit

i) einer Verbindung der Formel IV

$$Hal—A'—CN \qquad (IV)$$

worin Hal für ein Halogenatom steht und A' die vorstehend angegebene Bedeutung hat, umsetzt, oder

ii) einer Verbindung der Formel V

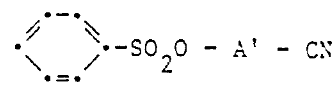

(V)

worin A' die vorstehend angegebene Bedeutung hat, umsetzt, oder

iii) einer Verbindung der Formel VI

$$Hal—A'—COOR_{12} \quad (VI)$$

worin Hal für ein Halogenatom und $R_{12}$ für eine Alkylgruppe mit 1 bis 6 Kohlenstoffatomen stehen und A' die vorstehend angegebene Bedeutung hat, umsetzt und die erhaltenen Ester der Formel VII

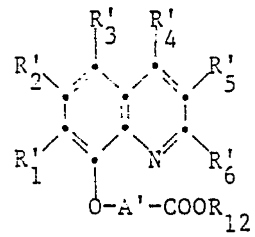

(VII)

worin $R_1'$, $R_2'$, $R_3'$, $R_4'$, $R_5'$, $R_6'$, A' und $R_{12}$ die vorstehend angegebenen Bedeutungen haben, mit Ammoniak in die entsprechenden Amide der Formel VIII

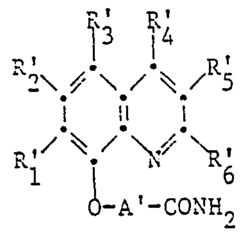

(VIII)

worin $R_1'$, $R_2'$, $R_3'$, $R_4'$, $R_5'$, $R_6'$ und A' die vorstehend angegebenen Bedeutungen haben, überführt und anschliessend dehydratisiert, und/oder

c) zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für Amidoxim, welches am Sauerstoffatom acyliert sein kann, steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Cyan steht, mit Hydroxylamin oder einem Säuresalz des Hydroxylamins umsetzt, und/oder

d) zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für acyliertes Amidoxim steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Amidoxim steht, acyliert.

53. Verfahren nach Anspruch 52, dadurch gekennzeichnet, dass man zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für acyliertes Amidoxim der Formel

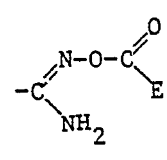

steht, wobei E $—R_7$, $—OR_8$, $—SR_9$ oder $—NR_{10}R_{11}$ ist, worin $R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist, $R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist, $R_{11}$ Wasserstoff, $C_1$-$C_8$-Alkyl oder $C_1$-$C_3$-Alkoxy oder $R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, eine Verbindung der Formel Ia, in welcher $R_1'$, $R_2'$, $R_3'$, $R_4'$,

$R_5'$, $R_6'$ und A' die für Formel Ia angegebenen Bedeutungen haben und Z' für Amidoxim steht, mit einer Verbindung der Formel IX

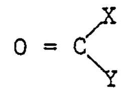

(IX)

worin X für ein Halogenatom und Y für $—R_7$, $—OR_8$, $—SR_9$ oder $—NR_{10}R_{11}$ steht, wobei $R_7$, $R_8$, $R_9$, $R_{10}$ und $R_{11}$ die vorstehend angegebenen Bedeutungen haben, oder X und Y gemeinsam für die Iminogruppe $=N—R_{10}$ stehen, umsetzt.

54. Verfahren zur selektiven Bekämpfung von Unkräutern in Kulturpflanzenbeständen, dadurch gekennzeichnet, dass man die Kulturpflanzenbestände, Teile der Kulturpflanzen oder Anbauflächen der Kulturpflanzen mit einem Herbizid und einer Verbindung der Formel I

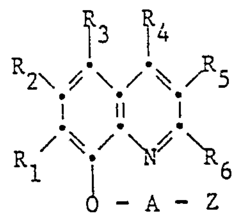

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $—CH_2—$, $—CH_2—CH_2—$ oder $—CH(CH_3)—$ und

Z Cyan oder Amidoxim, welches am Sauerstoffatom acyliert sein kann, bedeuten, unter Einschluss ihrer Säureadditionssalze und Metallkomplexe, oder einem Mittel, welches eine dieser Verbindungen enthält, behandelt.

55. Mittel zur selektiven Bekämpfung von Unkräutern in Kulturpflanzenbeständen, dadurch gekennzeichnet, dass es eine Verbindung der Formel I

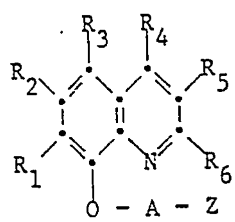

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $—CH_2—$, $—CH_2—CH_2—$ oder $—CH(CH_3)—$ und

Z Cyan oder Amidoxim, welches am Sauerstoffatom acyliert sein kann, bedeuten, unter Einschluss ihrer Säureadditionssalze und Metallkomplexe, und ein Herbizid enthält.

**Patentansprüche** (für den Vertragsstaat AT)

1. Verfahren zum Schützen von Kulturpflanzen gegen schädigende Wirkungen von agressiven Agrarchemikalien, dadurch gekennzeichnet, dass man die Kulturpflanzen, Teile dieser Pflanzen oder für den Anbau der Kulturpflanzen bestimmte Böden mit einer Verbindung der Formel I

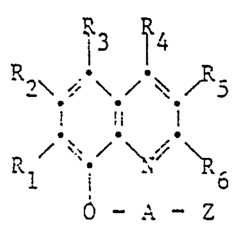

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyano,

$R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder Amidoxim, welches am Sauerstoffatom acyliert sein kann, bedeuten, unter Einschluss ihrer Säureadditionssalze und Metallkomplexe, oder einem Mittel, welches eines dieser Verbindungen enthält, behandelt.

2. Verfahren nach Anspruch 1, dadurch gekennzeichnet, dass man eine Verbindung der Formel I, in welcher $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder eine der Gruppen

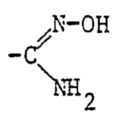 oder 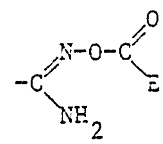

bedeuten, wobei

E für $-R_7$, $-OR_8$, $-SR_9$ oder $-NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder $R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, oder ein Mittel, welches eine dieser Verbindungen enthält, verwendet.

3. Verfahren nach Anspruch 2, dadurch gekennzeichnet, dass man eine Verbindung der Formel I, in welcher

$R_1$ Wasserstoff, Chlor, Brom, Jod oder Nitro, $R_2$ Wasserstoff,

$R_3$ Wasserstoff, Fluor, Chlor, Brom, Jod, $C_1$-$C_3$-Alkyl oder Nitro,

$R_4$ Wasserstoff, Brom oder Methyl, $R_5$ Wasserstoff, $R_6$ Wasserstoff oder Methyl, A $-CH_2-$, $-CH_2-CH_2-$ oder $-CH(CH_3)-$ und Z Cyan,

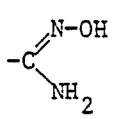 oder 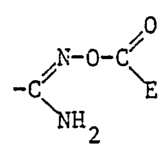

bedeuten, wobei

E für $-R_7$, $-OR_8$, $-SR_9$ oder $-NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono- oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder durch Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy oder

$R_{10}$ und $R_{11}$ gemeinsam mit dem Stickstoffatom, an das sie gebunden sind, einen Piperidin- oder Morpholinring bedeuten, oder ein Mittel, welches eine dieser Verbindungen enthält, verwendet.

4. Verfahren nach Anspruch 3, dadurch gekennzeichnet, dass man eine Verbindung der Formel I, in

welcher

$R_1$ Wasserstoff, Chlor, Brom oder Jod, $R_2$ Wasserstoff, $R_3$ Wasserstoff, Chlor oder Nitro, $R_4$ und $R_5$ Wasserstoff, $R_6$ Wasserstoff oder Methyl, A $-CH_2-$, $-CH_2-CH_2-$ oder $-CH(CH_3)-$ und Z Cyan,

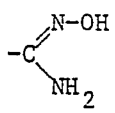 oder 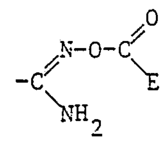

bedeuten, wobei

E für $-R_7$, $-OR_8$, $-SR_9$ oder $-NR_{10}R_{11}$ steht, worin

$R_7$ Methyl, Aethyl, n-Propyl, Isopropyl, n-Butyl, tert.Butyl, Isobutyl, Chlormethyl, Brommethyl, 2-Chloräthyl, 3-Chlor-n-propyl, 1,2-Dichloräthyl, Methoxymethyl, n-Propoxymethyl, sek.Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten, oder ein Mittel, welches eine dieser Verbindungen enthält, verwendet.

5. Verfahren nach Anspruch 4, dadurch gekennzeichnet, dass man eine Verbindung der Formel I, in welcher $R_1$ Wasserstoff, Chlor, Brom oder Jod, $R_2$ Wasserstoff, $R_3$ Wasserstoff oder Chlor, $R_4$ und $R_5$ Wasserstoff, $R_6$ Wasserstoff oder Methyl, A $-CH_2-$ und Z Cyan,

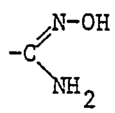 oder 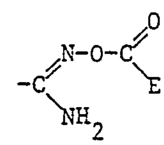

bedeuten, wobei E für $-R_7$, $-OR_8$ oder $-NR_{10}R_{11}$ steht, worin $R_7$ Chlormethyl, $R_8$ Methyl, $R_{10}$ Isopropyl und $R_{11}$ Wasserstoff bedeuten, oder ein Mittel, welches eine dieser Verbindungen enthält, verwendet.

6. Verfahren nach Anspruch 5, dadurch gekennzeichnet, dass man 5-Chlor-8-(cyanomethoxy)-chinolin oder ein Mittel, welches diese Verbindung enthält, verwendet.

7. Verfahren nach Anspruch 5, dadurch gekennzeichnet, dass man 5-Chlor-7-jod-8-(cyanomethoxy)-chinolin oder ein Mittel, welches diese Verbindung enthält, verwendet.

8. Verfahren nach Anspruch 5, dadurch gekennzeichnet, dass man O-(Methoxycarbonyl)-2-(8-chinolinoxy)-acetamidoxim oder ein Mittel, welches diese Verbindung enthält, verwendet.

9. Verfahren nach Anspruch 1 zum Schützen von Kulturpflanzen gegen schädigende Wirkungen von Pflanzenschutzmitteln.

10. Verfahren nach Anspruch 9 zum Schützen von Kulturpflanzen gegen schädigende Wirkungen von Herbiziden.

11. Verfahren nach Anspruch 10 zum Schützen von Kulturpflanzen gegen schädigende Wirkungen von substituierten Pyridyloxyphenoxypropionsäureestern.

12. Verfahren nach Anspruch 11 zum Schützen von Kulturpflanzen gegen schädigende Wirkungen von 2-[4-(3,5-Dichlorpyridyl-2-oxy)-phenoxy]-propionsäure-2-propinylester.

13. Verfahren nach Anspruch 10 zum Schützen von Kulturpflanzen gegen schädigende Wirkungen von Phenylharnstoffen.

14. Verfahren nach Anspruch 10 zum Schützen von Kulturpflanzen gegen schädigende Wirkungen von Sulfonylharnstoffen.

15. Verfahren nach Anspruch 1 zum Schützen von Reis, Mais, Weizen, Roggen, Gerste, Hafer, Baumwolle, Zuckerrüben, Zuckerrohr und Soja.

16. Mittel zum Schützen von Kulturpflanzen gegen schädigende Wirkungen von aggressiven Agrarchemikalien, dadurch gekennzeichnet, dass es eine Verbindung der Formel I

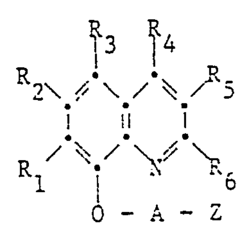 (I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyano,

$R_4$, $R_5$ und $R_6$ unabhängig voneinander Wasserstoff, Halogen oder $C_1$-$C_3$-Alkyl, A eine der Gruppen $—CH_2—$, $—CH_2—CH_2—$ oder $—CH(CH_3)—$ und Z Cyan oder Amidoxim, welches am Sauerstoffatom acyliert sein kann, bedeuten, unter Einschluss ihrer Säureadditionssalze und Metallkomplexe, enthält.

17. Mittel nach Anspruch 16, dadurch gekennzeichnet, dass es eine Verbindung der Formel I, in welcher $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $—CH_2—$, $—CH_2—CH_2—$ oder $—CH(CH_3)—$ und

Z Cyan oder eine der Gruppen

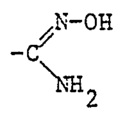 oder 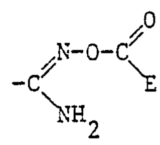

bedeuten, wobei

E für $—R_7$, $—OR_8$, $—SR_9$ oder $—NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, enthält.

18. Mittel nach Anspruch 17, dadurch gekennzeichnet, dass es eine Verbindungen der Formel I, in welcher

$R_1$ Wasserstoff, Chlor, Brom, Jod oder Nitro, $R_2$ Wasserstoff,

$R_3$ Wasserstoff, Fluor, Chlor, Brom, Jod, $C_1$-$C_3$-Alkyl oder Nitro,

$R_4$ Wasserstoff, Brom oder Methyl, $R_5$ Wasserstoff, $R_6$ Wasserstoff oder Methyl, A $—CH_2—$, $—CH_2—CH_2—$ oder $—CH(CH_3)—$ und Z Cyan,

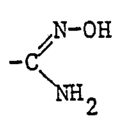 oder 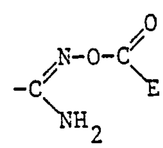

bedeuten, wobei

E für $—R_7$, $—OR_8$, $—SR_9$ oder $—NR_{10}R_{11}$ steht, worin

$R_7$ $C_1$-$C_7$-Alkyl, $C_1$-$C_3$-Alkyl, welches durch 1 bis 3 Chlor- oder Bromatome substituiert ist, $C_1$-$C_4$-Alkoxymethyl, $C_3$-$C_6$-Cycloalkyl, $C_2$-$C_3$-Alkenyl, Phenyl, welches unsubstituiert oder durch ein oder zwei Substituenten aus der Gruppe Chlor, Nitro und Methyl substituiert ist, Benzyl, welches unsubstituiert oder durch Chlor oder Nitro monosubstituiert ist, einen unsubstituierten oder durch Chlor oder Brom mono- oder disubstituierten Thiophen-, Furan-, Tetrahydrofuran- oder Pyrimidinring,

$R_8$ $C_1$-$C_4$-Alkyl, durch Chlor oder Brom monosubstituiertes Aethyl, $C_2$-$C_3$-Alkenyl, Propinyl, Phenyl, welches unsubstituiert oder Nitro monosubstituiert ist, oder Benzyl, welches unsubstituiert oder durch Nitro monosubstituiert ist,

$R_9$ $C_1$-$C_7$-Alkyl,

$R_{10}$ $C_1$-$C_4$-Alkyl, Chloräthyl, Phenyl, welches unsubstituiert oder durch ein oder zwei Substituenten aus der Gruppe Chlor, Methoxy und Trifluormethyl substituiert ist, und

$R_{11}$ Wasserstoff, Methyl oder Methoxy

oder $R_{10}$ und $R_{11}$ gemeinsam mit dem Stickstoffatom, an das sie gebunden sind, einen Piperidin- oder Morpholinring bedeuten, enthält.

19. Mittel nach Anspruch 18, dadurch gekennzeichnet, dass es eine Verbindung der Formel I, in

welcher

$R_1$ Wasserstoff, Chlor, Brom oder Jod, $R_2$ Wasserstoff, $R_3$ Wasserstoff, Chlor oder Nitro, $R_4$ und $R_5$ Wasserstoff, $R_6$ Wasserstoff oder Methyl,

A $—CH_2—$, $—CH_2—CH_2—$ oder $—CH(CH_3)—$ und Z Cyan,

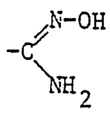 oder 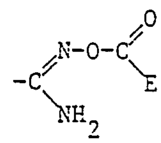

bedeuten, wobei

E für $—R_7$, $—OR_8$, $—SR_9$ oder $—NR_{10}R_{11}$ steht, worin

$R_7$ Methyl, Aethyl, n-Propyl, Isopropyl, n-Butyl, tert.Butyl, Isobutyl, Chlormethyl, Brommethyl, 2-Chloräthyl, 3-Chlor-n-propyl, 1,2-Dichloräthyl, Methoxymethyl, n-Propoxymethyl, sek.-Butoxymethyl, Cyclopropyl, Vinyl, 1-Propenyl, Isopropenyl, Phenyl, 2-Chlorphenyl, 4-Chlorphenyl, Benzyl, 2-Thienyl, 2-Furyl, 5-Brom-2-furyl, 2-Tetrahydrofuryl oder 2,4-Dichlorpyrimidin-5-yl,

$R_8$ Methyl, Aethyl, n-Propyl, n-Butyl, 2-Bromäthyl, Allyl, Phenyl oder Benzyl,

$R_9$ Aethyl, Isopropyl oder n-Pentyl,

$R_{10}$ Methyl, Aethyl, Isopropyl, n-Butyl, Phenyl, 3-Trifluormethylphenyl, 4-Chlorphenyl, 2,5-Dichlorphenyl, und

$R_{11}$ Wasserstoff oder Methoxy bedeuten, enthält.

20. Mittel nach Anspruch 19, dadurch gekennzeichnet, dass es eine Verbindung der Formel I, in welcher $R_1$ Wasserstoff, Chlor, Brom oder Jod, $R_2$ Wasserstoff, $R_3$ Wasserstoff oder Chlor, $R_4$ und $R_5$ Wasserstoff, $R_6$ Wasserstoff oder Methyl, A $—CH_2—$ und Z Cyan,

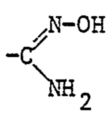 oder 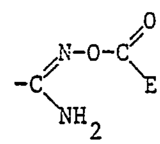

bedeuten, wobei

E für $—R_7$, $—OR_8$ oder $NR_{10}R_{11}$ steht, worin $R_7$ Chlormethyl, $R_8$ Methyl, $R_{10}$ Isopropyl und $R_{11}$ Wasserstoff bedeuten, enthält.

21. Mittel nach Anspruch 20, dadurch gekennzeichnet, dass es 5-Chlor-8-(cyanomethoxy)-chinolin enthält.

22. Mittel nach Anspruch 20, dadurch gekennzeichnet, dass es 5-Chlor-7-jod-8-(cyanomethoxy)-chinolin enthält.

23. Mittel nach Anspruch 20, dadurch gekennzeichnet, dass es O-(Methoxycarbonyl)-2-(8-chinolinoxy)-acetamidoxim enthält.

24. Mittel nach Anspruch 16, dadurch gekennzeichnet, dass es eine Verbindung der Formel I und ein Herbizid enthält.

25. Mittel nach Anspruch 24, dadurch gekennzeichnet, dass es als Herbizid eine Verbindung der Formel (A)

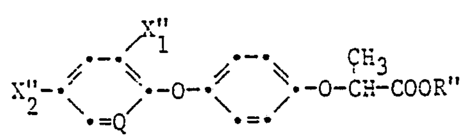 (A)

worin

$X_1''$ Wasserstoff oder Halogen,

$X_2''$ Wasserstoff, Halogen oder Trifluormethyl,

Q das Fragment =N— oder =CH—,

R'' $C_1$-$C_4$-Alkyl, welches unsubstituiert oder durch $C_1$-$C_4$-Alkoxy substituiert ist, $C_3$-$C_4$-Alkenyl, $C_3$-$C_4$-Alkinyl oder

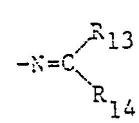

$R_{13}$ $C_1$-$C_4$-Alkyl, $R_{14}$ $C_1$-$C_4$-Alkyl oder $R_{13}$ und $R_{14}$ gemeinsam $C_4$-$C_5$-Alkylen bedeuten, enthält.

26. Mittel nach Anspruch 24, dadurch gekennzeichnet, dass es als Herbizid einen substituierten Pyridyloxyphenoxypropionsäureester enthält.

27. Mittel nach Anspruch 24, dadurch gekennzeichnet, dass es als Herbizid 2-[4-(3,5-Dichlorpyridyl-2-oxy)-phenoxy]-propionsäure-2-propinylester enthält.

28. Mittel nach Anspruch 24, dadurch gekennzeichnet, dass es als Herbizid einen Phenylharnstoff enthält.

29. Mittel nach Anspruch 24, dadurch gekennzeichnet, dass es als Herbizid einen Sulfonylharnstoff enthält.

30. Mittel nach Anspruch 24, dadurch gekennzeichnet, dass es als Verbindung der Formel I 5-Chlor-8-(cyanomethoxy)-chinolin und als Herbizid 2-[4-(3,5-Dichlorpyridyl-2-oxy)-phenoxy]-propionsäure-2-propinylester enthält.

31. Mittel nach Anspruch 16, dadurch gekennzeichnet, dass es eine Verbindung der Formel Ia

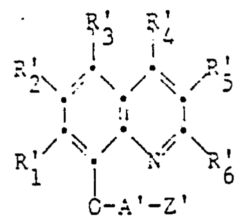

(Ia)

worin $R_1'$, $R_2'$ und $R_3'$ unabhängig voneinander Wasserstoff, Halogen, $C_1$-$C_3$-Alkyl, $C_1$-$C_3$-Alkoxy, Nitro oder Cyano,

$R_4'$, $R_5'$ und $R_6'$ unabhängig voneinander Wasserstoff, Halogen oder $C_1$-$C_3$-Alkyl,

A' eine der Gruppen $—CH_2—$, $—CH_2—CH_2—$ oder $—CH(CH_3)—$ und

Z' Cyan oder eine der Gruppen

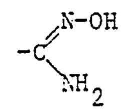

oder

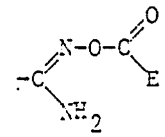

bedeuten, wobei

E für $—R_7$, $—OR_8$, $—SR_9$ oder $—NR_{10}R_{11}$ steht, worin

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, unter Einschluss ihrer Säureadditionssalze und Metallkomplexe, mit der Massgabe, dass Z' nicht für Cyan oder die Gruppe

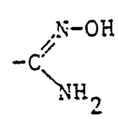

steht, wenn gleichzeitig $R_1'$, $R_2'$, $R_4'$, $R_5'$ und $R_6'$ Wasserstoff, $R'_3$ Wasserstoff oder Chlor und A' $—CH_2—$ oder $—CH(CH_3)—$ bedeuten, enthält.

32. Verfahren zur Herstellung von Verbindungen der Formel Ia

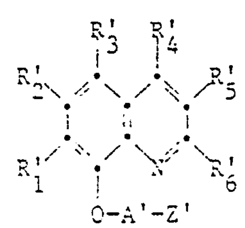

(Ia)

worin $R_1'$, $R_2'$ und $R_3'$ unabhängig voneinander Wasserstoff, Halogen, $C_1$-$C_3$-Alkyl, $C_1$-$C_3$-Alkoxy, Nitro oder Cyan,

$R_4'$, $R_5'$ und $R_6'$ unabhängig voneinander Wasserstoff, Halogen oder $C_1$-$C_3$-Alkyl,

A' eine der Gruppen $—CH_2—$, $CH_2—CH_2—$ oder $—CH(CH_3)—$ und

Z' Cyan oder Amidoxim, welches am Sauerstoffatom acyliert sein kann, bedeuten, unter Einschluss ihrer Säureadditionssalze und Metallkomplexe, mit der Massgabe, dass Z' nicht für Cyan oder Amidoxim steht, wenn gleichzeitig $R_1'$, $R_2'$, $R_4'$, $R_5'$ und $R_6'$ Wasserstoff $R_3'$ Wasserstoff oder Chlor und A' $—CH_2—$ oder $—CH(CH_3)—$ bedeuten, dadurch gekennzeichnet, dass man

a) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für die Gruppe $—CH_2—CH_2—$ und Z' für Cyan steht, eine Verbindung der Formel II

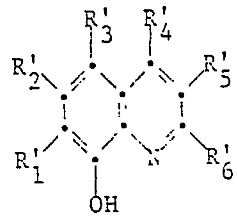

(II)

worin $R_1'$, $R_2'$, $R_3'$, $R_4'$, $R_5'$ und $R_6'$ die vorstehend angegebenen Bedeutungen haben, mit einer Verbindung der Formel III

$$CH_2{=}CH—CN \qquad (III)$$

umsetzt, oder

b) zur Herstellung von Verbindungen der Formel Ia, in denen $R_1'$, $R_2'$, $R_3'$, $R_4'$, $R_5'$ und $R_6'$ die für Formel Ia angegebenen Bedeutungen haben, A' für eine der Gruppen $—CH_2—$ oder $—CH(CH_3)—$ und Z' für Cyan steht, eine Verbindung der Formel II

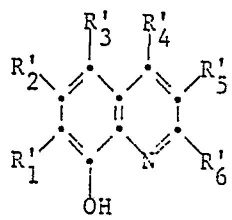

(II)

worin $R_1'$, $R_2'$, $R_3'$, $R_4'$, $R_5'$ und $R_6'$ die für Formel II angegebenen Bedeutungen haben, mit

i) einer Verbindung der Formel IV

$$Hal—A'—CN \qquad (IV)$$

worin Hal für ein Halogenatom steht und A' die vorstehend angegebene Bedeutung hat, umsetzt, oder

ii) einer Verbindung der Formel V

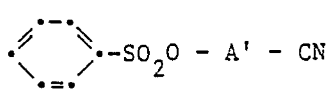

(V)

worin A' die vorstehend angegebene Bedeutung hat, umsetzt, oder

iii) einer Verbindung der Formel VI

$$Hal—A'—COOR_{12} \qquad (VI)$$

worin Hal für ein Halogenatom und $R_{12}$ für eine Alkylgruppe mit 1 bis 6 Kohlenstoffatomen stehen und A' die vorstehend angegebene Bedeutung hat, umsetzt und die erhaltenen Ester der Formel VII

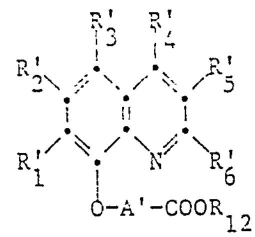

(VII)

worin $R_1'$, $R_2'$, $R_3'$, $R_4'$, $R_5'$, $R_6'$, A' und $R_{12}$ die vorstehend angegebenen Bedeutungen haben, mit Ammoniak in die entsprechenden Amide der Formel VIII

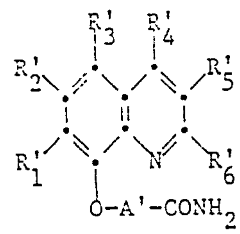

(VIII)

worin $R_1'$, $R_2'$, $R_3'$, $R_4'$, $R_5'$, $R_6'$ und A' die vorstehend angegebenen Bedeutungen haben, überführt und anschliessend dehydratisiert, und/oder

c) zur Herstellung derjenigen Verbindungen der Formel Ia, in denen $R_1'$, $R_2'$, $R_3'$, $R_4'$, $R_5'$, $R_6'$ and A' die für Formel Ia angegebenen Bedeutungen haben und Z' für Amidoxim, welches am Sauerstoffatom acyliert sein kann, steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Cyan steht, mit Hydroxylamin oder einem Säuresalz des Hydroxylamins umsetzt, und/oder

d) zur Herstellung derjenigen Verbindungen der Formel Ia, in denen $R_1'$, $R_2'$, $R_3'$, $R_4'$, $R_5'$, $R_6'$ und A' die für Formel Ia angegebenen Bedeutungen haben und Z' für acyliertes Amidoxim steht, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Amidoxim steht, acyliert.

33. Verfahren nach Anspruch 32, dadurch gekennzeichnet, dass man zur Herstellung derjenigen Verbindungen der Formel Ia, in denen $R'_1$, $R_2'$, $R_3'$, $R_4'$, $R_5'$, $R_6'$ und A' die für Formel Ia angegebenen Bedeutungen haben und Z' für acyliertes Amidoxim der Formel

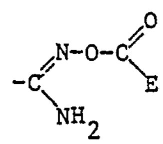

steht, wobei E —$R_7$, —$OR_8$, —$SR_9$ oder —$NR_{10}R_{11}$ ist, worin $R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O und S enthält und unsubstituiert oder durch Halogen substituiert ist, $R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist, $R_{11}$ Wasserstoff, $C_1$-$C_8$-Alkyl oder $C_1$-$C_3$-Alkoxy oder $R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten, eine Verbindung der Formel Ia, in welcher $R_1'$, $R_2'$, $R_3'$, $R_4'$, $R_5'$, $R_6'$ und A' die für Formel Ia angegebenen Bedeutungen haben und Z' für Amidoxim steht, mit einer Verbindung der Formel IX

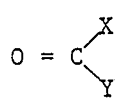

(IX)

worin X für ein Halogenatom und Y für —$R_7$, —$OR_8$, —$SR_9$ oder —$NR_{10}R_{11}$ steht, wobei $R_7$, $R_8$, $R_9$, $R_{10}$

und $R_{11}$ die vorstehend angegebenen Bedeutungen haben, oder X und Y gemeinsam für die Iminogruppe $=N-R_{10}$ stehen, umsetzt.

34. Verfahren zur selektiven Bekämpfung von Unkräutern in Kulturpflanzenbeständen, dadurch gekennzeichnet, dass man die Kulturpflanzenbestände, Teile der Kulturpflanzen oder Anbauflächen der Kulturpflanzen mit einem Herbizid und einer Verbindung der Formel I

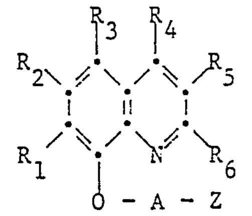

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder Amidoxim, welches am Sauerstoffatom acyliert sein kann, bedeuten, unter Einschluss ihrer Säureadditionssalze und Metallkomplexe, oder einem Mittel, welches eine dieser Verbindungen enthält, behandelt.

35. Mittel zur selektiven Bekämpfung von Unkräutern in Kulturpflanzenbeständen, dadurch gekennzeichnet, dass es eine Verbindung der Formel I

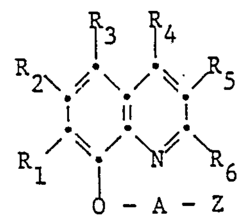

(I)

worin $R_1$, $R_2$ und $R_3$ unabhängig voneinander Wasserstoff, Halogen, $C_1$-$C_3$-Alkyl, $C_1$-$C_3$-Alkoxy, Nitro oder Cyan,

$R_4$, $R_5$ und $R_6$ unabhängig voneinander Wasserstoff, Halogen oder $C_1$-$C_3$-Alkyl,

A eine der Gruppen $-CH_2-$, $-CH_2-CH_2-$ oder $-CH(CH_3)-$ und

Z Cyan oder Amidoxim, welches am Sauerstoffatom acyliert sein kann, bedeuten, unter Einschluss ihrer Säureadditionssalze und Metallkomplexe, und ein Herbizid enthält.

**Claims** (for the Contracting States : DE, FR, CH, LI, IT, NL, BE, SE)

1. A method of protecting cultivated plants against the harmful effects of aggressive agricultural chemicals, which method comprises treating the cultivated plants or parts of these plants, or the soil intended for the growing of the cultivated plants, with a compound of the formula I

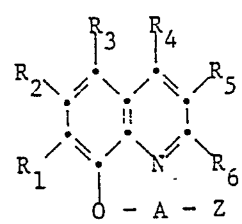

(I)

wherein

$R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is any one of the groups $-CH_2-$, $-CH_2-CH_2$ or $-CH(CH_3)-$, and

Z is cyano, or amide oxime which may be acylated at the oxygen atom, or an acid addition salt or a metal complex thereof, or with a composition containing such compound as active ingredient.

2. A method according to claim 1, which method comprises the use of a compound of the formula I in which $R_1$, $R_2$ and $R_3$ are each independently hydrogen, halogen, $C_1$-$C_3$-alkyl, $C_1$-$C_3$-alkoxy, nitro or

cyano ; $R_4$, $R_5$ and $R_6$ are each independently hydrogen, halogen or $C_1$-$C_3$-alkyl ; A is any one of the groups $-CH_2$, $-CH_2-CH_2-$ or $-CH(CH_3)-$ ; and Z is cyano, or one of the groups :

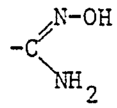
or
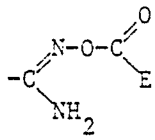

wherein E is $-R_7$, $-OR_8$, $-SR_9$ or $-NR_{10}R_{11}$, wherein $R_7$ is $C_1$-$C_7$-alkyl which is unsubstituted or substituted by halogen or $C_1$-$C_4$-alkoxy, or $R_7$ is $C_3$-$C_6$-cycloalkyl, $C_1$-$C_4$-alkenyl, $C_3$-$C_6$-alkynyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen, $R_8$, $R_9$ and $R_{10}$ are each independently $C_1$-$C_8$-alkyl which is unsubstituted or substituted by halogen, or are each $C_2$-$C_4$-alkenyl, $C_3$-$C_6$-alkynyl,l phenyl which is unsubstituted or substituted by halogen, $C_1$-$C_3$-alkyl, $C_1$-$C_3$-alkoxy, trifluormethyl or nitro, or they are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may contain a further hetero atom from the group N, O and S ; or an acid addition salt or a metal complex thereof, or a composition which contains such a compound as active ingredient.

3. A method according to claim 2, which method comprises the use of a compound of the formula I in which $R_1$ is hydrogen, chlorine, bromine, iodine or nitro ; $R_2$ is hydrogen ; $R_3$ is hydrogen, fluorine, chlorine, bromine, iodine, $C_1$-$C_3$-alkyl or nitro ; $R_4$ is hydrogen, bromine or methyl ; $R_5$ is hydrogen ; $R_6$ is hydrogen or methyl ; A is $-CH_2-$, $-CH_2-CH_2-$ or $-CH(CH_3)-$ ; and Z is cyano,

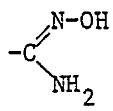
or
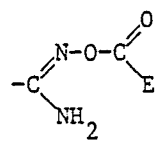

wherein E is $-R_7$, $-OR_8$, $-SR_9$ or $-NR_{10}R_{11}$, where $R_7$ is $C_1$-$C_7$-alkyl, $C_1$-$C_3$-alkyl which is substituted by 1 to 3 chlorine or bromine atoms, or is $C_1$-$C_4$-alkoxymethyl, $C_3$-$C_6$-cycloalkyl, $C_2$-$C_3$-alkenyl, phenyl which is unsubstituted or substituted by one or two substituents from the group : chlorine, nitro and methyl, or it is benzyl which is unsubstituted or monosubstituted by chlorine or nitro, or it is a thiophene, furan, tetrahydrofuran or pyrimidine ring, each of which is unsubstituted or mono- or disubstituted by chlorine or bromine, $R_8$ is $C_1$-$C_4$-alkyl, ethyl which is monosubstituted by chlorine or bromine, or it is $C_2$-$C_3$-alkenyl, propynyl, phenyl which is unsubstituted or monosubstituted by nitro, or it is benzyl which is unsubstituted or monosubstituted by nitro, $R_9$ is $C_1$-$C_7$-alkyl, $R_{10}$ is $C_1$-$C_4$-alkyl, chloroethyl, or phenyl which is unsubstituted or substituted by one or two substituents from the group : chlorine, methoxy and trifluoromethyl, and $R_{11}$ is hydrogen, methyl or methoxy, or $R_{10}$ and $R_{11}$, together with the nitrogen atom to which they are bound, form a piperidine or morpholine ring ; or a composition containing such a compound as active ingredient.

4. A method according to claim 3, which method comprises the use of a compound of the formula I in which $R_1$ is hydrogen, chlorine, bromine or iodine ; $R_2$ is hydrogen ; $R_3$ is hydrogen, chlorine or nitro ; $R_4$ and $R_5$ are hydrogen ; $R_6$ is hydrogen or methyl ; A is $-CH_2$, $-CH_2-CH_2-$ or $-CH(CH_3)-$ ; and Z is cyano,

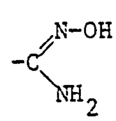
or
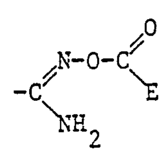

wherein E is $-R_7$, $-OR_8$, $-SR_9$ or $-NR_{10}R_{11}$, where $R_7$ is methyl, ethyl, n-propyl, isopropyl, n-butyl, tert-butyl, isobutyl, chloromethyl, bromomethyl, 2-chloroethyl, 3-chloro-n-propyl, 1,2-dichloroethyl, methoxymethyl, n-propoxymethyl, sec-butoxymethyl, cyclopropyl, vinyl, 1-propenyl, isopropenyl, phenyl, 2-chlorophenyl, 4-chlorophenyl, benzyl, 2-thienyl, 2-furyl, 5-bromo-2-furyl, 2-tetrahydrofuryl or 2,4-dichloropyrimidin-5-yl, $R_8$ is methyl, ethyl, n-propyl, n-butyl, 2-bromoethyl, allyl, phenyl or benzyl, $R_9$ is ethyl, isopropyl or n-pentyl, $R_{10}$ is methyl, ethyl, isopropyl, n-butyl, phenyl, 3-trifluoromethylphenyl, 4-chlorophenyl or 2,5-dichlorophenyl, and $R_{11}$ is hydrogen or methoxy ; or a composition containing such a compound as active ingredient.

5. A method according to claim 4, which method comprises the use of a compound of the formula I in which $R_1$ is hydrogen, chlorine, bromine or iodine ; $R_2$ is hydrogen ; $R_3$ is hydrogen or chlorine ; $R_4$ and

$R_5$ are hydrogen ; $R_6$ is hydrogen or methyl ; A is $—CH_2—$ ; and Z is cyano,

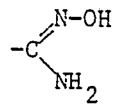

or

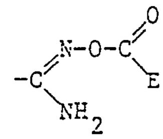

wherein E is $—R_7$, $—OR_8$ or $—NR_{10}R_{11}$, where $R_7$ is chloromethyl, $R_8$ is methyl, $R_{10}$ is isopropyl, and $R_{11}$ is hydrogen ; or a composition containing such a compound as active ingredient.

6. A method according to claim 5, which comprises the use of 5-chloro-8-(cyanomethoxy)quinoline or a composition containing this compound.

7. A method according to claim 5, which comprises the use of 5-chloro-7-iodo-8-(cyanomethoxy)quinoline or a composition containing this compound.

8. A method according to claim 5, which comprises the use of 0-(methoxycarbonyl)-2-(8-quinolinoxy)acetamide oxime or a composition containing this compound.

9. A method according to claim 1 for the protection of cultivated plants against harmful effects of plant protection products.

10. A method according to claim 9 for the protection of cultivated plants against harmful effects of herbicides.

11. A method according to claim 10 for the protection of cultivated plants against harmful effects of substituted pyridyloxyphenoxypropionic acid esters.

12. A method according to claim 11 for the protection of cultivated plants against harmful effects of 2-[4-(3,5-dichloropyridyl-2-oxy)-phenoxy]propionic acid 2-propynyl ester.

13. A method according to claim 10 for the protection of cultivated plants against harmful effects of phenylureas.

14. A method according to claim 10 for the protection of cultivated plants against harmful effects of sulfonylureas.

15. A method according to claim 1 for the protection of rice, maize, wheat, rye, barley, oats, cotton, sugar beet, sugar cane and soybeans.

16. A composition for the protection of cultivated plants against harmful effects of aggressive agricultural chemicals, which composition contains a compound of the formula I

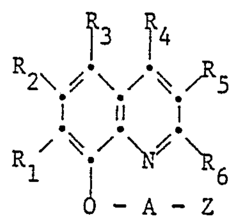

(I)

wherein

$R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is any one of the groups $—CH_2—$, $—CH_2—CH_2—$ or $—CH(CH_3)—$, and

Z is cyano, or amide oxime which may be acylated at the oxygen atom, or an acid addition salt or a metal complex thereof.

17. A composition according to claim 16, which composition contains a compound of the formula I in which $R_1$, $R_2$ and $R_3$ are each independently hydrogen, halogen, $C_1$-$C_3$-alkyl, $C_1$-$C_3$-alkoxy, nitro or cyano, $R_4$, $R_5$ and $R_6$ are each independently hydrogen, halogen or $C_1$-$C_3$-alkyl,

A is any one of the groups $—CH_2$, $—CH_2—CH_2$ or $—CH(CH_3)—$, and

Z is cyano or one of the groups

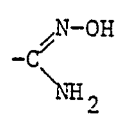

or

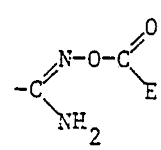

wherein E is $—R_7$, $—OR_8$, $—SR_9$ or $NR_{10}R_{11}$, wherein $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen,

$R_8$, $R_9$ and $R_{10}$ are each independently $C_1$-$C_8$-alkyl which is unsubstituted or substituted by halogen, or are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or they are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S ; or an acid addition salt or a metal complex thereof.

18. A composition according to claim 17, which composition contains a compound of the formula I in which

$R_1$ is hydrogen, chlorine, bromine, iodine or nitro,
$R_2$ is hydrogen,
$R_3$ is hydrogen, fluorine, chlorine, bromine, iodine, $C_1$-$C_3$-alkyl or nitro,
$R_4$ is hydrogen, bromine or methyl,
$R_5$ is hydrogen,
$R_6$ is hydrogen or methyl,
A is $-CH_2-$, $-CH_2-CH_2-$ or $-CH(CH_3)-$, and
Z is cyano,

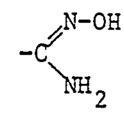 or 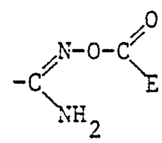

wherein E is $-R_7$, $-OR_8$, $-SR_9$ or $-NR_{10}R_{11}$, where $R_7$ is $C_1$-$C_7$-alkyl, $C_1$-$C_3$-alkyl which is substituted by 1 to 3 chlorine or bromine atoms, or it is $C_1$-$C_4$-alkoxymethyl, $C_3$-$C_6$-cycloalkyl, $C_2$-$C_3$-alkenyl, phenyl which is unsubstituted or substituted by one or two substituents from the group : chlorine, nitro and methyl, or it is benzyl which is unsubstituted or monosubstituted by chlorine or nitro, or it is a thiophene, furan, tetrahydrofuran or pyrimidine ring, each of which is unsubstituted or mono- or disubstituted by chlorine or bromine, $R_8$ is $C_1$-$C_4$-alkyl, ethyl which is monosubstituted by chlorine or bromide, or it is $C_2$-$C_3$-alkenyl, propynyl, phenyl which is unsubstituted or monosubstituted by nitro, or it is benzyl which is unsubstituted or monosubstituted by nitro, $R_9$ is $C_1$-$C_7$-alkyl, $R_{10}$ is $C_1$-$C_4$-alkyl, chloroethyl, or phenyl which is unsubstituted or substituted by one or two substituents from the group : chlorine, methoxy and trifluoromethyl, and $R_{11}$ is hydrogen, methyl or methoxy, or $R_{10}$ and $R_{11}$, together with the nitrogen atom to which they are bound, form a piperidine or morpholine ring.

19. A composition according to claim 18, which composition contains a compound of the formula I in which $R_1$ is hydrogen, chlorine, bromine or iodine ; $R_2$ is hydrogen ; $R_3$ is hydrogen, chlorine or nitro ; $R_4$ and $R_5$ are hydrogen ; $R_6$ is hydrogen or methyl ; A is $-CH_2-$, $-CH_2CH_2-$ or $-CH(CH_3)-$, and Z is cyano

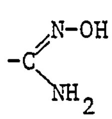 or 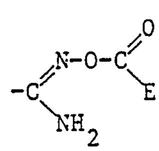

wherein E is $-R_7$, $-OR_8$, $-SR_9$ or $-NR_{10}R_{11}$, where $R_7$ is methyl, ethyl, n-propyl, isopropyl, n-butyl, tert-butyl, isobutyl, chloromethyl, bromomethyl, 2-chloroethyl, 3-chloro-n-propyl, 1,2-dichloroethyl, methoxymethyl, n-propoxymethyl, sec-butoxymethyl, cyclopropyl, vinyl, 1-propenyl, isopropenyl, phenyl, 2-chlorophenyl, 4-chlorophenyl, benzyl, 2-thienyl, 2-furyl, 5-bromo-2-furyl, 2-tetrahydrofuryl or 2,4-dichloropyrimidin-5-yl, $R_8$ is methyl, ethyl, n-propyl, n-butyl, 2-bromoethyl, allyl, phenyl or benzyl, $R_9$ is ethyl, isopropyl or n-pentyl, $R_{10}$ is methyl, ethyl, isopropyl, n-butyl, phenyl, 3-trifluoromethylphenyl, 4-chlorophenyl or 2,5-dichlorophenyl, and $R_{11}$ is hydrogen or methoxy.

20. A composition according to claim 19, which composition contains a compound of the formula I in which $R_1$ is hydrogen, chlorine, bromine or iodine ; $R_2$ is hydrogen ; $R_3$ is hydrogen or chlorine ; $R_4$ and $R_5$ are hydrogen ; $R_6$ is hydrogen or methyl ; A is $-CH_2-$ ; and Z is cyano

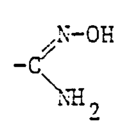 or 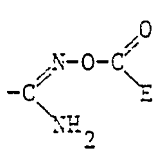

wherein E is $-R_7$, $-OR_8$ or $-NR_{10}R_{11}$, where $R_7$ is chloromethyl, $R_8$ is methyl, $R_{10}$ is isopropyl, and $R_{11}$ is hydrogen.

21. A composition according to claim 20 which contains 5-chloro-8-(cyanomethoxy) quinoline.

22. A composition according to claim 20 which contains 5-chloro-7-iodo-8-(cyanomethoxy) quinoline.

23. A composition according to claim 20 which contains O-(methoxycarbonyl)-2-(8-quinolinoxy) acetamide oxime.

24. A composition according to claim 16, which composition contains a compound of the formula I and a herbicide.

25. A composition according to claim 24, which composition contains, as herbicide, a compound of the formula (A)

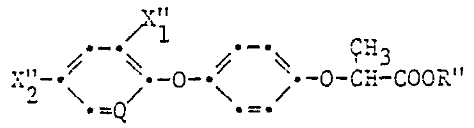

(A)

wherein

$X_1''$ is hydrogen or halogen,

$X_2''$ is hydrogen, halogen or trifluoromethyl,

Q is the fragment =N— or =CH—,

R'' is $C_1$-$C_4$-alkyl which is unsubstituted or substituted by $C_1$-$C_4$-alkoxy, or it is $C_3$-$C_4$-alkenyl, $C_3$-$C_4$-alkynyl or

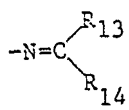

where $R_{13}$ is $C_1$-$C_4$-alkyl, $R_{14}$ is $C_1$-$C_4$-alkyl, or $R_{13}$ and $R_{14}$ together are $C_4$-$C_5$-alkylene.

26. A composition according to claim 24, which composition contains as herbicide a substituted pyridyloxyphenoxypropionic acid ester.

27. A composition according to claim 24, which composition contains as herbicide: 2-[4-(3,5-dichloropyridyl-2-oxy) phenoxy]propionic acid 2-propynyl ester.

28. A composition according to claim 24, which composition contains as herbicide a phenylurea.

29. A composition according to claim 24, which composition contains as herbicide a sulfonylurea.

30. A composition according to claim 24, which composition contains as compound of the formula I: 5-chloro-8-(cyanomethoxy)quinoline; and as herbicide: 2-[4-(3,5-dichloropyridyl-2-oxy) phenoxy]propionic acid 2-propynyl ester.

31. A composition according to claim 16, which composition contains a compound of the formula Ia

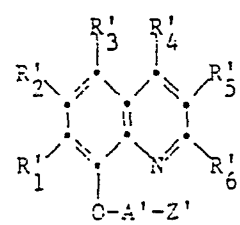

(Ia)

wherein $R_1'$, $R_2'$, and $R_3'$ are each independently hydrogen, halogen, $C_1$-$C_3$-alkyl, $C_1$-$C_3$-alkoxy, nitro or cyano,

$R_4'$, $R_5'$ and $R_6'$ are each independently hydrogen, halogen or $C_1$-$C_3$-alkyl,

A' is any one of the groups $—CH_2—$, $—CH_2—CH_2—$ or $—CH(CH_3)—$, and

Z' is cyano or one of the groups

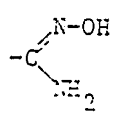

or

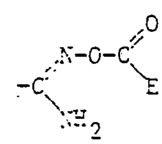

wherein

E is $—R_7$, $—OR_8$, $—SR_9$ or $—NR_{10}R_{11}$, where $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by

halogen, nitro or $C_1$-$C_3$-alkyl, or $R_7$ is a 5- or 6-membered heterocyclic ring which contains one or two hetero atoms from the group N, O and S, and which is unsubstituted or substituted by halogen ; $R_8$, $R_9$ and $R_{10}$ are each independently $C_1$-$C_8$-alkyl which is unsubstituted or substituted by halogen, or they are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S ; or an acid addition salt or a metal complex thereof, with the proviso that Z' is not cyano or the group

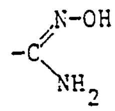

when simultaneously $R_1'$, $R_2'$, $R_4'$, $R_5'$, and $R_6'$ are hydrogen, $R_3'$ is hydrogen or chlorine, and A' is $-CH_2-$ or $-CH(CH_3)-$.

32. A compound of the formula Ia

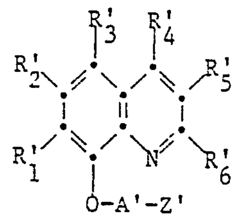

(Ia)

wherein $R_1'$, $R_2'$ and $R_3'$ are each independently hydrogen, halogen, $C_1$-$C_3$-alkyl, $C_1$-$C_3$-alkoxy, nitro or cyano,

$R_4'$, $R_5'$ and $R_6'$ are each independently hydrogen, halogen or $C_1$-$C_3$-alkyl,

A' is any one of the groups $-CH_2-$, $-CH_2-CH_2-$ or $-CH(CH_3)-$, and

Z' is cyano, or amide oxime which may be acylated at the oxygen atom,

or an acid addition salt or a metal complex thereof, with the proviso that Z' is not cyano or amide oxime when simultaneously $R_1'$, $R_2'$, $R_4'$, $R_5'$ and $R_6'$ are hydrogen, $R_3'$ is hydrogen or chlorine, and A' is $-CH_2$ or $-CH(CH_3)-$.

33. A compound according to claim 32, wherein

$R_1'$, $R_2'$ and $R_3'$ are each independently hydrogen, halogen, $C_1$-$C_3$-alkyl, $C_1$-$C_3$-alkoxy, nitro or cyano,

$R_4'$, $R_5'$ and $R_6'$ are each hydrogen, halogen or $C_1$-$C_3$-alkyl,

A' is any one of the groups $-CH_2-$, $-CH_2-CH_2-$ or $-CH(CH_3)-$, and

Z' is cyano, or either one of the groups

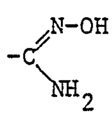

or

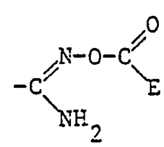

in which E is $-R_7$, $-OR_8$, $-SR_9$ or $-NR_{10}R_{11}$, wherein $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen ; $R_8$, $R_9$ and $R_{10}$ are each independently $C_1$-$C_8$-alkyl which is unsubstituted or substituted by halogen, or are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or are each benzyl which is unsbustituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S ; or an acid addition salt or a metal complex thereof, with the proviso that Z' is not cyano or amide oxime when simultaneously $R_1'$, $R_2'$, $R_4'$, $R_5'$ and $R_6'$ are hydrogen, $R_3'$ is hydrogen or chlorine, and A' is $-CH_2$ or $-CH(CH_3)-$.

34. A compound according to claim 33, wherein $R_1'$ is hydrogen, chlorine, bromine, iodine or nitro ; $R_2'$ is hydrogen ; $R_3'$ is hydrogen, fluorine, chlorine, bromine, iodine, $C_1$-$C_3$-alkyl or nitro ; $R_4'$ is hydrogen, bromine or methyl ; $R_5'$ is hydrogen ; $R_6'$ is hydrogen or methyl ; A' is $-CH_2-$, $-CH_2-CH_2-$

or —CH($CH_3$)— ; and Z' is cyano,

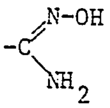 or 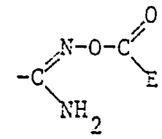

in which

E is —$R_7$, —$OR_8$, —$SR_9$ or —$NR_{10}R_{11}$, wherein $R_7$ is $C_1$-$C_7$-alkyl, $C_1$-$C_3$-alkyl which is substituted by 1 to 3 chlorine or bromine atoms, or $R_7$ is $C_1$-$C_4$-alkoxymethyl, $C_3$-$C_6$-cycloalkyl, $C_2$-$C_3$-alkenyl, phenyl which is unsubstituted or substituted by one or two substituents from the group chlorine, nitro and methyl, or it is benzyl which is unsubstituted or monosubstituted by chlorine or nitro, or $R_7$ is a thiophene, furan, tetrahydrofuran or pyrimidine ring, each of which is unsubstituted or mono- or disubstituted by chlorine or bromine, $R_8$ is $C_1$-$C_4$-alkyl, ethyl monosubstituted by chlorine or bromine, or it is $C_2$-$C_3$-alkenyl, propynyl, phenyl which is unsubstituted or monosubstituted by nitro, or it is benzyl which is unsubstituted or monosubstituted by nitro, $R_9$ is $C_1$-$C_7$-alkyl, $R_{10}$ is $C_1$-$C_4$-alkyl, chloroethyl, or phenyl which is unsubstituted or substituted by one or two substituents from the group chlorine, methoxy and trifluoromethyl, and $R_{11}$ is hydrogen, methyl or methoxy, or $R_{10}$ and $R_{11}$, together with the nitrogen atom to which they are bound, form a piperidine or morpholine ring, with the proviso that Z' is not cyano or amide oxime when simultaneously $R_1'$, $R_2'$, $R_4'$, $R_5'$ and $R_6'$ are hydrogen, $R_3'$ is hydrogen or chlorine, and A' is —$CH_2$— or —CH($CH_3$)—.

35. A compound according to claim 34, wherein $R_1'$ is hydrogen, chlorine, bromine or iodine ; $R_2'$ is hydrogen ; $R_3'$ is hydrogen, chlorine or nitro ; $R_4'$ and $R_5'$ are hydrogen ; $R_6'$ is hydrogen or methyl ; A' is —$CH_2$—, —$CH_2$—$CH_2$— or —CH($CH_3$)—, and Z' is cyano,

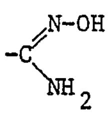 or 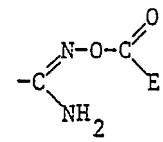

in which

E is —$R_7$, —$OR_8$, —$SR_9$ or —$NR_{10}R_{11}$, wherein $R_7$ is methyl, ethyl, n-propyl, isopropyl, n-butyl, tert-butyl, isobutyl, chloromethyl, bromomethyl, 2-chloroethyl, 3-chloro-n-propyl, 1,2-dichloroethyl, methoxymethyl, n-propoxymethyl, sec-butoxymethyl, cyclopropyl, vinyl, 1-propenyl, isopropenyl, phenyl, 2-chlorophenyl, 4-chlorophenyl, benzyl, 2-thienyl, 2-furyl, 5-bromo-2-furyl, 2-tetrahydrofuryl or 2,4-dichloropyrimidin-5-yl, $R_8$ is methyl, ethyl, n-propyl, n-butyl, 2-bromoethyl, allyl, phenyl or benzyl, $R_9$ is ethyl, isopropyl or n-pentyl, $R_{10}$ is methyl, ethyl, isopropyl, n-butyl, phenyl, 3-trifluoromethylphenyl, 4-chlorophenyl or 2,5-dichlorophenyl, and $R_{11}$ is hydrogen or methoxy, with the proviso that Z' is not cyano or amide oxime when $R_1'$, $R_2'$, $R_4'$, $R_5'$ and $R_6'$ are hydrogen, $R_3'$ is hydrogen or chlorine, and A' is —$CH_2$— or —CH($CH_3$)—.

36. A compound according to claim 35, wherein $R_1'$ is hydrogen, chlorine, bromine or iodine ; $R_2'$ is hydrogen ; $R_3'$ is hydrogen or chlorine ; $R_4'$ and $R_5'$ are hydrogen ; $R_6$ is hydrogen or methyl ; A' is —$CH_2$— ; and Z' is cyano,

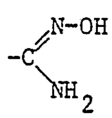 or 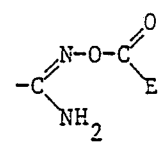

in which E is —$R_7$, —$OR_8$ or $NR_{10}R_{11}$, wherein $R_7$ is chloromethyl, $R_8$ is methyl, $R_{10}$ is isopropyl, and $R_{11}$ is hydrogen, with the priviso that Z' is not cyano or amide oxime when simultaneously $R_1'$, $R_2'$, $R_4'$, $R_5'$ and $R_6'$ are hydrogen, $R_3'$ is hydrogen or chlorine, and A' is —$CH_2$— or —CH($CH_3$)—.

37. 2-Methyl-8-(cyanomethoxy)quinoline.

38. 2-(2-Methyl-8-quinolinoxy)acetamide oxime.

39. O-(Isopropylaminocarbonyl)-2-(8-quinolinoxy)acetamide oxime.

40. O-(Chloromethylcarbonyl)-2-(8-quinolinoxy)acetamide oxime.

41. 2-(5-Chloro-7-bromo-8-quinolinoxy)acetamide oxime.

42. 5-Chloro-7-bromo-8-(cyanomethoxy)quinoline.

43. O-(Methoxycarbonyl)-2-(8-quinolinoxy)acetamide oxime.

44. 2-(5-Chloro-7-iodo-8-quinolinoxy)acetamide oxime.

45. O-(Isopropylaminocarbonyl)-2-(5-chloro-7-bromo-8-quinolinoxy)-acetamide oxime.

46. 2-(2-Methyl-5,7-dichloro-8-quinolinoxy)acetamide oxime.

47. 5,7-Dichloro-8-(cyanomethoxy)quinoline.
48. O-(Isopropylaminocarbonyl)-2-(5-chloro-7-iodo-8-quinolinoxy)-acetamide oxime.
49. 2-Methyl-5,7-dichloro-8-(cyanomethoxy)quinoline.
50. O-(Isopropyloaminocarbonyl)-2-(2-methyl-5,7-dichloro-5,7-dichloro-8-quinolinoxy)acetamide oxime.
51. 5-Chloro-7-iodo-8-(cyanomethoxy)quinoline.
52. A process for the preparation of a compound of the formula Ia

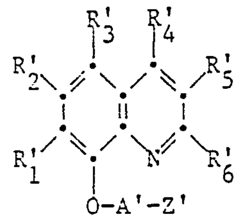

(Ia)

wherein $R_1'$, $R_2'$ and $R_3'$ are each independently hydrogen, halogen, $C_1$-$C_3$-alkyl, $C_1$-$C_3$-alkoxy, nitro or cyano,

$R_4'$, $R_5'$ and $R_6'$ are each independently hydrogen, halogen or $C_1$-$C_3$-alkyl,

A' is any one of the groups $-CH_2-$, $-CH_2-CH_2-$ or $-CH(CH_3)-$, and

Z' is cyano, or amide oxime which may be acylated at the oxygen atom,

or of an acid addition salt or a metal complex thereof, with the proviso that Z' is not cyano or amide oxime when simultaneously $R_1'$, $R_2'$, $R_4'$, $R_5'$ and $R_6'$ are hydrogen, $R_3'$ is hydrogen or chlorine, and A' is $-CH_2-$ or $-CH(CH_3)-$,

which process is characterised in that:

a) a compound of the formula Ia in which $R_1'$, $R_2'$, $R_3'$, $R_4'$, $R_5'$ and $R_6'$ are as defined for the formula Ia, A' is the group $-CH_2-CH_2-$, and Z' is cyano, is prepared by reacting a compound of the formula II

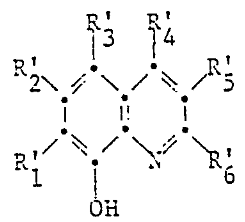

(II)

wherein $R_1'$, $R_2'$, $R_3'$, $R_4'$, $R_5'$, and $R_6'$ are as defined above, with a compound of the formula III

$$CH_2{=}CH-CN \qquad (III)$$

or

b) a compound of the formula Ia in which $R_1'$, $R_2'$, $R_3'$, $R_4'$, $R_5'$ and $R_6'$ are as defined for the formula Ia, A' is either the group $-CH_2-$ or $-CH(CH_3)-$, and Z' is cyano, is prepared by reacting a compound of the formula II

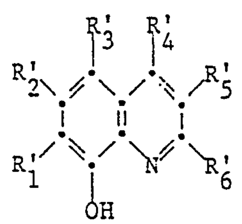

(II)

wherein $R_1'$, $R_2'$, $R_3'$, $R_4'$, $R_5'$ and $R_6'$ are as defined for the formula II

i) with a compound of the formula IV

$$Hal-A'-CN \qquad (IV)$$

wherein Hal is a halogen atom, and A' is as defined above, or

ii) with a compound of the formula V

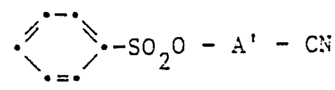

(V)

wherein A' is as defined above, or

iii) with a compound of the formula VI

$$Hal—A'—COOR_{12} \quad (VI)$$

wherein Hal is a halogen atom, and $R_{12}$ is an alkyl group having 1 to 6 carbon atoms, and A' is as defined above ; and converting the resultant ester of the formula VII

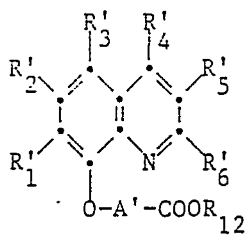

(VII)

wherein $R_1'$, $R_2'$, $R_3'$, $R_4'$, $R_5'$, $R_6'$, A' and $R_{12}$ are as defined above, with ammonia into the corresponding amide of the formula VIII

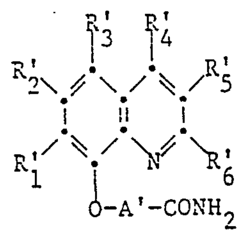

(VIII)

wherein $R_1'$, $R_2'$, $R_3'$, $R_4'$, $R_5'$, $R_6'$ and A' are as defined above, and subsequently dehydrating the product obtained ; and/or

c) a compound of the formula Ia in which $R_1'$, $R_2'$, $R_3'$, $R_4'$, $R_5'$, $R_6'$ and A' are as defined for the formula Ia, and Z' is amide oxime which may be acylated at the oxygen atom, is prepared by reacting a compound of the formula Ia wherein $R_1'$, $R_2'$, $R_3'$, $R_4'$, $R_5'$, $R_6'$ and A' are as defined for the formula Ia, and Z' is cyano, with hydroxylamine or with an acid salt of hydroxylamine ; and/or

d) a compound of the formula Ia in which $R_1'$, $R_2'$, $R_3'$, $R_4'$, $R_5'$, $R_6'$ and A' are as defined for the formula Ia, and Z' is acylated amide oxime, is prepared by acylating a compound of the formula Ia wherein $R_1'$, $R_2'$, $R_3'$, $R_4'$, $R_5'$, $R_6'$ and A' are as defined for the formula Ia, and Z' is amide oxime.

53. A process according to claim 52, characterised in that a compound of the formula Ia in which $R_1'$, $R_2'$, $R_3'$, $R_4'$, $R_5'$, $R_6'$ and A' are as defined for the formula Ia, and Z' is acylated amide oxime of the formula

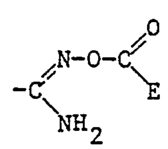

wherein E is $—R_7$, $—OR_8$, $—SR_9$ or $—NR_{10}R_{11}$ in which $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen, $R_8$, $R_9$ and $R_{10}$ are each independently $C_1$-$C_8$-alkyl which is unsubstituted or substituted by halogen, or they are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or are each benzyl which is unsubstituted or substituted by halogen or nitro, and $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S, is produced by reacting a compound of the

formula Ia, wherein $R_1'$, $R_2'$, $R_3'$, $R_4'$, $R_5'$, $R_6'$ and A' are as defined for the formula Ia, and Z' is amide oxime, with a compound of the formula IX

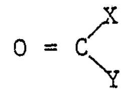

(IX)

in which X is a halogen atom, and Y is $—R_7$, $—OR_8$, $—SR_9$ or $—NR_{10}R_{11}$, wherein $R_7$, $R_8$, $R_9$, $R_{10}$ and $R_{11}$ are as defined above, or X and Y together are the imino group $=N—R_{10}$.

54. A method of selectively controlling weeds in crops of cultivated plants, which method comprises treating the cultivated plants or parts of the cultivated plants, or cultivated areas for cultivated plants, with a herbicide, and a compound of the formula I

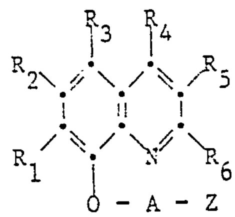

(I)

wherein $R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is a group $—CH_2—$, $—CH_2—CH_2—$ or $—CH(CH_3)—$, and

Z is cyano, or amide oxime which may be acylated at the oxygen atom,

or an acid addition salt or a metal complex thereof, or a composition containing such a compound.

55. A composition for selectively controlling weeds in crops of cultivated plants, which composition contains a compound of the formula I

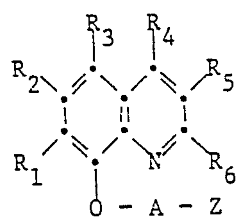

(I)

wherein $R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is any one of the groups $—CH_2—$, $—CH_2—CH_2—$ or $—CH(CH_3)—$, and

Z is cyano, or amide oxime which may be acylated at the oxygen atom,

or an acid addition salt or a metal complex thereof, and a herbicide.

**Claims** (for the Contracting State AT)

1. A method of protecting cultivated plants against the harmful effects of aggressive agricultural chemicals, which method comprises treating the cultivated plants or parts of these plants, or the soil intended for the growing of the cultivated plants, with a compound of the formula I

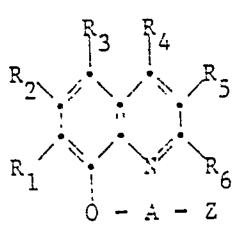

(I)

wherein

$R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,
A is any one of the groups $—CH_2—$, $—CH_2—CH_2$ or $—CH(CH_3)—$, and
Z is cyano, or amide oxime which may be acylated at the oxygen atom,
or an acid addition salt or a metal complex thereof, or with a composition containing such compound as active ingredient.

2. A method according to claim 1, which method comprises the use of a compound of the formula I in which $R_1$, $R_2$ and $R_3$ are each independently hydrogen, halogen, $C_1$-$C_3$-alkyl, $C_1$-$C_3$-alkoxy, nitro or cyano ; $R_4$, $R_5$ and $R_6$ are each independently hydrogen, halogen or $C_1$-$C_3$-alkyl ; A is any one of the groups $—CH_2$, $—CH_2—CH_2—$ or $—CH(CH_3)—$ ; and Z is cyano, or one of the groups :

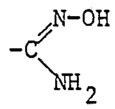 or 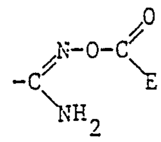

wherein E is $—R_7$, $—OR_8$, $—SR_9$ or $—NR_{10}R_{11}$, wherein $R_7$ is $C_1$-$C_7$-alkyl which is unsubstituted or substituted by halogen or $C_1$-$C_4$-alkoxy, or $R_7$ is $C_3$-$C_6$-cycloalkyl, $C_1$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen, $R_8$, $R_9$ and $R_{10}$ are each independently $C_1$-$C_8$-alkyl which is unsubstituted or substituted by halogen, or are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or they are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may contain a further hetero atom from the group N, O and S ; or an acid addition salt or a metal complex thereof, or a composition which contains such a compound as active ingredient.

3. A method according to claim 2, which method comprises the use of a compound of the formula I in which $R_1$ is hydrogen, chlorine, bromine, iodine or nitro ; $R_2$ is hydrogen ; $R_3$ is hydrogen, fluorine, chlorine, bromine, iodine, $C_1$-$C_3$-alkyl or nitro ; $R_4$ is hydrogen, bromine or methyl ; $R_5$ is hydrogen ; $R_6$ is hydrogen or methyl ; A is $—CH_2—$, $—CH_2—CH_2—$ or $—CH(CH_3)—$ ; and Z is cyano,

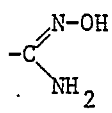 or 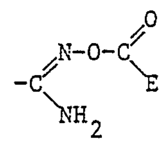

wherein E is $—R_7$, $—OR_8$, $—SR_9$ or $—NR_{10}R_{11}$, where $R_7$ is $C_1$-$C_7$-alkyl, $C_1$-$C_3$-alkyl which is substituted by 1 to 3 chlorine or bromine atoms, or is $C_1$-$C_4$-alkoxymethyl, $C_3$-$C_6$-cycloalkyl, $C_2$-$C_3$-alkenyl, phenyl which is unsubstituted or substituted by one or two substituents from the group : chlorine, nitro and methyl, or it is benzyl which is unsubstituted or monosubstituted by chlorine or nitro, or it is a thiophene, furan, tetrahydrofuran or pyrimidine ring, each of which is unsubstituted or mono- or disubstituted by chlorine or bromine, $R_8$ is $C_1$-$C_4$-alkyl, ethyl which is monosubstituted by chlorine or bromine, or it is $C_2$-$C_3$-alkenyl, propynyl, phenyl which is unsubstituted or monosubstituted by nitro, or it is benzyl which is unsubstituted or monosubstituted by nitro, $R_9$ is $C_1$-$C_7$-alkyl, $R_{10}$ is $C_1$-$C_4$-alkyl, chloroethyl, or phenyl which is unsubstituted or substituted by one or two substituents from the group : chlorine, methoxy and trifluoromethyl, and $R_{11}$ is hydrogen, methyl or methoxy, or $R_{10}$ and $R_{11}$, together with the nitrogen atom to which they are bound, form a piperidine or morpholine ring ; or a composition containing such a compound as active ingredient.

4. A method according to claim 3, which method comprises the use of a compound of the formula I in which $R_1$ is hydrogen, chlorine, bromine or iodine ; $R_2$ is hydrogen ; $R_3$ is hydrogen, chlorine or nitro ; $R_4$ and $R_5$ are hydrogen ; $R_6$ is hydrogen or methyl ; A is $—CH_2$; $—CH_2—CH_2—$ or $—CH(CH_3)—$ ; and Z is cyano,

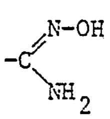 or 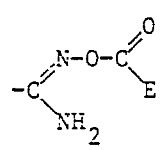

wherein E is $—R_7$, $—OR_8$, $—SR_9$ or $—NR_{10}R_{11}$, where $R_7$ is methyl, ethyl, n-propyl, isopropyl, n-butyl, tert-butyl, isobutyl, chloromethyl, bromomethyl, 2-chloroethyl, 3-chloro-n-propyl, 1,2-dichloroethyl, methoxymethyl, n-propoxymethyl, sec-butoxymethyl, cyclopropyl, vinyl, 1-propenyl, isopropenyl, phenyl,

2-chlorophenyl, 4-chlorophenyl, benzyl, 2-thienyl, 2-furyl, 5-bromo-2-furyl, 2-tetrahydrofuryl or 2,4-dichloropyrimidin-5-yl, $R_8$ is methyl, ethyl, n-propyl, n-butyl, 2-bromoethyl, allyl, phenyl or benzyl, $R_9$ is ethyl, isopropyl or n-pentyl, $R_{10}$ is methyl, ethyl, isopropyl, n-butyl, phenyl, 3-trifluoromethylphenyl, 4-chlorophenyl or 2,5-dichlorophenyl, and $R_{11}$ is hydrogen or methoxy ; or a composition containing such a compound as active ingredient.

5. A method according to claim 4, which method comprises the use of a compound of the formula I in which $R_1$ is hydrogen, chlorine, bromine or iodine ; $R_2$ is hydrogen ; $R_3$ is hydrogen or chlorine ; $R_4$ and $R_5$ are hydrogen ; $R_6$ is hydrogen or methyl ; A is $-CH_2-$ ; and Z is cyano.

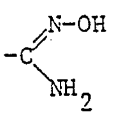

or

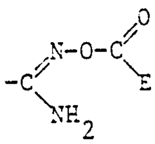

wherein E is $-R_7$, $-OR_8$ or $-NR_{10}R_{11}$, where $R_7$ is chloromethyl, $R_8$ is methyl, $R_{10}$ is isopropyl, and $R_{11}$ is hydrogen ; or a composition containing such a compound as active ingredient.

6. A method according to claim 5, which comprises the use of 5-chloro-8-(cyanomethoxy)quinoline or a composition containing this compound.

7. A method according to claim 5, which comprises the use of 5-chloro-7-iodo-8-(cyanomethoxy)quinoline or a composition containing this compound.

8. A method according to claim 5, which comprises the use of O-(methoxycarbonyl)-2-(quinolinoxy) acetamide oxime or a composition containing this compound.

9. A method according to claim 1 for the protection of cultivated plants against harmful effects of plant protection products.

10. A method according to claim 9 for the protection of cultivated plants against harmful effects of herbicides.

11. A method according to claim 10 for the protection of cultivated plants against harmful effects of substituted pyridyloxyphenoxypropionic acid esters.

12. A method according to claim 11 for the protection of cultivated plants against harmful effects of 2-[4-(3,5-dichloropyridyl-2-oxy)-phenoxy]propionic acid 2-propynyl ester.

13. A method according to claim 10 for the protection of cultivated plants against harmful effects of phenylureas.

14. A method according to claim 10 for the protection of cultivated plants against harmful effects of sulfonylureas.

15. A method according to claim 1 for the protection of rice, maize, wheat, rye, barley, oats, cotton, sugar beet, sugar cane and soybeans.

16. A composition for the protection of cultivated plants against harmful effects of aggressive agricultural chemicals, which composition contains a compound of the formula I

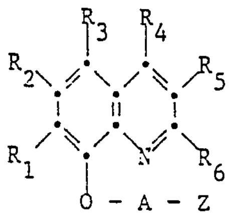

(I)

wherein

$R_1$, $R_2$ and $R_3$ are each independently hydrogen, halogen, $C_1$-$C_3$-alkyl, $C_1$-$C_3$-alkoxy, nitro or cyano,
$R_4$, $R_5$ and $R_6$ are each independently hydrogen, halogen or $C_1$-$C_3$-alkyl,
A is any one of the groups $-CH_2-$, $-CH_2-CH_2-$ or $-CH(CH_3)-$, and
Z is cyano, or amide oxime which may be acylated at the oxygen atom,
or an acid addition salt or a metal complex thereof.

17. A composition according to claim 16, which composition contains a compound of the formula I in which $R_1$, $R_2$ and $R_3$ are each independently hydrogen, halogen, $C_1$-$C_3$-alkyl, $C_1$-$C_3$-alkoxy, nitro or cyano, $R_4$, $R_5$ and $R_6$ are each independently hydrogen, halogen or $C_1$-$C_3$-alkyl,
A is any one of the groups $-CH_2$, $-CH_2-CH_2$ or $-CH(CH_3)-$, and
Z is cyano or one of the groups

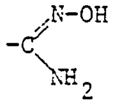

or

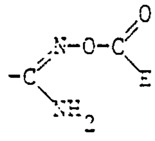

wherein E is $—R_7$, $—OR_8$, $—SR_9$ or $—NR_{10}R_{11}$, wherein $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen, $R_8$, $R_9$ and $R_{10}$ are each independently $C_1$-$C_8$-alkyl which is unsubstituted or substituted by halogen, or are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or they are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S ; or an acid addition salt or a metal complex thereof.

18. A composition according to claim 17, which composition contains a compound of the formula I in which

$R_1$ is hydrogen, chlorine, bromine, iodine or nitro,
$R_2$ is hydrogen,
$R_3$ is hydrogen, fluorine, chlorine, bromine, iodine, $C_1$-$C_3$-alkyl or nitro,
$R_4$ is hydrogen, bromine or methyl,
$R_5$ is hydrogen,
$R_6$ is hydrogen or methyl,
A is $—CH_2—$, $—CH_2—CH_2—$ or $—CH(CH_3)—$, and
Z is cyano,

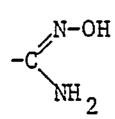 or 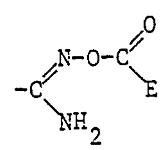

wherein E is $—R_7$, $—OR_8$, $—SR_9$ or $—NR_{10}R_{11}$, where $R_7$ is $C_1$-$C_7$-alkyl, $C_1$-$C_3$-alkyl which is substituted by 1 to 3 chlorine or bromine atoms, or it is $C_1$-$C_4$-alkoxymethyl, $C_3$-$C_6$-cycloalkyl, $C_2$-$C_3$-alkenyl, phenyl which is unsubstituted or substituted by one or two substituents from the group : chlorine, nitro and methyl, or it is benzyl which is unsubstituted or monosubstituted by chlorine or nitro, or it is a thiophene, furan, tetrahydrofuran or pyrimidine ring, each of which is unsubstituted or mono- or disubstituted by chlorine or bromine, $R_8$ is $C_1$-$C_4$-alkyl, ethyl which is monosubstituted by chlorine or bromine, or it is $C_2$-$C_3$-alkenyl, propynyl, phenyl which is unsubstituted or monosubstituted by nitro, or it is benzyl which is unsubstituted or monosubstituted by nitro, $R_9$ is $C_1$-$C_7$-alkyl, $R_{10}$ is $C_1$-$C_4$-alkyl, chloroethyl, or phenyl which is unsubstituted or substituted by one or two substituents from the group : chlorine, methoxy and trifluoromethyl, and $R_{11}$ is hydrogen, methyl or methoxy, or $R_{10}$ and $R_{11}$, together with the nitrogen atom to which they are bound, form a piperidine or morpholine ring.

19. A composition according to claim 18, which composition contains a compound of the formula I in which $R_1$ is hydrogen, chlorine, bromine or iodine ; $R_2$ is hydrogen ; $R_3$ is hydrogen, chlorine or nitro ; $R_4$ and $R_5$ are hydrogen ; $R_6$ is hydrogen or methyl ; A is $—CH_2—$, $—CH_2CH_2—$ or $—CH(CH_3)—$, and Z is cyano

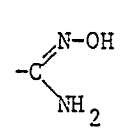 or 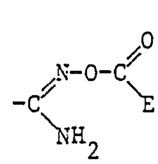

wherein E is $—R_7$, $—OR_8$, $—SR_9$ or $—NR_{10}R_{11}$, where $R_7$ is methyl, ethyl, n-propyl, isopropyl, n-butyl, tert-butyl, isobutyl, chloromethyl, bromomethyl, 2-chloroethyl, 3-chloro-n-propyl, 1,2-dichloroethyl, methoxymethyl, n-propoxymethyl, sec-butoxymethyl, cyclopropyl, vinyl, 1-propenyl, isopropenyl, phenyl, 2-chlorophenyl, 4-chlorophenyl, benzyl, 2-thienyl, 2-furyl, 5-bromo-2-furyl, 2-tetrahydrofuryl or 2,4-dichloropyrimidin-5-yl, $R_8$ is methyl, ethyl, n-propyl, n-butyl, 2-bromoethyl, allyl, phenyl or benzyl, $R_9$ is ethyl, isopropyl or n-pentyl, $R_{10}$ is methyl, ethyl, isopropyl, n-butyl, phenyl, 3-trifluoromethylphenyl, 4-chlorophenyl or 2,5-dichlorophenyl, and $R_{11}$ is hydrogen or methoxy.

20. A composition according to claim 19, which composition contains a compound of the formula I in which $R_1$ is hydrogen, chlorine, bromine or iodine ; $R_2$ is hydrogen ; $R_3$ is hydrogen or chlorine ; $R_4$ and $R_5$ are hydrogen ; $R_6$ is hydrogen or methyl ; A is $—CH_2—$ ; and Z is cyano

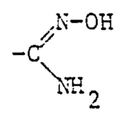 or 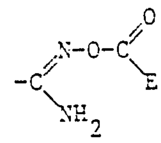

wherein E is $-R_7$, $OR_8$ or $-NR_{10}R_{11}$, where $R_7$ is chloromethyl, $R_8$ is methyl, $R_{10}$ is isopropyl, and $R_{11}$ is hydrogen.

21. A composition according to claim 20 which contains 5-chloro-8-(cyanomethoxy)quinoline.

22. A composition according to claim 20 which contains 5-chloro-7-iodo-8-(cyanomethoxy)quinoline.

23. A composition according to claim 20 which contains O-(methoxycarbonyl)-2-(8-quinolinoxy)acetamide oxime.

24. A composition according to claim 16, which composition contains a compound of the formula I and a herbicide.

25. A composition according to claim 24, which composition contains, as herbicide, a compound of the formula (A)

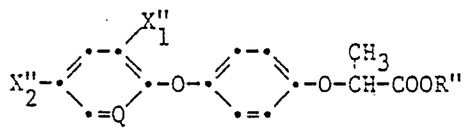

(A)

wherein

$X_1''$ is hydrogen or halogen,

$X_2''$ is hydrogen, halogen or trifluoromethyl,

Q is the fragment =N— or =CH—,

R'' is $C_1$-$C_4$-alkyl which is unsubstituted or substituted by $C_1$-$C_4$-alkoxy, or it is $C_3$-$C_4$-alkenyl, $C_3$-$C_4$-alkynyl or

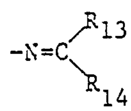

where $R_{13}$ is $C_1$-$C_4$-alkyl, $R_{14}$ is $C_1$-$C_4$-alkyl, or $R_{13}$ and $R_{14}$ together are $C_4$-$C_5$-alkylene.

26. A composition according to claim 24, which composition contains as herbicide a substituted pyridyloxyphenoxypropionic acid ester.

27. A composition according to claim 24, which composition contains as herbicide: 2-[4-(3,5-dichloropyridyl-2-oxy)phenoxy] propionic acid 2-propynyl ester.

28. A composition according to claim 24, which composition contains as herbicide a phenylurea.

29. A composition according to claim 24, which composition contains as herbicide a sulfonylurea.

30. A composition according to claim 24, which composition contains as compound of the formula I: 5-chloro-8-(cyanomethoxy)quinoline; and as herbicide: 2-[4-(3,5-dichloropyridyl-2-oxy)phenoxy] propionic acid 2-propynyl ester.

31. A composition according to claim 16, which composition contains a compound of the formula Ia

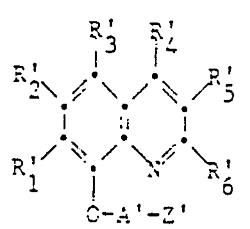

(Ia)

wherein

$R_1'$, $R_2'$, and $R_3'$ are each independently hydrogen, halogen, $C_1$-$C_3$-alkyl, $C_1$-$C_3$-alkoxy, nitro or cyano,

$R_4'$, $R_5'$, and $R_6'$ are each independently hydrogen, halogen or $C_1$-$C_3$-alkyl,

A' is any one of the groups $-CH_2-$, $-CH_2-CH_2-$ or $-CH(CH_3)-$, and

Z' is cyano or one of the groups

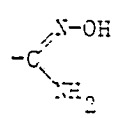 or 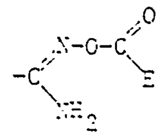

wherein E is —$R_7$, —$OR_8$, —$SR_9$ or —$NR_{10}R_{11}$, where $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen ; $R_8$, $R_9$ and $R_{10}$ are each independently $C_1$-$C_8$-alkyl which is unsubstituted or substituted by halogen, or they are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or are each benzyl which is unsubstituted or substituted by halogen or nitro, $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S ; or an acid addition salt or a metal complex thereof, with the proviso that Z' is not cyano or the group

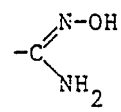

when simultaneously $R_1'$, $R_2'$, $R_4'$, $R_5'$, and $R_6'$ are hydrogen, $R_3'$ is hydrogen or chlorine, and A' is —$CH_2$— or —$CH(CH_3)$—.

32. A process for the preparation of a compound of the formula Ia

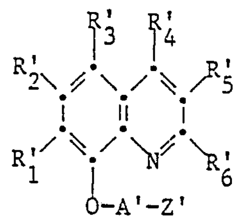

(Ia)

wherein $R_1'$, $R_2'$, and $R_3'$ are each independently hydrogen, halogen, $C_1$-$C_3$-alkyl, $C_1$-$C_3$-alkoxy, nitro or cyano,

$R_4'$, $R_5'$, and $R_6'$ are each independently hydrogen, halogen or $C_1$-$C_3$-alkyl,

A' is any one of the groups —$CH_2$—, —$CH_2$—$CH_2$ or —$CH(CH_3)$—, and

Z' is cyano, or amide oxime which may be acylated at the oxygen atom,

or of an acid addition salt or a metal complex thereof, with the proviso that Z' is not cyano or amide oxime when simultaneously $R_1'$, $R_2'$, $R_4'$, $R_5'$, and $R_6'$ are hydrogen, $R_3$ is hydrogen or chlorine, and A' is —$CH_2$— or —$CH(CH_3)$—,

which process is characterised in that :

a) a compound of the formula Ia in which $R_1'$, $R_2'$, $R_3'$, $R_4'$, $R_5'$ and $R_6'$ are as defined for the formula Ia, A' is the group —$CH_2$—$CH_2$—, and Z' is cyano, is prepared by reacting a compound of the formula II

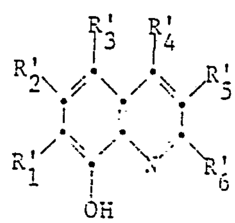

(II)

wherein $R_1'$, $R_2'$, $R_3'$, $R_4'$, $R_5'$, and $R_6'$ are as defined above, with a compound of the formula III

$$CH_2{=}CH{—}CN \quad (III) ;$$

or

b) a compound of the formula Ia in which $R_1'$, $R_2'$, $R_3'$, $R_4'$, $R_5'$ and $R_6'$ are as defined for the formula Ia, A' is either the group —$CH_2$— or —$CH(CH_3)$—, and Z' is cyano, is prepared by reacting a

compound of the formula II

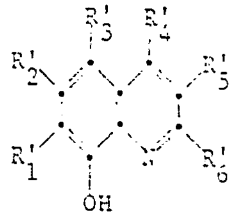

(II)

wherein $R_1'$, $R_2'$, $R_3'$, $R_4'$, $R_5'$ and $R_6'$ are as defined for the formula II

i) with a compound of the formula IV

$$Hal—A'—CN \quad (IV),$$

wherein Hal is a halogen atom, and A' is as defined above, or

ii) with a compound of the formule V

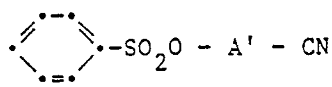

(V)

wherein A' is as defined above, or

iii) with a compound of the formula VI

$$Hal—A'—COOR_{12} \quad (VI),$$

wherein Hal is a halogen atom, and $R_{12}$ is an alkyl group having 1 to 6 carbon atoms, and A' is as defined above ; and converting the resultant ester of the formula VII

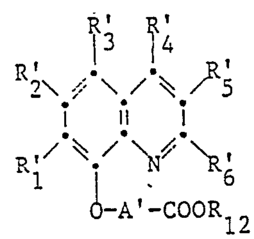

(VII)

wherein $R_1'$, $R_2'$, $R_3'$, $R_4'$, $R_5'$, $R_6'$, A' and $R_{12}$ are as defined above, with ammonia into the corresponding amide of the formula VIII

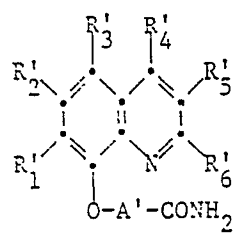

(VIII)

wherein $R_1'$, $R_2'$, $R_3'$, $R_4'$, $R_5'$, $R_6'$ and A' are as defined above, and subsequently dehydrating the product obtained ; and/or

c) a compound of the formula Ia in which $R_1'$, $R_2'$, $R_3'$, $R_4'$, $R_5'$, $R_6'$ and A' are as defined for the formula Ia, and Z' is amide oxime which may be acylated at the oxygen atom, is prepared by reacting a compound of the formula Ia wherein $R_1'$, $R_2'$, $R_3'$, $R_4'$, $R_5'$, $R_6'$ and A' are as defined for the formula Ia, and Z' is cyano, with hydroxylamine or with an acide salt of hydroxylamine ; and/or

d) a compound of the formula Ia in which $R_1'$, $R_2'$, $R_3'$, $R_4'$, $R_5'$, $R_6'$ and A' are as defined for the formula Ia, and Z' is acylated amide oxime, is prepared by acylating a compound of the formula Ia wherein $R_1'$, $R_2'$, $R_3'$, $R_4'$, $R_5'$, $R_6'$ and A' are as defined for the formula Ia, and Z' is amide oxime.

33. A process according to claim 32, characterised in that a compound of the formula Ia in which $R_1'$, $R_2'$, $R_3'$, $R_4'$, $R_5'$, $R_6'$ and A' are as defined for the formula Ia, and Z' is acylated amide oxime of the formula

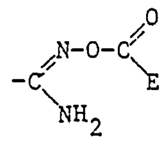

wherein E is $—R_7$, $—OR_8$, $—SR_9$ or $—NR_{10}R_{11}$ in which $R_7$ is $C_1$-$C_7$-alkyl which is unsubstituted or substituted by halogen or $C_1$-$C_4$-alkoxy, or $R_7$ is $C_3$-$C_6$-cycloalkyl, $C_2$-$C_4$-alkenyl, phenyl which is unsubstituted or substituted by halogen, nitro or $C_1$-$C_3$-alkyl, or it is benzyl which is unsubstituted or substituted by halogen, nitro or $C_1$-$C_3$-alkyl, or $R_7$ is a 5- or 6-membered heterocyclic ring which contains one or two hetero atoms from the group N, O and S, and which is unsubstituted or substituted by halogen, $R_8$, $R_9$ and $R_{10}$ are each independently $C_1$-$C_8$-alkyl which is unsubstituted or substituted by halogen, or theyf are each $C_2$-$C_4$-alkenyl, $C_3$-$C_6$-alkynyl, phenyl which is unsubstituted or substituted by halogen, $C_1$-$C_3$-alkyl, $C_1$-$C_3$-alkoxy, trifluoromethyl or nitro, or are each benzyl which is unsubstituted or substituted by halogen or nitro, and $R_{11}$ is hydrogen, $C_1$-$C_8$-alkyl or $C_1$-$C_3$-alkoxy, or $R_{10}$ and $R_{11}$, together with the nitrogen atom to which they are bound, form a 5- or 6-membered heterocyclic radical which may also contain a further hetero atom from the group N, O and S, is produced by reacting a compound of the formula Ia, wherein $R_1'$, $R_2'$, $R_3'$, $R_4'$, $R_5'$, $R_6'$ and A' are as defined for the formula Ia, and Z' is amide oxime, with a compound of the formula IX

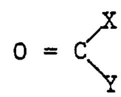 (IX)

in which X is a halogen atom, and Y is $—R_7$, $—OR_8$, $—SR_9$ or $—NR_{10}R_{11}$, wherein $R_7$, $R_8$, $R_9$, $R_{10}$ and $R_{11}$ are as defined above, or X and Y together are the imino group $=N—R_{10}$.

34. A method of selectively controlling weeds in crops of cultivated plants, which method comprises treating the cultivated plants or parts of the cultivated plants, or cultivated areas for cultivated plants, with a herbicide, and a compound of the formula I

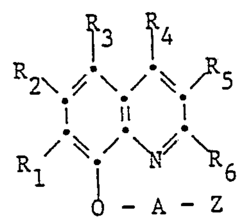 (I)

wherein $R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is a group $—CH_2—$, $—CH_2—CH_2—$ or $—CH(CH_3)—$, and

Z is cyano, or amide oxime which may be acylated at the oxygen atom,

or an acid addition salt or a metal complex thereof, or a composition containing such a compound.

35. A composition for selectively controlling weeds in crops of cultivated plants, which composition contains a compound of the formula I

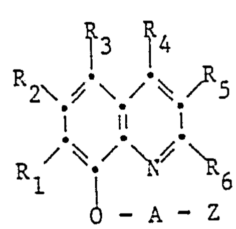 (I)

wherein $R_1$, $R_2$ and $R_3$ are each independently hydrogen, halogen, $C_1$-$C_3$-alkyl, $C_1$-$C_3$-alkoxy, nitro or cyano,

$R_4$, $R_5$ and $R_6$ are each independently hydrogen, halogen or $C_1$-$C_3$-alkyl,

A is any one of the groups $—CH_2—$, $—CH_2—CH_2—$ or $—CH(CH_3)—$, and

Z is cyano, or amide oxime which may be acylated at the oxygen atom,

or an acid addition salt or a metal complex thereof, and a herbicide.

**Revendications** (pour les Etats contractants : BE, CH, DE, FR, IT, LI, NL, SE)

1. Procédé pour protéger des végétaux cultivés contre les effets nocifs de produits agrochimiques agressifs, caractérisé en ce que l'on traite les végétaux cultivés, des parties de ces végétaux ou les sols prévus pour la culture de ces végétaux, par un composé de formule I :

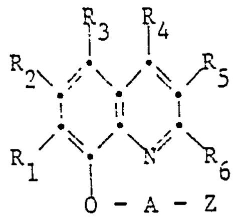

(I)

dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, ou un produit contenant l'un des composés.

2. Procédé selon la revendication 1, caractérisé en ce que l'on utilise un composé de formule I dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$ ;

A représente l'un des groupes $-CH_2-$, $-CH_2-CH_2-$ ou $-CH(CH_3)-$, et

Z représente un groupe cyano ou l'un des groupes

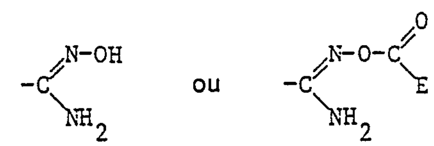

dans lesquels

E représente $-R_7$, $-OR_8$, $-SR_9$ ou $-NR_{10}R_{11}$, et

$R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou benzyle non substitué ou substitué par des halogènes ou des groupes nitro,

$R_{11}$ représente l'hydrogène, un groupe alkyle en $C_1$-$C_8$ ou alcoxy en $C_1$-$C_3$, ou bien

$R_{10}$ et $R_{11}$ forment ensemble et avec l'atome d'azote auquel ils sont reliés un hétérocycle à 5 ou 6 chaînons qui peut encore contenir un autre hétéroatome du groupe formé par N, O et S, y compris leurs sels formés par addition avec des acides et complexes métalliques, ou un produit contenant l'un de ces composés.

3. Procédé selon la revendication 2, caractérisé en ce que l'on utilise un composé de formule I dans laquelle

$R_1$ représente l'hydrogène, le chlore, le brome, l'iode ou un groupe nitro,

$R_2$ représente l'hydrogène,

$R_3$ représente l'hydrogène, le fluor, le chlore, le brome, l'iode, un groupe alkyle en $C_1$-$C_3$ ou nitro,

$R_4$ représente l'hydrogène, le brome ou un groupe méthyle,

$R_5$ représente l'hydrogène,

$R_6$ représente l'hydrogène ou un groupe méthyle,

A représente $-CH_2-$, $-CH_2-CH_2-$ ou $-CH(CH_3)-$, et

Z représente un groupe cyano,

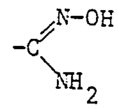 ou 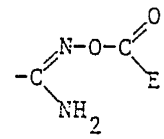

dans lesquels

E représente $-R_7$, $-OR_8$, $-SR_9$ ou $-NR_{10}R_{11}$, et

$R_7$ représente un groupe alkyle en $C_1$-$C_7$, alkyle en $C_1$-$C_3$ substitué par 1 à 3 atomes de chlore ou de brome, (alcoxy en $C_1$-$C_4$)-méthyle, cycloalkyle en $C_3$-$C_6$, alcényle en $C_2$-$C_3$, phényle non substitué ou portant 1 ou 2 substituants du groupe formé par le chlore, les groupes nitro et méthyle, benzyle non substitué ou monosubstitué par le chlore ou un groupe nitro, un cycle thiophène, furanne, tétrahydrofuranne ou pyrimidine non substitué ou mono- ou di-substitué par le chlore ou le brome,

$R_8$ représente un groupe alkyle en $C_1$-$C_4$, éthyle monosubstitué par le chlore ou le brome, alcényle en $C_2$-$C_3$, propynyle, phényle non substitué ou monosubstitué par un groupe nitro ou benzyle non substitué ou monosubstitué par un groupe nitro,

$R_9$ représente un groupe alkyle en $C_1$-$C_7$,

$R_{10}$ représente un groupe alkyle en $C_1$-$C_4$, chloréthyle, phényle non substitué ou portant 1 ou 2 substituants choisis parmi le chlore, les groupes méthoxy et trifluorométhyle, et

$R_{11}$ représente l'hydrogène, un groupe méthyle ou méthoxy, ou bien

$R_{10}$ et $R_{11}$ forment ensemble et avec l'atome d'azote auquel ils sont reliés un cycle pipéridine ou morpholine, ou un produit contenant l'un de ces composés.

4. Procédé selon la revendication 3, caractérisé en ce que l'on utilise un composé de formule I dans laquelle

$R_1$ représente l'hydrogène, le chlore, le brome ou l'iode,

$R_2$ représente l'hydrogène, $R_3$ représente l'hydrogène, le chlore ou un groupe nitro, $R_4$ et $R_5$ représentent l'hydrogène, $R_6$ représente l'hydrogène ou un groupe méthyle,

A représente $-CH_2-$, $-CH_2-CH_2-$ ou $-CH(CH_3)-$ et Z représente un groupe cyano,

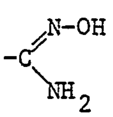 ou 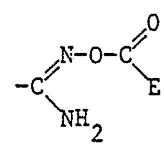

dans lesquels

E représente $-R_7$, $-OR_8$, $-SR_9$ ou $-NR_{10}R_{11}$, et

$R_7$ représente un groupe méthyle, éthyle, n-propyle, isopropyle, n-butyle, tert.-butyle, isobutyle, chlorométhyle, bromométhyle, 2-chloréthyle, 3-chloro-n-propyle, 1,2-dichloréthyle, méthoxyméthyle, n-propoxyméthyle, sec.-butoxyméthyle, cyclopropyle, vinyle, 1-propényle, isopropényle, phényle, 2-chlorophényle, 4-chlorophényle, benzyle, 2-thiényle, 2-furyle, 5-bromo-2-buryle, 2-tétrahydrofuryle ou 2,4-dichloropyrimidine-5-yle,

$R_8$ représente un groupe méthyle, éthyle, n-propyle, n-butyle, 2-brométhyle, allyle, phényle ou benzyle,

$R_9$ représente un groupe éthyle, isopropyle ou n-pentyle,

$R_{10}$ représente un groupe méthyle, éthyle, isopropyle, n-butyle, phényle, 3-trifluorométhylphényle, 4-chlorophényle, 2,5-dichlorophényle, et

$R_{11}$ représente l'hydrogène ou un groupe méthoxy, ou un produit contenant l'un de ces composés.

5. Procédé selon la revendication 4, caractérisé en ce que l'on utilise un composé de formule I dans laquelle

$R_1$ représente l'hydrogène, le chlore, le brome ou l'iode,

$R_2$ représente l'hydrogène, $R_3$ représente l'hydrogène ou le chlore, $R_4$ et $R_5$ représentent l'hydrogène, $R_6$ représente l'hydrogène ou un groupe méthyle, A représente $-CH_2-$ et Z représente un groupe cyano,

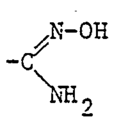 ou 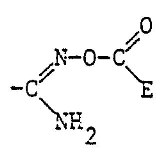

et E représente $-R_7$, $-OR_8$ ou $-NR_{10}R_{11}$, et

$R_7$ représente un groupe chlorométhyle, $R_8$ un groupe méthyle, $R_{10}$ un groupe isopropyle et $R_{11}$ l'hydrogène, ou un produit contenant l'un de ces composés.

6. Procédé selon la revendication 5, caractérisé en ce que l'on utilise la 5-chloro-8-(cyanométhoxy)-quinoléine ou un produit contenant ce composé.

7. Procédé selon la revendication 5, caractérisé en ce que l'on utilise la 5-chloro-7-iodo-8-(cyanométhoxy)-quinoléine ou un produit contenant ce composé.

8. Procédé selon la revendication 5, caractérisé en ce que l'on utilise l'O-(méthoxycarbonyl)-2-(8-quinoléinoxy)-acétamidoxime ou un produit contenant ce composé.

9. Procédé selon la revendication 1, pour la protection des végétaux cultivés contre les effets nocifs des produits phytosanitaires.

10. Procédé selon la revendication 9, pour la protection des végétaux cultivés contre les effets nocifs des herbicides.

11. Procédé selon la revendication 10, pour la protection des végétaux cultivés contre les effets nocifs des esters pyridyloxyphénoxypropioniques substitués.

12. Procédé selon la revendication 11, pour la protection des végétaux cultivés contre les effets nocifs du 2-[4-(3,5-dichloropyridyl-2-oxy)-phénoxy]-propionate de 2-propynyle.

13. Procédé selon la revendication 10, pour la protection des végétaux cultivés contre les effets nocifs des phénylurées.

14. Procédé selon la revendication 10, pour la protection des végétaux cultivés contre les effets nocifs des sulfonylurées.

15. Procédé selon la revendication 1, pour la protection du riz, du maïs, du blé, du seigle, de l'orge, de l'avoine, du coton, des betteraves à sucre, de la canne à sucre et du soja.

16. Produit pour la protection des végétaux cultivés contre les effets nocifs des produits agrochimiques agressifs, caractérisé en ce qu'il contient un composé de formule I :

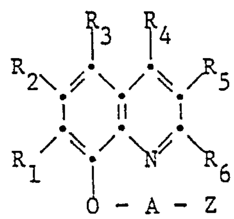

(I)

dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $—CH_2—$, $—CH_2—CH_2—$ ou $—CH(CH_3)—$ et

Z représente un groupe cyno ou amidoxime qui peut être acylé sur l'atome d'oxygène, y compris leurs sels formés par addition avec des acides et complexes métalliques.

17. Produit selon la revendication 16, caractérisé en ce qu'il contient un composé de formule I dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $—CH_2—$, $—CH_2—CH_2—$ ou $—CH(CH_3)—$, et

Z représente un groupe cyano ou l'un des groupes

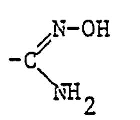

ou

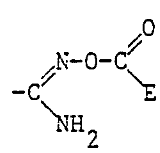

dans lesquels

E représente $—R_7$, $—OR_8$, $—SR_9$ ou $—NR_{10}R_{11}$, et

$R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, alkyle en $C_1$-$C_3$, alcoxy en $C_1$-$C_3$, trifluorométhyle ou nitro, ou benzyle non substitué ou substitué par des halogènes ou des groupes nitro,

$R_{11}$ représente l'hydrogène, un groupe alkyle en $C_1$-$C_8$ ou alcoxy en $C_1$-$C_3$, ou bien

$R_{10}$ et $R_{11}$ forment ensemble et avec l'atome d'azote auquel ils sont reliés, un hétérocycle à 5 ou 6 chaînons qui peut encore contenir un autre hétéroatome du groupe formé par N, O et S, y compris ses sels formés par addition avec des acides et complexes métalliques.

18. Produit selon la revendication 17, caractérisé en ce qu'il contient un composé de formule I dans laquelle

$R_1$ représente l'hydrogène, le chlore, le brome, l'iode ou un groupe nitro, $R_2$ représente l'hydrogène, $R_3$ représente l'hydrogène, le fluor, le chlore, le brome, l'iode, un groupe alkyle en $C_1$-$C_3$ ou nitro, $R_4$ représente l'hydrogène, le brome ou un groupe méthyle, $R_5$ représente l'hydrogène, $R_6$ représente l'hydrogène ou un groupe méthyle, A représente $—CH_2—$, $—CH_2—CH_2—$ ou $—CH(CH_3)—$ et Z représente un groupe cyano,

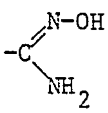 ou 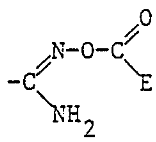 .

dans lesquels

E représente $—R_7$, $—OR_8$, $—SR_9$ ou $—NR_{10}R_{11}$, et

$R_7$ représente un groupe alkyle en $C_1$-$C_7$, alkyle en $C_1$-$C_3$ substitué par 1 à 3 atomes de chlore ou de brome, (alcoxy en $C_1$-$C_4$)-méthyle, cycloalkyle en $C_3$-$C_6$, alcényle en $C_2$-$C_3$, phényle non substitué ou portant 1 ou 2 substituants du groupe formé par le chlore, les groupes nitro et méthyle, benzyle non substitué ou monosubstitué par le chlore ou un groupe nitro, un cycle thiophène, furanne, tétrahydrofuranne ou pyrimidine non substitué ou mono- ou di-substitué par le chlore ou le brome,

$R_8$ représente un groupe alkyle en $C_1$-$C_4$, éthyle monosubstitué par le chlore ou le brome, alcényle en $C_2$-$C_3$, propynyle, phényle non substitué ou monosubstitué par un groupe nitro ou benzyle non substitué ou monosubstitué par un groupe nitro,

$R_9$ représente un groupe alkyle en $C_1$-$C_7$,

$R_{10}$ représente un groupe alkyle en $C_1$-$C_4$, chloréthyle, phényle non substitué ou portant 1 ou 2 substituants formés par le chlore, les groupes méthoxy et trifluorométhyle, et

$R_{11}$ représente l'hydrogène, un groupe méthyle ou méthoxy, ou bien

$R_{10}$ et $R_{11}$ forment ensemble et avec l'atome d'azote auquel ils sont reliés, un cycle pipéridine ou morpholine.

19. Produit selon la revendication 18, caractérisé en ce qu'il contient un composé de formule I dans laquelle

$R_1$ représente l'hydrogène, le chlore, le brome ou l'iode, $R_2$ représente l'hydrogène, $R_3$ représente l'hydrogène, le chlore ou un groupe nitro, $R_4$ et $R_5$ représentent l'hydrogène, $R_6$ représente l'hydrogène ou un groupe méthyle,

A représente $—CH_2—$, $—CH_2—CH_2—$ ou $—CH(CH_3)—$ et Z représente un groupe cyano,

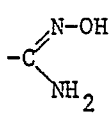 ou 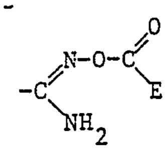

dans lesquels

E représente $—R_7$, $—OR_8$, $—SR_9$ ou $—NR_{10}R_{11}$, et $R_7$ représente un groupe méthyle, éthyle, n-propyle, isopropyle, n-butyle, tert.-butyle, isobutyle, chlorométhyle, bromométhyle, 2-chloréthyle, 3-chloro-n-propyle, 1,2-dichloréthyle, méthoxyméthyle, n-propoxyméthyle, sec.-butoxyméthyle, cyclopropyle, vinyle, 1-propényle, isopropényle, phényle, 2-chlorophényle, 4-chlorophényle, benzyle, 2-thiényle, 2-furyle, 5-bromo-2-furyle, 2-tétrahydrofuryle ou 2,4-dichloropyrimidine-5-yl,

$R_8$ représente un groupe méthyle, éthyle, n-propyle, n-butyle, 2-brométhyle, allyle, phényle ou benzyle,

$R_9$ représente un groupe méthyle, isopropyle ou n-pentyle,

$R_{10}$ représente un groupe méthyle, éthyle, isopropyle, n-butyle, phényle, 3-trifluorométhylphényle, 4-chlorophényle, 2,5-dichlorophényle, et

$R_{11}$ représente l'hydrogène ou un groupe méthoxy.

20. Produit selon la revendication 19, caractérisé en ce qu'il contient un composé de formule I dans laquelle $R_1$ représente l'hydrogène, le chlore, le brome ou l'iode, $R_2$ représente l'hydrogène, $R_3$ représente l'hydrogène ou le chlore, $R_4$ et $R_5$ représentent l'hydrogène, $R_6$ représente l'hydrogène ou un groupe méthyle, A représente $—CH_2—$ et Z représente un groupe cyano,

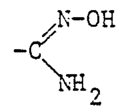 ou 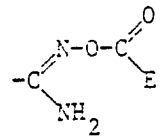

dans lesquels E représente —$R_7$, —$OR_8$ ou —$NR_{10}R_{11}$, et $R_7$ représente un groupe chlorométhyle $R_8$ représente un groupe méthyle, $R_{10}$ un groupe isopropyle et $R_{11}$ l'hydrogène.

21. Produit selon la revendication 20, caractérisé en ce qu'il contient de la 5-chloro-8-(cyanométhoxy)-quinoléine.

22. Produit selon la revendication 20, caractérisé en ce qu'il contient de la 5-chloro-7-iodo-8-(cyanométhoxy)-quinoléine.

23. Produit selon la revendication 20, caractérisé en ce qu'il contient de l'O-(méthoxycarbonyl)-2-(8-quinoléinoxy)-acétamidoxime.

24. Produit selon la revendication 16, caractérisé en ce qu'il contient un composé de formule I et un herbicide.

25. Produit selon la revendication 24, caractérisé en ce qu'il contient en tant qu'herbicide un composé de formule A :

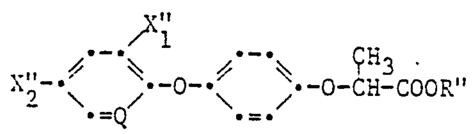 (A)

dans laquelle

$X_1''$ représente l'hydrogène ou un halogène,

$X_2''$ représente l'hydrogène, un halogène ou un groupe trifluorométhyle,

Q représente le fragment =N— ou =CH—,

R'' représente un groupe alkyle en $C_1$-$C_4$ non substitué ou substitué par un groupe alcoxy en $C_1$-$C_4$, un groupe alcényle en $C_3$-$C_4$, alcynyle en $C_3$-$C_4$ ou

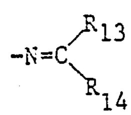

$R_{13}$ représente un groupe alkyle en $C_1$-$C_4$,

$R_{14}$ représente un groupe alkyle en $C_1$-$C_4$, ou bien

$R_{13}$ et $R_{14}$ forment ensemble un groupe alkylène en $C_4$-$C_5$.

26. Produit selon la revendication 24, caractérisé en ce qu'il contient en tant qu'herbicide un ester pyridyloxyphénoxypropionique.

27. Produit selon la revendication 24, caractérisé en ce qu'il contient en tant qu'herbicide le 2-[4-(3,5-dichloropyridyl-2-oxy)-phénoxy]-propionate de 2-propynyle.

28. Produit selon la revendication 24, caractérisé en ce qu'il contient en tant qu'herbicide une phénylurée.

29. Produit selon la revendication 24, caractérisé en ce qu'il contient en tant qu'herbicide une sulfonylurée.

30. Produit selon la revendication 24, caractérisé en ce qu'il contient en tant que composé de formule I, la 5-chloro-8-(cyanométhoxy)-quinoléine et en tant qu'herbicide le 2-[4-(3,5-dichloropyridyl-2-oxy)-phénoxy]-propionate de 2-propynyle.

31. Produit selon la revendication 16, caractérisé en ce qu'il contient un composé de formule Ia :

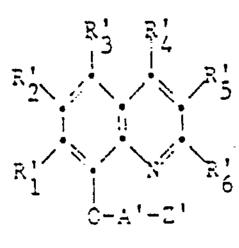 (Ia)

dans laquelle

$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano,

$R_4'$, $R_5'$ et $R_6'$ représentent chacun, indépendamment les uns des autres, l'hydrogène, un halogène

ou un groupe alkyle en $C_1$-$C_3$,

A' représente l'un des groupes —$CH_2$—, —$CH_2$—$CH_2$— ou —$CH(CH_3)$—, et

Z' représente un groupe cyano ou l'un des groupes

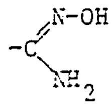

ou

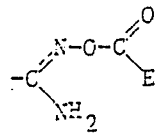

dans lesquels

E représente —$R_7$, —$OR_8$, —$SR_9$ ou —$NR_{10}R_{11}$, et

$R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S, et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou benzyle non substitué ou substitué par des halogènes ou des groupes nitro,

$R_{11}$ représente l'hydrogène, un groupe alkyle en $C_1$-$C_8$ ou alcoxy en $C_1$-$C_3$, ou bien

$R_{10}$ et $R_{11}$ forment ensemble et avec l'atome d'azote auquel ils sont reliés, un hétérocycle à 5 ou 6 chaînons qui peut contenir un autre hétéroatome du groupe formé par N, O et S, y compris leurs sels formés par addition avec des acides et complexes métalliques, avec la restriction que Z' ne peut représenter un groupe cyano ou le groupe

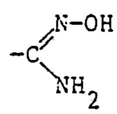

lorsque, en même temps, $R_1'$, $R_2'$, $R_4'$, $R_5'$ et $R_6'$ représentent l'hydrogène, $R'_3$ représente l'hydrogène ou le chlore et A' représente —$CH_2$— ou —$CH(CH_3)$—.

32. Composés de formule Ia

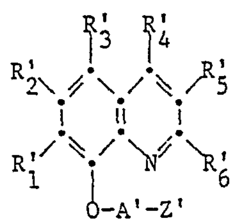

(Ia)

dans laquelle

$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano, $R_4'$, $R_5'$ et $R_6'$ représentent chacun, indépendamment les uns des autres, l'hydrogène, un halogène ou un groupe alkyle en $C_1$-$C_3$,

A' représente l'un des groupes —$CH_2$—, —$CH_2$—$CH_2$— ou —$CH(CH_3)$—, et

Z' représente un groupe cyano ou amidoxime qui peut être acylé sur l'atome d'oxygène, y compris leurs sels formés par addition avec des acides et complexes métalliques, avec la restriction que Z' ne peut représenter un groupe cyano ou amidoxime lorsque, en même temps, $R_1'$, $R_2'$, $R_4'$, $R_5'$ et $R_6'$ représentent l'hydrogène, $R_3'$ représente l'hydrogène ou le chlore et A' le groupe —$CH_2$— ou —$CH(CH_3)$—.

33. Composés selon la revendication 32, caractérisés en ce que :

$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano,

$R_4'$, $R_5'$ et $R_6'$ représentent chacun, indépendamment les uns des autres, l'hydrogène ou un groupe alkyle en $C_1$-$C_3$,

A' représente l'un des groupes —$CH_2$—, —$CH_2$—$CH_2$— ou —$CH(CH_3)$—, et

Z' représente un groupe cyano ou l'un des groupes

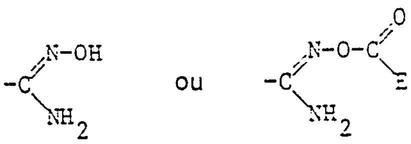

dans lesquels

E représente $-R_7$, $-OR_8$, $-SR_9$ ou $-NR_{10}R_{11}$, et

$R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué poar des halogènes, alkyle en $C_1$-$C_3$, alcoxy en $C_1$-$C_3$, trifluorométhyle ou nitro, ou un groupe benzyle non substitué ou substitué par des halogènes ou des groupes nitro,

$R_{11}$ représente l'hydrogène, un groupe alkyle en $C_1$-$C_8$ ou alcoxy en $C_1$-$C_3$ ou bien

$R_{10}$ et $R_{11}$ forment ensemble et avec l'atome d'azote auquel ils sont reliés un hétérocycle à 5 ou 6 chaînons qui peut encore contenir un autre hétéroatome du groupe formé par N, O et S,

y compris leurs sels formés par addition avec des acides et complexes métalliques, avec la restriction que Z' ne peut représenter un groupe cyano ou amidoxime lorsque en même temps, $R_1'$, $R_2'$, $R_4'$, $R_5'$ et $R_6'$ représentent l'hydrogène, $R_3'$ représente l'hydrogène ou le chlore et A' un groupe $-CH_2-$ ou $-CH(CH_3)-$.

34. Composés selon la revendication 33, caractérisés en ce que $R_1'$ représente l'hydrogène, le chlore, le brome, l'iode ou un groupe nitro, $R_2'$ représente l'hydrogène, $R_3'$ l'hydrogène, le fluor, le chlore, le brome, l'iode, un groupe alkyle en $C_1$-$C_3$ ou nitro, $R_4'$ l'hydrogène, le brome ou un groupe méthyle, $R_5'$ l'hydrogène, $R_6'$ l'hydrogène ou un groupe méthyle, A' un groupe $-CH_2-$, $-CH_2-CH_2-$ ou $-CH(CH_3)-$ et Z' un groupe cyano,

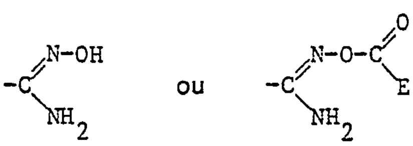

dans lesquels

E représente $-R_7$, $-OR_8$, $-SR_9$ ou $-NR_{10}R_{11}$, et

$R_7$ représente un groupe alkyle en $C_1$-$C_7$, alkyle en $C_1$-$C_3$ substitué par 1 à 3 atomes de chlore ou de brome, (alcoxy en $C_1$-$C_4$)-méthyle, cycloalkyle en $C_3$-$C_6$, alcényle en $C_2$-$C_3$, phényle non substitué ou portant 1 ou 2 substituants du groupe formé par le chlore, les groupes nitro et méthyle, benzyle non substitué ou monosubstitué par le chlore ou un groupe nitro, un cycle thiophène, furanne, tétrahydrofuranne ou pyrimidine non substitué ou mono- ou di-substitué par le chlore ou le brome,

$R_8$ représente un groupe alkyle en $C_1$-$C_4$, éthyle monosubstitué par le chlore ou le brome, alcényle en $C_2$-$C_3$, propynyle, phényle non substitué ou monosubstitué par un groupe nitro, ou benzyle non substitué ou monosubstitué par un groupe nitro,

$R_9$ représente un groupe alkyle en $C_1$-$C_7$,

$R_{10}$ représente un groupe alkyle en $C_1$-$C_4$, chloréthyle, phényle non substitué ou portant 1 ou 2 substituants du groupe formé par le chlore, les groupes méthoxy et trifluorométhyle, et

$R_{11}$ représente l'hydrogène, un groupe méthyle ou méthoxy, ou bien $R_{10}$ et $R_{11}$ forment ensemble et avec l'atome d'azote auquel ils sont reliés, un cycle pipéridine ou morpholine, avec la restriction que Z' ne peut représenter un groupe cyano ou amidoxime lorsque, en même temps, $R_1'$, $R_2'$, $R_4'$, $R_5'$ et $R_6'$ représentent l'hydrogène, $R_3'$ l'hydrogène ou le chlore et A' un groupe $-CH_2-$ ou $-CH(CH_3)-$.

35. Composés selon la revendication 34, caractérisés en ce que

$R_1'$ représente l'hydrogène, le chlore, le brome ou l'iode,

$R_2'$ représente l'hydrogène, $R_3'$ l'hydrogène, le chlore ou un groupe nitro, $R_4'$ et $R_5'$ l'hydrogène, $R_6'$ l'hydrogène ou un groupe méthyle, A' un groupe $-CH_2-$, $-CH_2-CH_2-$ ou $-CH(CH_3)-$ et Z' un groupe cyano,

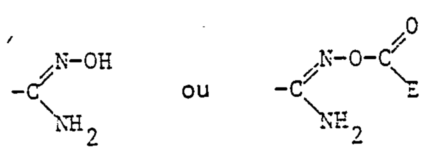

dans lesquels

E représente $-R_7$, $-OR_8$, $-SR_9$ ou $-NR_{10}R_{11}$, et

$R_7$ représente un groupe méthyle, éthyle, n-propyle, isopropyle, n-butyle, tert.-butyle, isobutyle, chlorométhyle, bromométhyle, 2-chloréthyle, 3-chloro-n-propyle, 1,2-dichloréthyle, méthoxyméthyle, n-propoxyméthyle, sec.-butoxyméthyle, cyclopropyle, vinyle, 1-propényle, isopropényle, phényle, 2-chlorophényle, 4-chlorophényle, benzyle, 2-thiényle, 2-furyle, 5-bromo-2-furyle, 2-tétrahydrofuryle ou 2,4-dichloropyrimidine-5-yle,

$R_8$ représente un groupe méthyle, éthyle, n-propyle, n-butyle, 2-brométhyle, allyle, phényle ou benzyle,

$R_9$ représente un groupe éthyle, isopropyle ou n-pentyle,

$R_{10}$ représente un groupe méthyle, éthyle, isopropyle, n-butyle, phényle, 3-trifluorométhylphényle, 4-chlorophényle, 2,5-dichlorophényle, et

$R_{11}$ représente l'hydrogène ou un groupe méthoxy, avec la restriction que Z' ne peut représenter un groupe cyano ou amidoxime lorsque $R_1'$, $R_2'$, $R_4'$, $R_5'$ et $R_6'$ représentent l'hydrogène, $R_3'$ l'hydrogène ou le chlore et A' un groupe $-CH_2-$ ou $-CH(CH_3)-$.

36. Composés selon la revendication 35, caractérisés en ce que $R_1'$ représente l'hydrogène, le chlore, le brome ou l'iode, $R_2'$ l'hydrogène, $R_3'$ l'hydrogène ou le chlore, $R_4'$ et $R_5'$ l'hydrogène, $R_6'$ l'hydrogène ou un groupe méthyle, A' représente $-CH_2-$ et Z' un groupe cyano

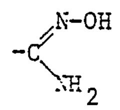

ou

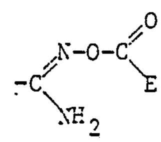

dans lesquels E représente $-R_7$, $-OR_8$ ou $-NR_{10}R_{11}$ et $R_7$ représente un groupe chlorométhyle, $R_8$ un groupe méthyle, $R_{10}$ un groupe isopropyle et $R_{11}$ l'hydrogène, avec la restriction que Z' ne peut représenter un groupe cyano ou amidoxime lorsque, en même temps, $R_1'$, $R_2'$, $R_4'$, $R_5'$ et $R_6'$ représentent l'hydrogène, $R_3'$ l'hydrogène ou le chlore et A' un groupe $-CH_2-$ ou $-CH(CH_3)-$.

37. La 2-méthyl-8-(cyanométhoxy)-quinoléine.

38. La 2-(2-méthyl-8-quinoléinoxy)-acétamidoxime.

39. L'O-(isopropylaminocarbonyl)-2-(8-quinoléinoxy)-acétamidoxime.

40. L'O-(chlorométhylcarbonyl)-2-(8-quinoléinoxy)-acétamidoxime.

41. La 2-(5-chloro-7-bromo-8-quinoléinoxy)-acétamidoxime.

42. La 5-chloro-7-bromo-8-(cyanométhoxy)-quinoléine.

43. L'O-(méthoxycarbonyl)-2-(8-quinoléinoxy)-acétamidoxime.

44. La 2-(5-chloro-7-iodo-8-quinoléinoxy)-acétamidoxime.

45. L'O-(isopropylaminocarbonyl)-2-(5-chloro-7-bromo-8-quinoléinoxy)-acétamidoxime.

46. La 2-(2-méthyl-5,7-dichloro-8-quinoléinoxy)-acétamidoxime.

47. La 5,7-dichloro-8-(cyanométhoxy)-quinoléine.

48. L'O-(isopropylaminocarbonyl)-2-(5-chloro-7-iodo-8-quinoléinoxy)-acétamidoxime.

49. La 2-méthyl-5,7-dichloro-8-(cyanométhoxy)-quinoléine.

50. L'O-(isopropylaminocarbonyl)-2-(2-méthyl-5,7-dichloro-8-quinoléinoxy)-acétamidoxime.

51. La 5-chloro-7-iodo-8-(cyanométhoxy)-quinoléine.

52. Procédé de préparation des composés de formule Ia :

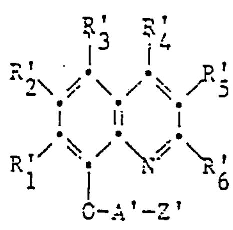

(Ia)

dans laquelle

$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano, $R_4'$, $R_5'$ et $R_6'$ représentent chacun, indépendamment les uns des autres, l'hydrogène, un halogène, ou un groupe alkyle en $C_1$-$C_3$,

A' représente l'un des groupes $-CH_2-$, $-CH_2-CH_2-$ ou $-CH(CH_3)-$, et

Z' représente un groupe cyano ou amidoxime qui peut être acylé sur l'atome d'oxygène,

y compris leurs sels formés par addition avec des acides et complexes métalliques, avec la restriction que Z' ne peut représenter un groupe cyano ou amidoxime lorsque, en même temps, $R_1'$, $R_2'$, $R_4'$, $R_5'$ et $R_6'$ représentent l'hydrogène, $R_3'$ l'hydrogène ou le chlore et A' un groupe $-CH_2-$ ou $-CH(CH_3)-$, caractérisé en ce que :

a) pour préparer les composés de formule Ia dans laquelle $R_1'$, $R_2'$, $R_3'$, $R_4'$, $R_5'$ et $R_6'$ ont les significations indiquées en référence à la formule Ia, A' représente le groupe $-CH_2-CH_2-$ et Z' un groupe cyano, on fait réagir un composé de formule II :

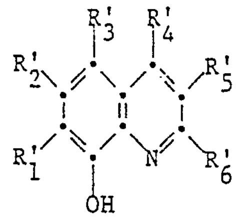

(II)

dans laquelle $R_1'$, $R_2'$, $R_3'$, $R_4'$, $R_5'$ et $R_6'$ ont les significations indiquées ci-dessus, avec un composé de formule III :

$$CH_2{=}CH{-}CN \qquad (III)$$

ou bien

b) pour préparer les composés de formule Ia dans laquelle $R_1'$, $R_2'$, $R_3'$, $R_4'$, $R_5'$ et $R_6'$ ont les significations indiquées en référence à la formule Ia, A' représente l'un des groupes $-CH_2-$ ou $-CH(CH_3)-$ et Z' un groupe cyano, on fait réagir un composé de formule II :

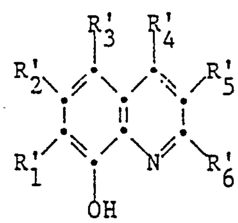

(II)

dans laquelle $R_1'$, $R_2'$, $R_3'$, $R_4'$, $R_5'$ et $R_6'$ ont les significations indiquées en référence à la formule II, avec

i) un composé de formule IV

$$Hal{-}A'{-}CN \qquad (IV)$$

dans laquelle Hal représente un atome d'halogène et A' a la signification indiquée ci-dessus, ou bien

ii) un composé de formule V :

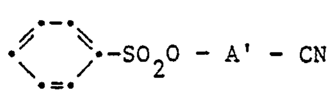

(V)

dans laquelle A' a la signification indiquée ci-dessus, ou bien

iii) un composé de formule VI :

$$Hal{-}A'{-}COOR_{12} \qquad (VI)$$

dans laquelle Hal représente un atome d'halogène et $R_{12}$ un groupe alkyle en $C_1$-$C_6$ et A' a la signification indiquée ci-dessus,

et on convertit les esters obtenus répondant à la formule VII :

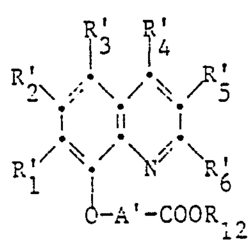

(VII)

dans laquelle $R_1'$, $R_2'$, $R_3'$, $R_4'$, $R_5'$, $R_6'$ et $R_{12}$ et A' ont les significations indiquées ci-dessus, par réaction avec l'ammoniac, en les amides correspondants de formule VIII :

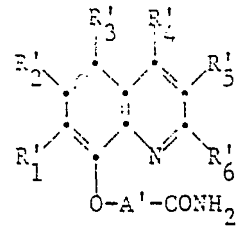

(VIII)

dans laquelle $R_1'$, $R_2'$, $R_3'$, $R_4'$, $R_5'$, $R_6'$ et A' ont les significations indiquées ci-dessus, et on fait suivre d'une déshydratation, et/ou

c) pour préparer les composés de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime qui peut acylé sur l'atome d'oxygène, on fait réagir un composé de formule Ia dans laquelle $R_1'$, $R_2'$, $R_3'$, $R_4'$, $R_5'$, $R_6'$ et A' ont les significations indiquées en référence à la formule Ia et Z' représente un groupe cyano, avec l'hydroxylamine ou un sel de l'hydroxylamine et d'un acide, et/ou

d) pour préparer les composés de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime acylé, on acyle un composé de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime.

53. Procédé selon la revendication 52, caractérisé en ce que, pour préparer les composés de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime acylé de formule

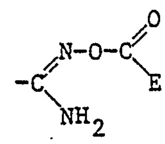

dans laquelle E représente $-R_7$, $-OR_8$, $-SR_9$ ou $-NR_{10}R_{11}$, et $R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons qui contient 1 ou 2 hétéroatomes du groupe formé par N, O et S, et est non substitué ou substitué par des halogènes, $R_8$, $R_9$ et $R_{10}$ représentent chacun, indépendamment les uns des autres, un groupe alkyle en $C_1$-$C_8$ non substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou un groupe benzyle non substitué par des halogènes ou des groupes nitro, $R_{11}$ représente l'hydrogène, un groupe alkyle en $C_1$-$C_8$ ou alcoxy en $C_1$-$C_3$, ou bien $R_{10}$ et $R_{11}$ forment ensemble et avec l'atome d'azote auquel ils sont reliés un hétérocycle à 5 ou 6 chaînons qui peut encore contenir un autre hétéroatome du groupe formé par N, O et S, on fait réagir un composé de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime, avec un composé de formule IX :

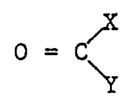

(IX)

dans laquelle X représente un atome d'halogène et Y représente $-R_7$, $-OR_8$, $-SR_9$ ou $-NR_{10}R_{11}$, et $R_7$, $R_8$, $R_9$, $R_{10}$ et $R_{11}$ ont les significations indiquées ci-dessus, ou bien X et Y forment ensemble le groupe imino $=N-R_{10}$.

54. Procédé pour combattre sélectivement les mauvaises herbes dans les cultures de végétaux utiles, caractérisé en ce que l'on traite les cultures, des parties des végétaux cultivés ou les terrains de culture par un herbicide et un composé de formule I :

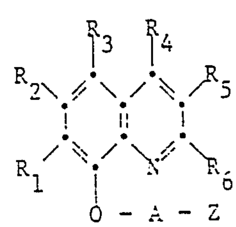

(I)

dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, ou par un produit contenant l'un de ces composés.

55. Produit pour combattre sélectivement les mauvaises herbes dans les cultures de végétaux utiles, caractérisé en ce qu'il contient un composé de formule I :

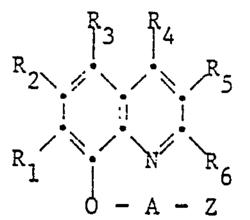

(I)

dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, et un herbicide.

**Revendications** (pour l'Etat contractant AT)

1. Procédé pour protéger les végétaux cultivés contre les effets nocifs de produits agrochimiques agressifs, caractérisé en ce que l'on traite les végétaux cultivés, des parties de ces végétaux ou les sols prévus pour la culture, par un composé de formule I :

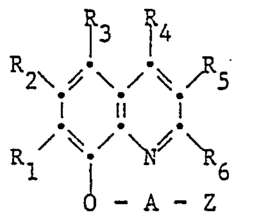

(I)

dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, ou par un produit contenant l'un de ces composés.

2. Procédé selon la revendication 1, caractérisé en ce que l'on utilise un composé de formule I dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou l'un des groupes

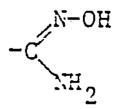

ou

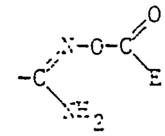

dans lesquels

E représente —$R_7$, —$OR_8$, —$SR_9$ ou —$NR_{10}R_{11}$, et $R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique contenant 5 à 6 chaînons et contenant 1 ou 2 hétéroatomes du groupe formé par N, O et S et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou un groupe benzyle non substitué ou substitué par des halogènes ou des groupes nitro,

$R_{11}$ représente l'hydrogène, un groupe alkyle en $C_1$-$C_8$ ou alcoxy en $C_1$-$C_3$, ou bien

$R_{10}$ et $R_{11}$ forment ensemble et avec l'atome d'azote auquel ils sont reliés un hétérocycle à 5 ou 6 chaînons qui peut encore contenir un autre hétéroatome du groupe formé par N, O et S, y compris leurs sels formés par addition avec des acides et complexes métalliques, ou un produit contenant l'un de ces composés.

3. Procédé selon la revendication 2, caractérisé en ce que l'on utilise un composé de formule I dans laquelle :

$R_1$ représente l'hydrogène, le brome, l'iode ou un groupe nitro,

$R_2$ représente l'hydrogène,

$R_3$ représente l'hydrogène, le fluor, le chlore, le brome, l'iode, un groupe alkyle en $C_1$-$C_3$ ou nitro,

$R_4$ représente l'hydrogène, le brome ou un groupe méthyle,

$R_5$ représente l'hydrogène,

$R_6$ représente l'hydrogène ou un groupe méthyle,

A représente —$CH_2$—, —$CH_2$—$CH_2$— ou —$CH(CH_3)$—, et

Z représente un groupe cyano,

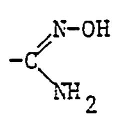

ou

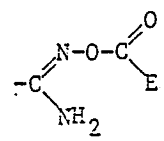

dans lesquels

E représente —$R_7$, —$OR_8$, —$SR_9$ ou —$NR_{10}R_{11}$, et

$R_7$ représente un groupe alkyle en $C_1$-$C_7$, alkyle en $C_1$-$C_3$ substitué par 1 à 3 atomes de chlore ou de brome, (alcoxy en $C_1$-$C_4$)-méthyle, cycloalkyle en $C_3$-$C_6$, alcényle en $C_2$-$C_3$, phényle non substitué ou portant 1 ou 2 substituants du groupe formé par le chlore, les groupes nitro et méthyle, benzyle non substitué ou monosubstitué par le chlore ou un groupe nitro, un cycle thiophène, furanne, tétrahydrofuranne ou pyrimidine non substitué ou mono- ou di-substitué par le chlore ou le brome,

$R_8$ représente un groupe alkyle en $C_1$-$C_4$, éthyle monosubstitué par le chlore ou le brome, alcényle en $C_2$-$C_3$, propynyle, phényle non substitué ou monosubstitué par un groupe nitro ou benzyle non substitué ou monosubstitué par un groupe nitro,

$R_9$ représente un groupe alkyle en $C_1$-$C_7$,

$R_{10}$ représente un groupe alkyle en $C_1$-$C_4$, chloréthyle, phényle non substitué ou portant 1 ou 2 substituants choisis parmi le chlore, les groupes méthoxy et trifluorométhyle, et

$R_{11}$ représente l'hydrogène, un groupe méthyle ou méthoxy, ou bien

$R_{10}$ et $R_{11}$ forment ensemble et avec l'atome d'azote auquel ils sont reliés un cycle pipéridine ou morpholine, ou un produit contenant l'un de ces composés.

4. Procédé selon la revendication 3, caractérisé en ce que l'on utilise un composé de formule I dans laquelle

$R_1$ représente l'hydrogène, le chlore, le brome ou l'iode,

$R_2$ représente l'hydrogène, $R_3$ représente l'hydrogène, le chlore ou un groupe nitro, $R_4$ et $R_5$ représentent l'hydrogène, $R_6$ représente l'hydrogène ou un groupe méthyle,

A représente —$CH_2$—, —$CH_2$—$CH_2$— ou —$CH(CH_3)$— et Z représente un groupe cyano,

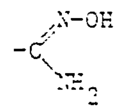

ou

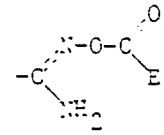

dans lesquels

E représente —$R_7$, —$OR_8$, —$SR_9$ ou —$NR_{10}R_{11}$, et

$R_7$ représente un groupe méthyle, éthyle, n-propyle, isopropyle, n-butyle, tert.-butyle, isobutyle, chlorométhyle, bromométhyle, 2-chloréthyle, 3-chloro-n-propyle, 1,2-dichloréthyle, méthoxyméthyle, n-propoxyméthyle, sec.-butoxyméthyle, cyclopropyle, vinyle, 1-propényle, isopropényle, phényle, 2-chlorophényle, 4-chlorophényle, benzyle, 2-thiényle, 2-furyle, 5-bromo-2-buryle, 2-tétrahydrofuryle ou 2,4-dichloropyrimidine-5-yle, $R_8$ représente un groupe méthyle, éthyle, n-propyle, n-butyle, 2-brométhyle, allyle, phényle ou benzyle, $R_9$ représente un groupe éthyle, isopropyle ou n-pentyle, $R_{10}$ représente un groupe méthyle, éthyle, isopropyle, n-butyle, phényle, 3-trifluorométhylphényle, 4-chlorophényle, 2,5-dichlorophényle, et $R_{11}$ représente l'hydrogène ou un groupe méthoxy, ou un produit contenant l'un de ces composés.

5. Procédé selon la revendication 4, caractérisé en ce que l'on utilise un composé de formule I dans laquelle $R_1$ représente l'hydrogène, le chlore, le brome ou l'iode, $R_2$ représente l'hydrogène, $R_3$ représente l'hydrogène ou le chlore, $R_4$ et $R_5$ représentent l'hydrogène, $R_6$ représente l'hydrogène ou un groupe méthyle, A représente —$CH_2$— et Z représente un groupe cyano,

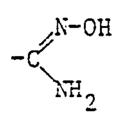

ou

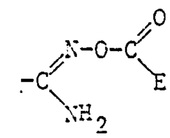

et

E représente —$R_7$, —$OR_8$ ou —$NR_{10}R_{11}$, et

$R_7$ représente un groupe chlorométhyle, $R_8$ un groupe méthyle, $R_{10}$ un groupe isopropyle et $R_{11}$ l'hydrogène, ou un produit contenant l'un de ces composés.

6. Procédé selon la revendication 5, caractérisé en ce que l'on utilise la 5-chloro-8-(cyanométhoxy)-quinoléine ou un produit contenant ce composé.

7. Procédé selon la revendication 5, caractérisé en ce que l'on utilise la 5-chloro-7-iodo-8-(cyanométhoxy)-quinoléine ou un produit contenant ce composé.

8. Procédé selon la revendication 5, caractérisé en ce que l'on utilise l'O-(méthoxycarbonyl)-2-(0-quinoléinoxy)-acétamidoxime ou un produit contenant ce composé.

9. Procédé selon la revendication 1, pour la protection des végétaux cultivés contre les effets nocifs des produits phytosanitaires.

10. Procédé selon la revendication 9, pour la protection des végétaux cultivés contre les effets nocifs des herbicides.

11. Procédé selon la revendication 10, pour la protection des végétaux cultivés contre les effets nocifs des esters pyridyloxyphénoxypropioniques substitués.

12. Procédé selon la revendication 11, pour la protection des végétaux cultivés contre les effets nocifs du 2-[4-(3,5-dichloropyridyl-2-oxy)-phénoxy]-propionate de 2-propynyle.

13. Procédé selon la revendication 10, pour la protection des végétaux cultivés contre les effets nocifs des phénylurées.

14. Procédé selon la revendication 10, pour la protection des végétaux cultivés contre les effets nocifs des sulfonylurées.

15. Procédé selon la revendication 1, pour la protection du riz, du maïs, du blé, du seigle, de l'orge, de l'avoine, du coton, des betteraves à sucre, de la canne à sucre et du soja.

16. Produit pour la protection des végétaux cultivés contre les effets nocifs des produits agrochimiques agressifs, caractérisé en ce qu'il contient un composé de formule I :

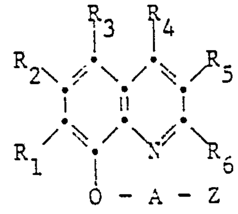

(I)

dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou

un groupe alkyle en $C_1$-$C_3$,

A représente l'un des groupes —$CH_2$—, —$CH_2$—$CH_2$— ou —$CH(CH_3)$— et

Z représente un groupe cyano ou amidoxime qui peut être acylé sur l'atome d'oxygène, y compris leurs sels formés par addition avec des acides et complexes métalliques.

17. Produit selon la revendication 16, caractérisé en ce qu'il contient un composé de formule I dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes —$CH_2$—, —$CH_2$—$CH_2$— ou —$CH(CH_3)$—, et

Z représente un groupe cyano ou l'un des groupes

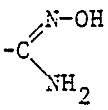 ou 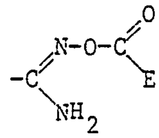

dans lesquels

E représente —$R_7$, —$OR_8$, —$SR_9$ ou —$NR_{10}R_{11}$, et

$R_7$ représente un groupe alkyle en $C_1$-$C_7$ non substitué ou substitué par des halogènes ou des groupes alcoxy en $C_1$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S et non substitué ou substitué par des halogènes,

$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, alkyle en $C_1$-$C_3$, alcoxy en $C_1$-$C_3$, trifluorométhyle ou nitro, ou benzyle non substitué ou substitué par des halogènes ou des groupes nitro,

$R_{11}$ représente l'hydrogène, un groupe alkyle en $C_1$-$C_8$ ou alcoxy en $C_1$-$C_3$, ou bien

$R_{10}$ et $R_{11}$ forment ensemble et avec l'atome d'azote auquel ils sont reliés, un hétérocycle à 5 ou 6 chaînons qui peut encore contenir un autre hétéroatome du groupe formé par N, O et S, y compris ses sels formés par addition avec des acides et complexes métalliques.

18. Produit selon la revendication 17, caractérisé en ce qu'il contient un composé de formule I dans laquelle $R_1$ représente l'hydrogène, le chlore, le brome, l'iode ou un groupe nitro, $R_2$ représente l'hydrogène, $R_3$ représente l'hydrogène, le fluor, le chlore, le brome, l'iode, un groupe alkyle en $C_1$-$C_3$ ou nitro, $R_4$ représente l'hydrogène, le brome ou un groupe méthyle, $R_5$ représente l'hydrogène, $R_6$ représente l'hydrogène ou un groupe méthyle, A représente —$CH_2$—, —$CH_2$—$CH_2$— ou —$CH(CH_3)$— et Z représente un groupe cyano,

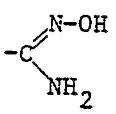 ou 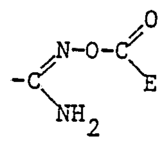

dans lesquels

E représente —$R_7$, —$OR_8$, —$SR_9$ ou —$NR_{10}R_{11}$, et

$R_7$ représente un groupe alkyle en $C_1$-$C_7$, alkyle en $C_1$-$C_3$ substitué par 1 à 3 atomes de chlore ou de brome, (alcoxy en $C_1$-$C_4$)-méthyle, cycloalkyle en $C_3$-$C_6$, alcényle en $C_2$-$C_3$, phényle non substitué ou portant 1 ou 2 substituants du groupe formé par le chlore, les groupes nitro et méthyle, benzyle non substitué ou monosubstitué par le chlore ou un groupe nitro, un cycle thiophène, furanne, tétrahydrofuranne ou pyrimidine non substitué ou mono- ou di-substitué par le chlore ou le brome,

$R_8$ représente un groupe alkyle en $C_1$-$C_4$, éthyle monosubstitué par le chlore ou le brome, alcényle en $C_2$-$C_3$, propynyle, phényle non substitué ou monosubstitué par un groupe nitro ou benzyle non substitué ou monosubstitué par un groupe nitro,

$R_9$ représente un groupe alkyle en $C_1$-$C_7$,

$R_{10}$ représente un groupe alkyle en $C_1$-$C_4$, chloréthyle, phényle non substitué ou portant 1 ou 2 substituants formés par le chlore, les groupes méthoxy et trifluorométhyle, et

$R_{11}$ représente l'hydrogène, un groupe méthyle ou méthoxy, ou bien

$R_{10}$ et $R_{11}$ forment ensemble et avec l'atome d'azote auquel ils sont reliés, un cycle pipéridine ou morpholine.

19. Produit selon la revendication 18, caractérisé en ce qu'il contient un composé de formule I dans laquelle

$R_1$ représente l'hydrogène, le chlore, le brome ou l'iode,

$R_2$ représente l'hydrogène, $R_3$ représente l'hydrogène, le chlore ou un groupe nitro, $R_4$ et $R_5$ représentent l'hydrogène, $R_6$ représente l'hydrogène ou un groupe méthyle,

A représente $—CH_2—$, $—CH_2—CH_2$ ou $—CH(CH_3)—$ et Z représente un groupe cyano,

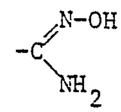

ou

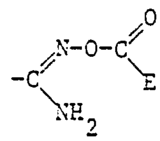

dans lesquels

E représente $—R_7$, $OR_8$, $—SR_9$ ou $—NR_{10}R_{11}$, et

$R_7$ représente un groupe méthyle, éthyle, n-propyle, isopropyle, n-butyle, tert.-butyle, isobutyle, chlorométhyle, bromométhyle, 2-chloréthyle, 3-chloro-n-propyle, 1,2-dichloréthyle, méthoxyméthyle, n-propoxyméthyle, sec.-butoxyméthyle, cyclopropyle, vinyle, 1-propényle, isopropényle, phényle, 2-chlorophényle, 4-chlorophényle, benzyle, 2-thiényle, 2-furyle, 5-bromo-2-furyle, 2-tétrahydrofuryle ou 2,4-dichloropyrimidine-5-yl,

$R_8$ représente un groupe méthyle, éthyle, n-propyle, n-butyle, 2-brométhyle, allyle, phényle ou benzyle,

$R_9$ représente un groupe méthyle, isopropyle ou n-pentyle,

$R_{10}$ représente un groupe méthyle, éthyle, isopropyle, n-butyle, phényle, 3-trifluorométhylphényle, 4-chlorophényle, 2,5-dichlorophényle, et

$R_{11}$ représente l'hydrogène ou un groupe méthoxy.

20. Produit selon la revendication 19, caractérisé en ce qu'il contient un composé de formule I dans laquelle

$R_1$ représente l'hydrogène, le chlore, le brome ou l'iode,

$R_2$ représente l'hydrogène ou le chlore, $R_4$ et $R_5$ représentent l'hydrogène, $R_6$ représente l'hydrogène ou un groupe méthyle, A représente $—CH_2—$ et Z représente un groupe cyano,

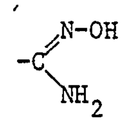

ou

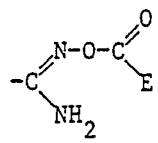

dans lesquels

E représente $—R_7$, $—OR_8$ ou $—NR_{10}R_{11}$, et $R_7$ représente un groupe chlorométhyle $R_8$ représente un groupe méthyle, $R_{10}$ un groupe isopropyle et $R_{11}$ l'hydrogène.

21. Produit selon la revendication 20, caractérisé en ce qu'il contient de la 5-chloro-8-(cyanométhoxy)-quinoléine.

22. Produit selon la revendication 20, caractérisé en ce qu'il contient de la 5-chloro-7-iodo-8-(cyanométhoxy)-quinoléine.

23. Produit selon la revendication 20, caractérisé en ce qu'il contient de l'O-(méthoxycarbonyl)-2-(8-quinoléinoxy)-acétamidoxime.

24. Produit selon la revendication 16, caractérisé en ce qu'il contient un composé de formule I et un herbicide.

25. Produit selon la revendication 24, caractérisé en ce qu'il contient en tant qu'herbicide un composé de formule A :

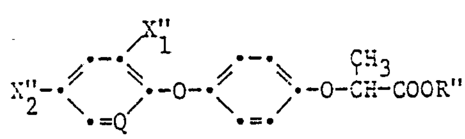

(A)

dans laquelle

$X_1''$ représente l'hydrogène ou un halogène,

$X_2''$ représente l'hydrogène, un halogène ou un groupe trifluorométhyle,

Q représente le fragment =N— ou =CH—,

R'' représente un groupe alkyle en $C_1$-$C_4$ non substitué ou substitué par un groupe alcoxy en $C_1$-$C_4$, un groupe alcényle en $C_3$-$C_4$, alcynyle en $C_3$-$C_4$ ou

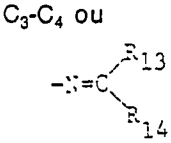

$R_{13}$ représente un groupe alkyle en $C_1$-$C_4$,
$R_{14}$ représente un groupe alkyle en $C_1$-$C_4$, ou bien
$R_{13}$ et $R_{14}$ forment ensemble un groupe alkylène en $C_4$-$C_5$.

26. Produit selon la revendication 24, caractérisé en ce qu'il contient en tant qu'herbicide un ester pyridyloxyphénoxypropionique.

27. Produit selon la revendication 24, caractérisé en ce qu'il contient en tant qu'herbicide le 2-[4-(3,5-dichloropyridyl-2-oxy)-phénoxy]-propionate de 2-propynyle.

28. Produit selon la revendication 24, caractérisé en ce qu'il contient en tant qu'herbicide une phénylurée.

29. Produit selon la revendication 24, caractérisé en ce qu'il contient en tant qu'herbicide une sulfonylurée.

30. Produit selon la revendication 24, caractérisé en ce qu'il contient en tant que composé de formule I, la 5-chloro-8-(cyanométhoxy)-quinoléine et en tant qu'herbicide le 2-[4-(3,5-dichloropyridyl-2-oxy)-phénoxy]-propionate de 2-propynyle.

31. Produit selon la revendication 16, caractérisé en ce qu'il contient un composé de formule Ia :

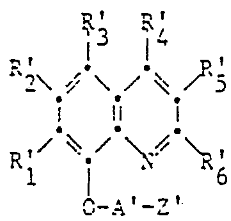

(Ia)

dans laquelle
$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano,
$R_4'$, $R_5'$ et $R_6'$ représentent chacun, indépendamment les uns des autres, l'hydrogène, un halogène ou un groupe alkyle en $C_1$-$C_3$,
A' représente l'un des groupes $-CH_2-$, $-CH_2-CH_2$ ou $-CH(CH_3)-$, et
Z' représente un groupe cyano ou l'un des groupes

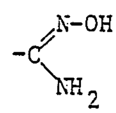

ou

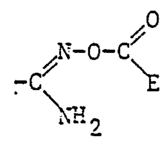

dans lesquels
E représente $-R_7$, $-OR_8$, $-SR_9$ ou $-NR_{10}R_{11}$, et
$R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons contenant 1 ou 2 hétéroatomes du groupe formé par N, O et S, et non substitué ou substitué par des halogènes,
$R_8$, $R_9$ et $R_{10}$ représentent chacun, indépendamment les uns des autres, un groupe alkyle en $C_1$-$C_8$ non substitué ou substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou benzyle non substitué ou substitué par des halogènes ou des groupes nitro,
$R_{11}$ représente l'hydrogène, un groupe alkyle en $C_1$-$C_8$ ou alcoxy en $C_1$-$C_3$, ou bien $R_{10}$ et $R_{11}$ forment ensemble et avec l'atome d'azote auquel ils sont reliés, un hétérocycle à 5 ou 6 chaînons qui peut contenir un autre hétéroatome du groupe formé par N, O et S, y compris leurs sels formés par addition avec des acides et complexes métalliques, avec la restriction que Z' ne peut représenter un groupe cyano ou le groupe

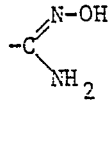

lorsque, en même temps, $R_1'$, $R_2'$, $R_4'$, $R_5'$ et $R_6'$ représentent l'hydrogène, $R_3'$ représente l'hydrogène ou le chlore et A' représente —$CH_2$— ou —$CH(CH_3)$—.

32. Procédé de préparation des composés de formule Ia :

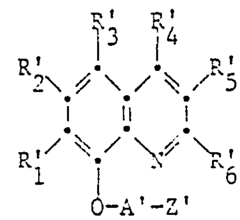

(Ia)

dans laquelle

$R_1'$, $R_2'$ et $R_3'$ représentent chacun, indépendamment les uns des autres, l'hydrogène, un halogène, un groupe alkyle en $C_1$-$C_3$, alcoxy en $C_1$-$C_3$, nitro ou cyano,

$R_4'$, $R_5'$ et $R_6'$ représentent chacun, indépendamment les uns des autres, l'hydrogène, un halogène ou un groupe alkyle en $C_1$-$C_3$,

A' représente l'un des groupes —$CH_2$—, —$CH_2$—$CH_2$ ou —$CH(CH_3)$—,

Z' représente un groupe cyano ou amidoxime qui peut être acylé sur l'atome d'oxygène,

y compris leurs sels formés par addition avec des acides et complexes métalliques, avec la restriction que Z' ne peut représenter un groupe cyano ou amidoxime lorsque, en même temps $R_1'$, $R_2'$, $R_4'$, $R_5'$ et $R_6'$ représentent l'hydrogène, $R_3'$ l'hydrogène ou le chlore et A' un groupe —$CH_2$— ou —$CH(CH_3)$—, caractérisé en ce que :

a) pour préparer les composés de formule Ia dans laquelle $R_1'$, $R_2'$, $R_3'$, $R_4'$ et $R_6'$ ont les significations indiquées en référence à la formule Ia, A' représente le groupe —$CH_2$—$CH_2$— et Z' un groupe cyano, on fait réagir un composé de formule II :

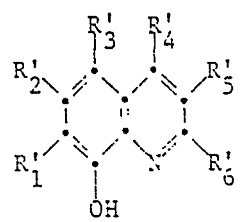

(II)

dans laquelle $R_1'$, $R_2'$, $R_3'$, $R_4'$, $R_5'$ et $R_6'$ ont les significations indiquées ci-dessus, avec un composé de formule III :

$$CH_2{=}CH{-}CN \qquad \text{(III)}$$

ou bien

b) pour préparer les composés de formule Ia dans laquelle $R_1'$, $R_2'$, $R_3'$, $R_4'$, $R_5'$ et $R_6'$ ont les significations indiquées en référence à la formule Ia, A' représente l'un des groupes —$CH_2$— ou —$CH(CH_3)$— et Z' un groupe cyano, on fait réagir un composé de formule II :

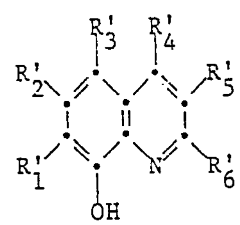

(II)

dans laquelle $R_1'$, $R_2'$, $R_3'$, $R_4'$, $R_5'$ et $R_6'$ ont les significations indiquées en référence à la formule II, avec

i) un composé de formule IV

$$Hal{-}A'{-}CN \qquad \text{(IV)}$$

dans laquelle Hal représente un atome d'halogène et A' a la signification indiquée ci-dessus, ou bien

ii) un composé de formule V :

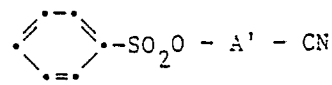 (V)

dans laquelle A' a la signification indiquée ci-dessus, ou bien

iii) un composé de formule VI :

$$Hal—A—COOR_{12} \quad (VI)$$

dans laquelle Hal représente un atome d'halogène et $R_{12}$ un groupe alkyle en $C_1$-$C_6$ et A' a la signification indiquée ci-dessus,
et on convertit les esters obtenus répondant à la formule VII :

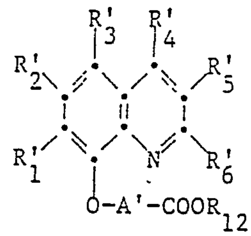 (VII)

dans laquelle $R_1'$, $R_2'$, $R_3'$, $R_4'$, $R_5'$, $R_6'$ et $R_{12}$ et A' ont les significations indiquées ci-dessus, par réaction avec l'ammoniac, en les amides correspondants de formule VIII :

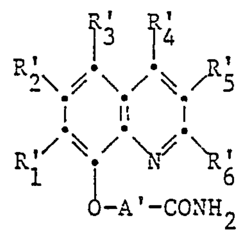 (VIII)

dans laquelle $R_1'$, $R_2'$, $R_3'$, $R_4'$, $R_5'$, $R_6'$ et A' ont les significations indiquées ci-dessus, et on fait suivre d'une déshydratation, et/ou

c) pour préparer les composés de formule Ia dans laquelle $R_1'$, $R_2'$, $R_3'$, $R_4'$, $R_5'$, $R_6'$, et A' ont les significations indiquées en référence à la formule Ia et Z' représente un groupe amidoxime qui peut être acylé sur l'atome d'oxygène, on fait réagir un composé de formule Ia dans laquelle $R_1'$, $R_2'$, $R_3'$, $R_4'$, $R_5'$, $R_6'$ et A' ont les significations indiquées en référence à la formule Ia et Z' représente un groupe cyano, avec l'hydroxylamine ou un sel de l'hydroxylamine et d'un acide, et/ou

d) pour préparer les composés de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime acylé, on acyle un composé de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime.

33. Procédé selon la revendication 32, caractérisé en ce que, pour préparer les composés de formule Ia dans laquelle $R_1'$, $R_2'$, $R_3'$, $R_4'$, $R_5'$, $R_6'$ et A' ont les significations indiquées en référence à la formule Ia et Z' représente un groupe amidoxime acylé de formule

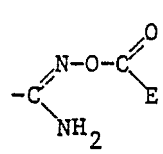

dans laquelle E représente —$R_7$, —$OR_8$, —$SR_9$ ou —$NR_{10}R_{11}$, et $R_7$ représente un groupe alkyle en $C_1$-$C_7$ non substitué ou substitué par des halogènes ou des groupes alcoxy en $C_1$-$C_4$, un groupe cycloalkyle en $C_3$-$C_6$, alcényle en $C_2$-$C_4$, phényle non substitué ou substitué par des halogènes, des groupes nitro ou alkyle en $C_1$-$C_3$, benzyle non substitué ou substitué par des halogènes, des groupes nitro ou alkyle en $C_1$-$C_3$, ou un noyau hétérocyclique à 5 ou 6 chaînons qui contient 1 ou 2 hétéroatomes du groupe formé par N, O et S, et est non substitué ou substitué par des halogènes, $R_8$, $R_9$ et $R_{10}$ représentent chacun, indépendamment les uns des autres, un groupe alkyle en $C_1$-$C_8$ non substitué par des halogènes, un groupe alcényle en $C_2$-$C_4$, alcynyle en $C_3$-$C_6$, phényle non substitué ou substitué par des halogènes, des groupes alkyle en $C_1$-$C_3$, alcoxy en $C_1$-$C_3$, trifluorométhyle ou nitro, ou un groupe benzyle non substitué par des halogènes ou des groupes nitro, $R_{11}$ représente l'hydrogène, un groupe alkyle en $C_1$-$C_8$ ou alcoxy $C_1$-$C_3$, ou bien $R_{10}$ et $R_{11}$ forment ensemble et avec l'atome d'azote auquel ils sont reliés un

hétérocycle à 5 ou 6 chaînons qui peut encore contenir un autre hétéroatome du groupe formé par N, O et S, on fait réagir un composé de formule Ia dans laquelle $R'_1$, $R'_2$, $R'_3$, $R'_4$, $R'_5$, $R'_6$ et A' ont les significations indiquées en référence à la formule Ia et Z' représente un groupe amidoxime, avec un composé de formule IX :

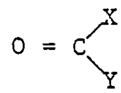

(IX)

dans laquelle X représente un atome d'halogène et Y représente $-R_7$, $-OR_8$, $SR_9$ ou $-NR_{10}R_{11}$, et $R_7$, $R_8$, $R_9$, $R_{10}$ et $R_{11}$ ont les significations indiquées ci-dessus ou bien X et Y forment ensemble le groupe imino $=N-R_{10}$.

34. Procédé pour combattre sélectivement les mauvaises herbes dans les cultures de végétaux utiles, caractérisé en ce que l'on traite les cultures, des parties des végétaux cultivés ou les terrains de culture par un herbicide et un composé de formule I :

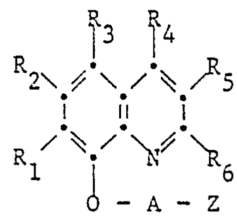

(I)

dans laquelle

$R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano,

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, ou par un produit contenant l'un de ces composés.

35. Produit pour combattre sélectivement les mauvaises herbes dans les cultures de végétaux utiles, caractérisé en ce qu'il contient un composé de formule I :

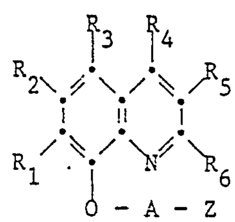

(I)

dans laquelle $R_1$, $R_2$ et $R_3$ représentent chacun, indépendamment les uns des autres, l'hydrogène, un halogène, un groupe alkyle en $C_1$-$C_3$, alcoxy en $C_1$-$C_3$, nitro ou cyano

$R_4$, $R_5$ et $R_6$ représentent chacun, indépendamment les uns des autres, l'hydrogène, un halogène ou un groupe alkyle en $C_1$-$C_3$,

A représente l'un des groupes $-CH_2-$, $-CH_2-CH_2-$ ou $-CH(CH_3)-$, et

Z représente un groupe cyano ou amidoxime qui peut être acylé sur l'atome d'oxygène, y compris leurs sels formés par addition avec des acides et complexes métalliques, et un herbicide.